(19) Europäisches Patentamt
European Patent Office
Office européen des brevets

(11) Veröffentlichungsnummer: **0 015 240**
**A1**

(12) # EUROPÄISCHE PATENTANMELDUNG

(21) Anmeldenummer: **80810051.5**

(22) Anmeldetag: **11.02.80**

(51) Int. Cl.³: **C 07 D 501/59**
C 07 D 501/00, C 07 D 501/20
//C07D233/38, C07D241/08

(30) Priorität: **16.02.79 CH 1548/79**

(43) Veröffentlichungstag der Anmeldung:
**03.09.80 Patentblatt 80/18**

(84) Benannte Vertragsstaaten:
**BE CH DE FR GB IT LU NL SE**

(71) Anmelder: **CIBA-GEIGY AG**
**Patentabteilung Postfach**
**CH-4002 Basel(CH)**

(72) Erfinder: **Fechtig, Bruno, Dr.**
**Hinterlindenweg 1**
**CH-4153 Reinach(CH)**

(72) Erfinder: **Scartazzini, Riccardo, Dr.**
**Conrad Ferdinand Meyer-Strasse 38**
**CH-4059 Basel(CH)**

(54) **Azacyclyl (thio) ureidoacetyl-Verbindungen und Verfahren zu ihrer Herstellung.**

(57) Die Erfindung betrifft Azacyclyl(thio)- ureiodoacetamido-cephem- Verbindungen der Formel

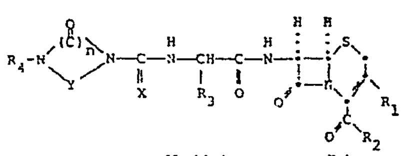

. (I).

worin $R_1$ Wasserstoff, Halogen, veräthertes Hydroxy oder Mercapto, oder einen Rest $-CH_2-C(=O)-R_6$, worin $R_6$ Wasserstoff, Hydroxy, Niederalkoxy, Niederalkyl oder gegebenenfalls substituiertes Amino darstellt, $R_2$ Hydroxy oder eine verätherte Hydroxygruppe, die, zusammen mit der Gruppe $-C=O$, eine unter physiologischen Bedingungen spaltbare veresterte Carboxylgruppe $-C(=O)-OR_5$ bildet, $R_3$ Wasserstoff, einen gegebenenfalls substituierten, ungesättigten cyclischen Kohlenwasserstoffrest oder Heterocyclyl, X Sauerstoff oder Schwefel, der Index n den Wert 1 oder 2, $R_4$ Wasserstoff, einen gegebenenfalls substituierten niederaliphatischen oder cycloaliphatischen Rest oder Acyl und Y ein die Stickstoffatome durch zwei Kohlenstoffatome trennendes Niederalkylen bedeuten, Salze von salzbildenden Verbindungen der Formel I, sowie Isomere davon, sowie Verfahren zu ihrer Herstellung, solche Stoffe der Formel I enthaltende Präparate und deren Verwendung sowie entsprechende neue Zwischenprodukte.

Die Herstellung dieser Verbindungen erfolgt in an sich bekannte Weise, z.B. durch Einführen der Acylgruppe in eine entsprechende 7ß-Amino-3-cephem-Verbindung.

Die Verbindungen haben antibiotische Wirksamkeit und können zur Bekämpfung von Infektionen verwendet werden.

EP 0 015 240 A1

0015240



BEZEICHNUNG GEÄNDERT
siehe Titelseite

CIBA-GEIGY AG
Basel Schweiz

4-12242/+

Azacyclyl(thio)ureidoacetyl-Verbindungen

Die Erfindung betrifft neue antibiotisch wirksame Azacyclyl(thio)ureidoacetamido-cephem-Verbindungen und Salze von solchen Verbindungen mit salzbildenden Gruppen, Verfahren zu ihrer Herstellung, diese Verbindungen enthaltende pharmazeutische Mittel mit antibiotischer Wirksamkeit und ihre Verwendung sowie neue Zwischenprodukte zu deren Herstellung.

Einige Azacyclyl(thio)ureidoacetamido-cephem-Verbindungen sind bereits als gut wirksame Antibiotika beschrieben worden, z.B. in den DE-OS 2.519.400, 2.702.552 oder 2.720.579. Die vorliegende Erfindung stellt neue 7β-[(2-Oxoimidazolidino- oder 2.3-Dioxopiperazino(thio)carbonylamino)-acetamido]-3-cephem-4-carbonsäureverbindungen zur Verfügung, die ein breites Wirkungsspektrum besitzen.

Die Erfindung betrifft insbesondere Verbindungen der Formel

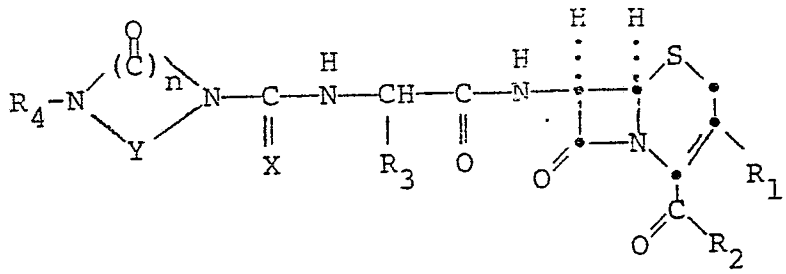

(I),

worin $R_1$ Wasserstoff, Halogen, veräthertes Hydroxy oder Mercapto, oder einen Rest $-CH_2-C(=O)-R_6$, worin $R_6$ Wasserstoff, Hydroxy, Niederalkoxy, Niederalkyl oder gegebenenfalls substituiertes Amino darstellt, $R_2$ Hydroxy oder eine verätherte Hydroxygruppe, die, zusammen mit der Gruppe -C=O, eine unter physiologischen Bedingungen spaltbare veresterte Carboxylgruppe $-C(=O)-OR_5$ bildet, $R_3$ Wasserstoff, einen gegebenenfalls substituierten, ungesättigten cyclischen Kohlenwasserstoffrest oder Heterocyclyl, X Sauerstoff oder Schwefel, der Index n den Wert 1 oder 2, $R_4$ Wasserstoff, einen gegebenenfalls substituierten niederaliphatischen oder cycloaliphatischen Rest oder Acyl und Y ein die Stickstoffatome durch zwei Kohlenstoffatome trennendes Niederalkylen bedeuten, Salze von salzbildenden Verbindungen der Formel I, sowie Isomere davon, Verfahren zu ihrer Herstellung, solche Stoffe enthaltende pharmazeutische Präparate und deren Verwendung, sowie geschützte Derivate von Verbindungen der Formel I und andere neue Zwischenprodukte.

Die vor- wie nachstehend genannten Allgemeinbezeichnungen für Gruppen haben, sofern nicht ausdrücklich anders definiert, die weiter unten angegebenen Bedeutungen.

$R_1$ ist als Halogen Fluor, Brom und insbesondere Chlor. Verätherte Hydroxy- oder Mercaptogruppen $R_1$ sind niederaliphatisch, cycloaliphatisch oder arylniederaliphatisch, Mercaptogruppen ausserdem auch aromatisch oder heterocyclisch veräthert. Niederaliphatisch verätherte Hydroxy-

oder Mercaptogruppen $R_1$ sind mit einem gegebenenfalls substituierten, einwertigen niederaliphatischen Kohlenwasserstoffrest, insbesondere mit einem entsprechenden Niederalkyl- oder Niederalkenylrest veräthert. Substituenten dieses Restes sind z.B. funktionelle Gruppen, z.B.Hydroxy, niederaliphatisches Acyloxy,z.B. Niederalkanoyloxy, Niederalkoxy oder Niederalkylthio, Halogen, z.B.Chlor, sowie funktionell abgewandeltes Carboxy oder gegebenenfalls substituiertes Amino. Cycloaliphatisch verätherte Hydroxy- oder Mercaptogruppen $R_1$ sind vorzugsweise gesättigt, können aber auch einfach ungesättigt sein und haben 3-8, vorzugsweise 5 oder 6 Ring-Kohlenstoffatome. Arylniederaliphatisch verätherte Hydroxy- oder Mercaptogruppen $R_1$ sind z.B. gegebenenfalls substituiertes Phenylniederalkoxy bzw. Phenylniederalkylmercapto, worin Niederalkyl bis zu 4 Kohlenstoffatome aufweist. Substituenten befinden sich im Arylteil und sind insbesondere Halogen, z.B. Chlor, Niederalkoxy, z.B. Methoxy, Niederalkyl, z.B. Methyl oder Nitro. Aromatisch verätherte Mercaptogruppen $R_1$ sind z.B. gegebenenfalls substituierte Phenylthiogruppen, worin Phenyl wie ein Phenylniederalkylrest substituiert sein kann.

Heterocyclisch verätherte Mercaptogruppen $R_1$ sind durch einen gegebenenfalls substituierten Heterocyclylrest veräthert. Derartige Heterocyclylreste sind in erster Linie gegebenenfalls substituierte, z.B. die unten genannten Substituenten enthaltende, sowie gegebenenfalls benzokondensierte monocyclische, fünfgliedrige Reste aromatischen Charakters, ferner entsprechende partiell gesättigte Reste sowie entsprechende sechsgliedrige Heterocyclylreste, wobei ein solcher Rest mindestens ein Heteroatom aus der Gruppe N, O oder S sowie 1-3 weitere Ring-Stickstoffatome enthält.

Beispiele für entsprechende 5-gliedrige Heterocyclylringe sind Oxazolyl, Isoxazolyl, Thiazolyl, Isothiazolyl, Imidazolyl, Benzimidazolyl, Pyrazolyl, Oxadiazolyl, z.B. 1,3,4-Oxadiazolyl oder 1,2,4-Oxadiazolyl, Thiadiazolyl, z.B. 1,3,4-Thiadiazolyl oder 1,2,4-Thiadiazolyl, Triazolyl, z.B. 1,2,3-Triazolyl oder 1,2,4-Triazolyl und Tetrazolyl, sowie entsprechende partiell gesättigte Reste, wie Imidazolinyl oder Thiazolinyl. Beispiele für sechsgliedrige Heterocyclylringe sind Pyridyl, Pyrazyl, Pyrimidyl, Pyridazinyl, Chinolyl, Isochinolyl, Chinazolyl, Indolyl und Indazolyl. Die vorgenannten Ringe sind über ein Ring-Kohlenstoffatom an das Thioäther-Schwefelatom gebunden. Bevorzugte Heterocyclylringe sind Pyridyl und gegebenenfalls substituierte 5-gliedrige Ringe mit einem der genannten Ring-Heteroatome und 1-3 weiteren Ring-Stickstoffatomen.

Substituenten im Heterocyclylteil solcher Heterocyclylthioreste $R_1$ sind gegebenenfalls substituiertes Niederalkyl, wie unten näher definiert, ferner Cycloalkyl, z.B. Cyclopentyl oder Cyclohexyl, Aryl, wie gegebenenfalls durch Halogen, z.B. Chlor, oder Nitro substituiertes Phenyl, Arylniederalkyl, z.B. Benzyl, Halogen, z.B. Fluor, Chlor oder Brom, gegebenenfalls substituiertes Amino, z.B. niederalkyliertes Amino, Niederalkanoylamino oder durch Halogen oder Carboxy substituiertes Niederalkanoylamino, Nitro, Hydroxy, Niederalkoxy, oder gegebenenfalls funktionell abgewandeltes Carboxy, wie Carboxy, verestertes Carboxy, z.B. Niederalkoxycarbonyl, gegebenenfalls substituiertes, z.B. N-mono- oder N,N-diniederalkyliertes Carbamoyl oder Cyan, sowie Oxo oder Oxido, wobei vorzugsweise nur ein solcher Substituent, in erster Linie an ein Ringkohlenstoffatom, aber auch, insbesondere gegebenenfalls substituiertes Niederalkyl und Oxido, an ein Ringstickstoffatom gebunden, vorhanden sein können. Gegebenenfalls substituiertes Niederalkyl steht als Substituent eines Heterocyclylrestes beispielsweise für durch Hydroxy, veräthertes Hydroxy, z.B. Niederalkoxy, insbesondere Methoxy, verestertes Hydroxy, z.B. Niederalkanoyloxy, Halogen, z.B. Chlor, Carboxy, verestertes Carboxy, z.B. Niederalkoxycarbonyl, Sulfo, amidiertes Sulfo, Amino, Mono- oder Diniederalkylamino, Acylamino, z.B. Niederalkanoylamino, oder substituiertes, z.B. durch Carboxy oder Halogen substituiertes Niederalkanoylamino, substituiertes Niederalkyl, insbesondere für entsprechendes Methyl oder Aethyl.

Entsprechende Beispiele für bevorzugte Substituenten im Heterocyclylteil eines Heterocyclylthiorestes $R_1$ sind Niederalkyl mit 1-4 C-Atomen, sowie entsprechendes, z.B. durch Hydroxy, Methoxy, Aethoxy, Acetoxy, Chlor, Trichlor, Carboxy, Aethoxycarbonyl, Cyan, Sulfo, Sulfamoyl, Amino, Dimethylamino, Diäthylamino, Acetylamino und 3-Chlorpropionylamino substituiertes Niederalkyl, wie entsprechendes Methyl oder in 1- oder besonders in 2-Stellung substituiertes Aethyl, sowie Phenyl, Halogen, z.B. Chlor oder Brom, gegebenenfalls niederalkyliertes Amino, z.B. Dimethylamino, und Niederalkoxy, z.B. Methoxy.

Eine gegebenenfalls substituierte Aminogruppe $R_6$ steht für niederalkyliertes, z.B. mono- oder diniederalkyliertes, durch Niederalkylen oder Heteroniederalkylen, wie Oxa-, Thia-, Aza- oder N-Niederalkylazaniederalkylen, cyclisch disubstituiertes Amino oder Arylamino, z.B. Anilino.

Bevorzugte Reste $R_1$ sind z.B. Halogen, z.B. Chlor, Niederalkoxy, z.B. Methoxy, Niederalkylthio, z.B. Methylthio, gegebenenfalls wie oben substituiertes Phenylthio, insbesondere Phenylthio, und gegebenenfalls durch oben genannte bevorzugte Substituenten des Heterocyclylrestes

substituiertes Thiazolylthio, Oxazolylthio, Oxadiazolylthio, z.B. 1,2,4- oder 1,3,4-Oxadiazolylthio, Thiadiazolylthio, z.B. 1,2,4- oder 1,3,4-Thiadiazolylthio, Triazolylthio, z.B. 1,2,4- oder 1,3,4- Triazolylthio, oder Tetrazolylthio, z.B. 5-Tetrazolylthio, Carboxymethyl, Niederalkoxycarbonylmethyl, z.B. Methoxycarbonylmethyl, Niederalkylcarbonylmethyl, z.B. Methylcarbonylmethyl und gegebenenfalls N-niederalkyliertes z.B. N-mono- oder diniederalkyliertes Carbamoylmethyl.

Eine unter physiologischen Bedingungen spaltbare veresterte Carboxylgruppe der Formel $-C(=O)-OR_5$ kann eine beliebige, an sich bekannte, physiologisch spaltbare und physiologisch verträgliche veresterte Carboxylgruppe sein und ist beispielsweise durch gegebenenfalls niederalkyliertes oder acyliertes Amino substituiertes Niederalkoxycarbonyl, vorzugsweise jedoch eine Gruppe der Teilformel

$$-\underset{\underset{O}{\parallel}}{C}-O-\underset{\underset{R_8}{|}}{CH}-T-R_7 \qquad (A)$$

worin $R_8$ Wasserstoff oder Niederalkyl mit 1-3 Kohlenstoffatomen, insbesondere Wasserstoff oder Methyl bedeutet, $R_7$ den Acylrest einer gegebenenfalls substituierten Carbonsäure mit bis zu 12 Kohlenstoffatomen, eines Kohlensäurehalbderivats, wie eines Niederalkylhalbesters oder eines am N-Atom gegebenenfalls niederalkyliertem, z.B. diniederalkylierten Kohlensäurehalbamids, oder einer aromatischen oder niederaliphatischen Sulfonsäure , sowie gegebenenfalls durch Hydroxy, Niederalkoxy, z.B. Methoxy, Amino oder Diniederalkylamino, z.B. Dimethylamino, substituiertes Niederalkyl, Cycloalkyl mit 3-7 Kohlenstoffatomen, gegebenenfalls durch Chlor, Hydroxy oder Methoxy substituiertes Phenyl oder gegebenenfalls entsprechend substituiertes Phenylniederalkyl, wie entsprechendes Benzyl, und/oder gegebenenfalls hydriertes und gegebenenfalls Benzo-kondensiertes fünf-oder sechs-gliedriges Heterocyclyl mit 1-2 Ring-Sauerstoff-, Schwefel- oder Stickstoffatomen in Nachbarstellung zum bindenden Kohlenstoffatom darstellt, T eine Aether-, Thioäther-, Sulfinyl-, Sulfonyl-, Imino-, Niederalkylimino- oder Acylimino-Gruppe bedeutet, oder worin die Gruppierung $-CH(R_8)-T-R_7$ oder $-T-R_7$ Bestandteil eines heterocyclischen Ringsystems ist.

Bevorzugte Gruppen (A) sind z.B. solche, worin $R_8$ Wasserstoff oder Methyl, T Sauerstoff oder Schwefel und $R_7$ der Acylrest einer Monocarbonsäure, beispielsweise gegebenenfalls durch Hydroxy, Niederalkoxy, z.B. Methoxy, Halogen, z.B. Chlor, Niederalkylthio, z.B. Methylthio, Amino oder Diniederalkylamino, z.B. Dimethylamino, substituiertes Niederalkanoyl, der Rest eines Kohlensäurehalbderivats, z.B. eines Kohlen- oder Thiokohlensäureniederalkylhalbesters, z.B. Niederalkoxycarbonyl oder -thiocarbonyl, oder -halbamids, z.B. Carbamoyl ist, oder worin $R_7$ gegebenenfalls durch Niederalkoxy substituiertes Niederalkyl ist.

Eine weitere bevorzugte Gruppe (A) ist diejenige, worin die Gruppierung $-CH(R_8)-T-R_7$ Bestandteil eines heterocyclischen Ringsystems ist, das, zusammen mit der Carbonyloxygruppe beispielsweise eine 5-Oxo-dihydro-2-furfuryloxycarbonyl-, 5-Oxo-tetrahydro-2-furfuryloxycarbonyl-, Phthalidyloxycarbonyl- oder 5,6-Dimethoxyphthalidyloxycarbonylgruppe bildet.

Die Gruppierung $-T-R_7$ kann ebenfalls Bestandteil eines heterocyclischen Ringsystems sein und ist dann beispielsweise eine Diacyliminogruppe, wie eine Succinimido-, Saccharimido- oder Phthalimidogruppe, die mit dem Rest $-CO-O-CH(R_8)-$ eine Gruppe (A), z.B. die Succinimidomethoxycarbonyl-, Saccharimidomethoxycarbonyl- oder Phthalimidomethoxycarbonylgruppe bildet.

Bevorzugte Beispiele für physiologisch spaltbare Gruppen $-C(=O)-OR_5$ sind z.B. in α-Stellung substituiertes, wie durch Niederalkanoyloxy, z.B. Acetoxy oder Pivaloyloxy, α-Aminoniederalkanoyloxy, z.B. Glycyloxy, Niederalkoxy, z.B. Methoxy, Niederalkoxycarbonyl, z.B. Methoxycarbonyloxy, sowie Carbamoyloxy oder Phthalidyloxy substituiertes Niederalkoxycarbonyl, insbesondere entsprechendes Methoxy- oder Aethoxycarbonyl, beispielsweise Acetoxymethoxycarbonyl, Pivaloyloxymethoxycarbonyl, Glycyloxymethoxycarbonyl, L-Valyloxymethoxycarbonyl, (L-Leucyloxy)-methoxycarbonyl, Methoxymethoxycarbonyl, Aethoxymethoxycarbonyl, α-(Methoxy)-äthoxycarbonyl, Methoxycarbonyloxymethoxycarbonyl, α-(Aethoxycarbonyloxy)-äthoxycarbonyl, Carbamoyloxymethoxycarbonyl oder 2-Phthalidyloxymethoxycarbonyl.

Ein gegebenenfalls substituierter ungesättigter cyclischer Kohlenwasserstoffrest $R_3$ ist insbesondere ein entsprechender cycloaliphatischer Rest mit 1-2 Doppelbindungen, von denen eine vom 1-ständigen (d.h. bindenden) Kohlenstoffatom ausgeht, oder ein entsprechender aromatischer Rest.

Ein solcher cycloaliphatischer Rest $R_3$ hat 5-8, vorzugsweise 6 Ringatome und ist ein entsprechender ein- oder zweifach ungesättigter Rest, beispielsweise Cycloalkenyl, wie Cyclopentenyl oder Cyclohexenyl, z.B. 1-Cyclohexenyl, oder Cycloalkadienyl, beispielsweise Cyclopentadienyl oder insbesondere Cyclohexadienyl, z.B. 1,4-Cyclohexadienyl. Substituenten dieser cycloaliphatischen Reste sind insbesondere Niederalkyl, z.B. Methyl, oder Niederalkoxy, z.B. Methoxy, Hydroxy, oder Niederalkanoyloxy, z.B. Acetoxy.

Ein aromatischer Rest $R_3$ ist gegebenenfalls ein- oder zweifach durch Hydroxy, Niederalkyl, Niederalkoxy, Niederalkylthio, Halogen, Nitro, Niederalkylsulfonylamino, Cyan,

Carbamoyloxy oder Niederalkanoyloxy substituiertes Naphthyl, z.B. α- oder β-Naphthyl und vor allem gegebenenfalls entsprechend substituiertes Phenyl. Bevorzugte Beispiele für aromatische Reste $R_3$ sind Phenyl, sowie durch Hydroxy, Cyan, Fluor, Chlor, Acetoxy, Carbamoyloxy oder Nitro, ein- oder zweifach substituiertes Phenyl.

Ein Heterocyclylrest $R_3$ enthält mindestens ein Heteroatom aus der Gruppe N, O oder S sowie gegebenenfalls 1-3 weitere Ringstickstoff-atome und ist vorzugsweise über ein Ring-Kohlenstoffatom, insbesondere über ein einem Heteroatom benachbartes Kohlenstoffatom, gebunden.

Substituenten eines solchen Heterocyclylrestes $R_3$ sind z.B. Halogen, insbesondere Chlor, Amino, Mono- und Diniederalkylamino, Hydroxy, Niederalkyl, z.B. Methyl, Niederalkoxy, z.B. Methoxy, Niederalkylthio, z.B. Methylthio, Niederalkylsulfonylamino, z.B. Mesylamino, oder gegebenenfalls funktionell abgewandeltes Carboxy, z.B. Carboxy, Carbamoyl, oder Niederalkoxycarbonyl z.B. Methoxycarbonyl oder Cyan.

Bevorzugte Beispiele für derartige Heterocyclylreste $R_3$ sind z.B. Thienyl, z.B. 2- oder 3-Thienyl, Furyl, z.B. 2- oder 3-Furyl, 4-Pyridyl, Tetrazolyl, z.B. 5-Tetrazolyl, Niederalkyl-, z.B. 1-Methyl-5-tetrazolyl, Thiazolyl, z.B. 2-Thiazolyl, Aminothiazolyl, z.B. 2-Amino-4-thiazolyl (bzw. die dazu tautomere Form 2-Iminothiazolin-4-yl) oder Thiadiazolyl, z.B. 1,3,4-Thiadiazol-5-yl, 2-Niederalkyl-, z.B. 2-Methyl-1,3,4-thiadiazol-5-yl,Pyrimidyl oder Dihydroxypyrimidyl, z.B. 2,4-Dihydroxy-5- oder -6-pyrimidyl.

$R_3$ bedeutet in erster Linie gegebenenfalls,wie oben angegeben,substituiertes Phenyl, z.B. Phenyl, p-Hydroxyphenyl, p-Acetoxyphenyl, p-Carbamoyloxyphenyl, Cyanphenyl, 3-Nitro-

4-hydroxyphenyl, m-Methylsulfonylaminophenyl, Thienyl, z.B. 2-Thienyl, Furyl, z.B. 2-Furyl, 2-Aminothiazol-4-yl (bzw. 2-Imino-thiazolin-4-yl) oder Cyclohexadienyl, z.B. 1,4-Cyclohexadienyl.

Ein niederaliphatischer Rest $R_4$ ist Niederalkyl, Niederalkenyl oder Niederalkinyl.

Ein Niederalkylrest $R_4$ ist insbesondere ein Rest mit 1-4 Kohlenstoffatomen, wie Methyl, Isopropyl oder Isobutyl, vor allem Aethyl.

Als Niederalkenyl besitzt $R_4$ vorzugsweise 2-5 Kohlenstoffatome und die Doppelbindung geht vorzugsweise vom bindenden Kohlenstoffatom (1-Stellung) aus. Beispiele für derartige Reste sind Vinyl, Propenyl und insbesondere 1-Isobutenyl.

Als Niederalkinyl besitzt $R_4$ vorzugsweise 2-4 Kohlenstoffatome und ist vor allem Aethinyl und 1-Propinyl.

Ein cycloaliphatischer Rest $R_4$ ist Cycloalkyl mit 3-8, vorzugsweise 3-6 C-Atomen und ist vor allem Cyclopropyl, Cyclobutyl, Cyclopentyl oder Cyclohexyl.

Substituenten für vorstehend genannte Reste $R_4$, insbesondere für ein entsprechendes Niederalkyl, sind Hydroxy, Niederalkanoyloxy, Niederalkoxycarbonyloxy, Niederalkoxy, Niederalkylthio, Halogen, insbesondere Chlor oder Brom, gegebenenfalls funktionell abgewandeltes Carboxy und gegebenenfalls niederalkyliertes, wie diniederalkyliertes Amino. Bevorzugte Substituenten sind Hydroxy und Niederalkoxy, die vorzugsweise am bindenden Kohlenstoffatom (also in 1-Stellung) fixiert sind. Beispiele für substituierte Reste dieser Art sind 1-Hydroxyäthyl, Methoxymethyl, 1-Methoxyäthyl, 2-Methoxyäthyl, 4-Methoxybutyl

und insbesondere 1-Hydroxyisobutyl.

Ein Acylrest $R_4$ ist ein von einer Carbonsäure mit bis zu 12 Kohlenstoffatomen, einem Halbester der Kohlensäure oder Thiokohlensäure, einer aromatischen oder niederaliphatischen Sulfonsäure oder einem gegebenenfalls N-subsituierten Halbamid der Kohlen-, Thiokohlen-, Iminokohlen- oder Schwefelsäure abgeleiteter Acylrest.

Ein Carbonsäurerest $R_4$ ist in erster Linie ein aromatischer, wie gegebenenfalls durch Halogen, z.B. Chlor, Niederalkyl, Hydroxy, Niederalkoxy, Diniederalkylamino, z.B. Dimethylamino, Niederalkylsulfonylamino, z.B. Mesylamino, Niederalkanoylamino, z.B. Acetamido, Amino oder Nitro substituierter Naphthoe- oder Benzoesäurerest, ein gegebenenfalls durch Niederalkyl oder Halogen, z.B. Chlor, substituierter heterocyclischer Carbonsäurerest, wie ein entsprechender Pyridin-, Thiophen-, Furan- oder Pyrrol-Carbonsäurerest und insbesondere ein gegebenenfalls durch Halogen, z.B. Chlor, Niederalkoxy, Niederalkanoyloxy, Cyan oder Phenoxy substituiertes niederaliphatisches Acyl, wie entsprechendes Niederalkanoyl, z.B. entsprechendes Acetyl. Ein (Thio)-Kohlensäurehalbesterrest $R_4$ ist Niederalkoxycarbonyl, Arylniederalkoxycarbonyl, z.B. Benzyloxycarbonyl, Cycloalkoxycarbonyl, z.B. Cyclohexyloxycarbonyl, oder Aryloxycarbonyl, z.B. Phenoxycarbonyl (oder entsprechendes -thiocarbonyl).

Ein von einer aromatischen Sulfonsäure abgeleiteter Acylrest $R_4$ ist 1- oder 2-Napththalinsulfonyl oder gegebenenfalls durch Halogen, z.B. Chlor oder Niederalkyl substituiertes Benzolsulfonyl, z.B. Benzolsulfonyl, Chlor-, z.B. o-Chlorbenzolsulfonyl oder Tosyl, z.B. p-Tosyl. Ein von einer niederaliphatischen Sulfonsäure abgeleiteter Acylrest $R_4$

ist vor allem Niederalkylsulfonyl, z.B. Mesyl.

Ein von einem gegebenenfalls N-substituierten, wie N-niederalkylierten Halbamid der Kohlensäure, Thiokohlensäure, Iminokohlensäure oder Schwefelsäure abgeleiteter Acylrest ist vor allem ein am Stickstoffatom gegebenenfalls durch Niederalkyl, Halogen-, z.B. Chlorniederalkyl oder Niederalkoxyniederalkyl, mono- oder di-substituiertes Carbamoyl, Thiocarbamoyl, Iminocarbamoyl oder Sulfamoyl.

Der Niederalkylenrest Y ist gegebenenfalls verzweigt und über einander benachbarte Kohlenstoffatome an die beiden Ring-Stickstoffatome gebunden. Y kann 2-7 Kohlenstoffatome enthalten und beispielsweise ein entsprechend gebundenes Heptylen, z.B. 1.2- oder 2.3-Heptylen, sein, ist aber vorzugsweise ein entsprechender Rest mit 2-4 Kohlenstoffatomen, z.B. 1.2- oder 2.3-Butylen, und insbesondere Aethylen.

Vorstehend wie nachfolgend können die verwendeten Allgemeinbegriffe z.B. folgende Bedeutung haben (sofern nicht ausdrücklich anders definiert).

In dieser Beschreibung der Erfindung bedeutet der Ausdruck "Nieder" in Gruppen und Verbindungen, dass die entsprechende Gruppe, sofern nicht ausdrücklich anders definiert, bis zu 7, bevorzugt bis zu 4 C-Atome enthält.

Niederalkyl ist z.B. Methyl, Aethyl, n-Propyl, Isopropyl, n-Butyl, Isobutyl, sek.Butyl oder tert.Butyl, ferner n-Pentyl, n-Hexyl, Isohexyl oder n-Heptyl.

Niederalkoxy ist z.B. Methoxy, Aethoxy, n-Propyloxy,

Isopropyloxy oder n-Butyloxy. Niederalkylthio ist insbesondere Methylthio und Aethylthio.

Niederalkenyl steht z.B. für Vinyl, 1- oder 2-Propenyl, 1-, 2- oder 3-Buten- oder -Isobutenyl, sowie Pentenyl, Hexenyl oder Heptenyl.

Niederalkinyl ist vor allem Aethinyl, ferner 1- oder 2-Propinyl und 1-, 2- oder 3-Butinyl.

Niederalkylen ist z.B. Aethylen, 1.2- oder 1.3-Propylen oder 1.2- oder 1.4-Butylen, ferner Pentylen, z.B. 1.2- oder 2.3-Pentylen, Hexylen, z.B. 1.2- oder 1.6-Hexylen oder Heptylen, z.B. 1.2-, 2.3- oder 2.7-Heptylen.

Heteroniederalkylen ist entsprechendes Oxa-, Thia-, Aza- oder N-Niederalkylazaniederalkylen, wobei der Heterorest ein nichtendständiges Kohlenstoffatom der Niederalkylenkette ersetzt, z.B. entsprechendes Pentylen, wie 1.5-Pentylen, z.B. 3-Oxa-, 2-Thia- oder 3-Aza-1,5-pentylen.

Niederalkyliertes Amino ist z.B. Niederalkylamino, wie Methylamino oder Aethylamino, oder Diniederalkylamino, wie Dimethylamino oder Diäthylamino. Durch Niederalkylen oder Heteroniederalkylen cyclisch disubstituiertes Amino wie Niederalkylen-, Azaniederalkylen-, N-Niederalkylazaniederalkylen-, Oxaniederalkylen oder Thianiederalkylenamino enthält 4-8, vorzugsweise 5 oder 6 Ringatome und ist beispielsweise Pyrrolidino, Piperidino, Piperazino, N-Methylpiperazino, Morpholino oder Thiamorpholino.

Niederaliphatische Acylreste sind gegebenenfalls, durch Halogen, z.B. Chlor bis zu dreifach, ferner durch Hydroxy, Niederalkoxy, Phenoxy, Cyan, funktionell abgewandeltes Carboxy oder gegebenenfalls substituiertes Amino substituierte Niederalkan- oder Niederalkencarbonsäure-Reste, wie entsprechende Formyl-, Propionyl-, Butyryl-, Isobutyryl-, Valeryl-, Acryl-, Methacryl- und insbesondere entsprechende Acetylreste, beispielsweise Acetyl, Methoxyacetyl, Acetoxyacetyl, Phenoxyacetyl, Cyanacetyl, Chloracetyl, Trichloracetyl oder Glycyl.

Aromatische Acylreste sind z.B. Benzoyl, Hydroxybenzoyl, z.B. p-Hydroxybenzoyl, Chlorbenzoyl, z.B. 3-Chlorbenzoyl oder Naphthoyl. Halbesterreste der Kohlensäure sind z.B. Niederalkoxy-, z.B. Methoxy-, Aethoxy- oder tert-Butoxycarbonyl, Cycloalkoxy-, z.B. Cyclohexyloxycarbonyl, Phenylniederalkoxy-, z.B. Benzyloxycarbonyl oder Phenoxycarbonyl. Halbamidreste der Kohlensäure sind z.B. Carbamoyl, N-Methyl- und N,N-Dimethylcarbamoyl, Pyrrolidinocarbonyl, Piperidinocarbonyl und Morpholinocarbonyl.

Funktionell abgewandeltes Carboxyl ist z.B. verestertes Carboxyl, wie gegebenenfalls substituiertes, z.B. durch Chlor oder Phenyl (bis zu dreifach) substituiertes Niederalkoxycarbonyl, z.B. Methoxy- oder Aethoxycarbonyl, ferner gegebenenfalls substituiertes, wie niederalkyliertes, z.B. diniederalkyliertes Carbamoyl, insbesondere Carbamoyl oder N,N-Dimethylcarbamoyl, sowie Cyan (als inneres Anhydrid von Carbamoyl).

Halogen steht für Fluor oder Brom, vorzugsweise jedoch für Chlor.

Salze sind insbesondere diejenigen von Verbindungen der Formel I mit sauren Gruppen, z.B. mit freien Carboxylgruppen. Solche Salze sind in erster Linie Metall- oder Ammoniumsalze, wie Alkalimetall- und Erdalkalimetall-, z.B. Natrium-, Kalium-, Magnesium- oder Calciumsalze, sowie Ammoniumsalze mit Ammoniak oder geeigneten organischen Aminen, wobei in erster Linie aliphatische, cycloaliphatische, cycloaliphatisch-aliphatische oder araliphatische primäre, sekundäre oder tertiäre Mono-, Di- oder Polyamine, sowie heterocyclische Basen für die Salzbildung in Frage kommen, wie Niederalkylamine, z.B. Triäthylamin, Hydroxyniederalkylamine, z.B. 2-Hydroxyäthylamin, Bis-(2-hydroxyäthyl)-amin oder Tris-(2-hydroxyäthyl)-amin, basische aliphatische Ester von Carbonsäuren, z.B. 4-Aminobenzoesäure-2-diäthylaminoäthylester, Niederalkylenamine, z.B. 1-Aethyl-piperidin, Cycloalkylamine, z.B. Dicyclohexylamin, oder Benzylamine, z.B. N,N'-Dibenzyl-äthylendiamin, ferner Basen vom Pyridintyp, z.B. Pyridin, Collidin oder Chinolin. Verbindungen der Formel I mit einer basischen Gruppe können Säureadditionssalze, z.B. mit anorganischen Säuren, wie Salzsäure, Schwefelsäure, oder Phosphorsäure, oder mit geeigneten organischen Carbon- oder Sulfonsäuren sowie mit Aminosäuren, wie Arginin und Lysin, bilden. Bei Anwesenheit von mehreren sauren oder basischen Gruppen können Mono- oder Polysalze gebildet werden. Verbindungen der Formel I mit einer freien Carboxylgruppe und freien Aminogruppe können auch in Form von inneren Salzen, d.h. in zwitterionischer Form vorliegen.

Zur Isolierung oder Reinigung können auch pharmazeutisch ungeeignete, z.B. schwerlösliche Salze Verwendung finden, z.B. solche mit schweren Erdalkali- oder Schwermetallen oder auch Salze mit starken organischen oder anorganischen Säuren,

.. solche von Säuren, die bei der Abspaltung von Schutzgruppen verwendet werden, z.B. solche mit Trifluoressigsäure. Zur therapeutischen Anwendung gelangen nur pharmazeutisch unbedenkliche Salze, die deshalb bevorzugt werden.

Die Verbindungen der Formel I und ihre pharmazeutisch verwendbaren, nicht-toxischen Salze sind wertvolle, antibiotisch wirksame Substanzen, die insbesondere als antibakterielle Antibiotika verwendet werden können. Beispielsweise sind sie <u>in vitro</u> gegen gram-positive und gram-negative Mikroorganismen, wie gegen gram-positive und gram-negative Kokken, z.B. Staphylokokken, Streptokokken, Mikrokokken oder Neisserien im Dosisbereich von 0.05-64 mcg/ml und insbesondere gegen gram-negative Stäbchen, z.B. Enterobakterien, Haemophilus- und Pseudomonas-Arten im Dosisbereich von 0.05 bis 64 mcg/ml wirksam.

Diese antimikrobielle Wirkung lässt sich auch <u>in vivo</u>, z.B. an der systemisch mit E. coli infizierten Maus nach subcutaner Injektion, z.B. im Bereich ab ca. 3 mg/kg, nachweisen.

Die neuen Verbindungen können deshalb entsprechend, z.B. in Form von antibiotisch wirksamen Präparaten, zur Behandlung von durch gram-positive oder gram-negative Mikroorganismen, insbesondere durch von Enterobakterien, verursachten Infektionen sowie zu Desinfektionszwecken Verwendung finden.

Die vorliegende Erfindung betrifft in erster Linie Verbindungen der Formel I, worin $R_1$ Wasserstoff, Halogen, Niederalkoxy, Niederalkylthio, gegebenenfalls durch Halogen, Niederalkoxy, Niederalkyl oder Nitro substituiertes Phenylthio, ein gegebenenfalls substituiertes und/oder gegebenenfalls benzo-kondensiertes monocyclisches, fünf- oder sechsgliedriges, aromatisches oder partiell gesättigtes Heterocyclylthio, wobei der Heteroring über ein Ring-Kohlenstoffatom an das Thioschwefelatom gebunden ist und mindestens ein Heteroatom aus der Gruppe N, O oder S sowie gegebenenfalls 1-3 weitere Ring N-Atome enthält, oder einen Carbonylmethylrest $-CH_2-C(=O)-R_6$, worin $R_6$ Wasserstoff, Hydroxy, Niederalkyl, Niederalkoxy oder eine gegebenenfalls niederalkyliertes oder durch Niederalkylen oder Oxa-, Thia-, Aza- oder N-Niederalkylazaniederalkylen cyclisch disubstituiertes Amino ist, bedeutet, $R_2$ Hydroxy oder, zusammen mit der Carbonylgruppe, eine physiologisch spaltbare veresterte Carboxylgruppe der Teilformel (A) darstellt, worin $R_7$ gegebenenfalls durch Niederalkoxy oder Diniederalkylamino substituiertes Niederalkanoyl, Niederalkoxycarbonyl, Niederalkylthiocarbonyl, Carbamoyl oder gegebenenfalls durch Niederalkoxy substituiertes Niederalkyl, $R_8$ Wasserstoff oder Methyl, und T Sauerstoff oder Schwefel darstellen oder worin die Teilformel (A) eine 5-Oxodihydro-2-furfuryloxycarbonyl-, 5-Oxo-tetrahydro-2-furfuryloxycarbonyl-, Phthalidyloxycarbonyl- oder 5,6-Dimethoxyphthalidyloxycarbonylgruppe oder die Succinimidomethoxycarbonyl-, Saccharimidomethoxycarbonyl- oder Phthalimidomethoxycarbonylgruppe bildet, $R_3$ für Phenyl, durch Hydroxy, Niederalkanoyloxy, Carbamoyloxy, Halogen, Niederalkylsulfonylamino, Cyan oder Nitro ein- oder zweifach substituiertes Phenyl, Thienyl, Furyl, Cyclohexenyl, Cyclohexadienyl, Tetrazolyl, oder Aminothiazolyl steht, X Sauerstoff oder Schwefel und n 1 oder 2 ist, $R_4$ Wasserstoff, gegebenenfalls durch Hydroxy, Niederalkanoyloxy, Niederalkoxycarbonyloxy, Phenoxycarbonyloxy, Niederalkoxy, Niederalkylthio, Halogen, gegebenenfalls funktionell abgewandeltes Carboxy, oder gegebenenfalls mono- oder diniederalkyliertes Amino substituiertes Niederalkyl sowie Niederalkenyl oder Acyl bedeutet und Y ein Aethylenrest ist, Salze von solchen Verbindungen mit salzbildenden Gruppen sowie Isomere von solchen Verbindungen der Formel I.

Besonders hervorzuheben sind Verbindungen der Formel I, worin $R_1$ Wasserstoff, Halogen, Niederalkoxy, Niederalkylthio, gegebenenfalls durch Niederalkyl, Chlor oder Fluor substituiertes Phenylthio, ein gegebenenfalls substituiertes monocyclisches, fünfgliedriges, aromatisches Heterocyclylthio, wobei der Heteroring über ein Ring-Kohlenstoffatom an das Thioschwefelatom gebunden ist und mindestens ein Sauerstoff- oder Schwefel- oder zwei N-Atome, sowie gegebenenfalls 1-2 weitere Ring-N-atome enthält, oder ein Carbonylmethylrest $-CH_2-C(=O)-R_6$, worin $R_6$ Hydroxy, Niederalkyl, Niederalkoxy, Amino, Niederalkylamino, Diniederalkylamino oder Niederalkylenimino bedeutet, ist, $R_2$ Hydroxy oder zusammen mit der Carbonylgruppe, eine physiologisch spaltbare veresterte Carboxylgruppe der Teilformel (A), worin $R_7$ Niederalkanoyl, Niederalkyl oder Niederalkoxyniederalkyl, $R_8$ Wasserstoff und T Sauerstoff darstellt, bedeutet oder die Teilformel (A) eine 5-Oxo-dihydro-2-furfuryloxycarbonyl-, 5-Oxo-tetrahydro-2-furfuryloxycarbonyl-, Phthalidyloxycarbonyl- oder 5,6-Dimethoxyphthalidyloxycarbonylgruppe oder die Succinimidomethoxycarbonyl, Saccharimidomethoxycarbonyl- oder Phthalimidomethoxycarbonylgruppe bildet, $R_3$ Phenyl, Hydroxy-, Carbamoyloxy-, Acetoxy-, Niederalkylsulfonylamino, Cyan- oder Chlor-hydroxy-phenyl, Thienyl, Furyl, Cyclohexenyl, Cyclohexadienyl, Tetrazolyl oder Aminothiazolyl darstellt, X Sauerstoff oder Schwefel und n 1 oder 2 ist, $R_4$ Wasserstoff, gegebenenfalls durch Hydroxy, Niederalkanoyloxy, Niederalkoxycarbonyloxy, Niederalkoxy, Niederalkylthio, Halogen, z.B. Chlor, Carboxy, Amino oder Diniederalkylamino substituiertes Niederalkyl, sowie Niederalkenyl oder gegebenenfalls durch Halogen, z.B. Chlor, Niederalkoxy, Niederalkanoyloxy, Cyan oder Phenoxy substituiertes Niederalkanoyl, gegebenenfalls durch Niederalkyl, Hydroxy, Niederalkoxy oder Chlor substituiertes Benzoyl, einen gegebenenfalls durch Hydroxy oder Chlor substituierten Pyridin-, Pyrimidin-oder Pyrazincarbonsäurerest, Niederalkoxycarbonyl, gegebenenfalls durch Halogen, z.B. Chlor oder Niederalkyl substituiertes Aryl-, wie Benzol- oder Napththalinsulfonyl, gegebenenfalls am N-Atom niederalkyliertes Carbamoyl, Thiocarbamoyl, Iminocarbamoyl (=Guanyl) oder Sulfamoyl, bedeutet, und Y ein Aethylenrest ist, sowie pharmazeutisch verwendbare Salze von solchen Verbindungen mit einer salzbildenden Gruppe, sowie Isomere davon.

Die Erfindung betrifft vor allem Verbindungen der Formel I, worin $R_1$ Wasserstoff, Fluor, Chlor, Niederalkoxy, Niederalkylthio, Phenylthio, Halogenphenylthio, z.B. Chlorphenylthio oder Fluorphenylthio, durch Halogen, gegebenenfalls niederalkyliertes Amino, Carboxy oder Sulfo gegebenenfalls substituiertes Niederalkyl, Phenyl, Halogen, Carboxy oder gegebenenfalls niederalkyliertes Amino substituiertes Thiazolylthio, Oxadiazolyl-, z.B. 1.3.4-Oxadiazolyl oder 1.2.4-Oxadiazolylthio, Thiadiazolyl-, z.B. 1.3.4-Thiadiazolyl- oder 1.2.4-Thiadiazolylthio, Triazolyl-, z.B. 1.2.3-Triazolyl- oder 1.2.4-Triazolylthio oder entsprechendes Tetrazolyl-, z.B. 5-Tetrazolylthio, Carboxymethyl, Niederalkoxycarbonylmethyl, Niederalkylcarbonylmethyl oder Carbamoylmethyl bedeutet, $R_2$ für Hydroxy, Niederalkanoyloxymethoxy, z.B. Pivaloyloxymethoxy, α-Aminoniederalkanoyloxymethoxy, z.B. Glycyloxymethoxy, Niederalkoxymethoxy, z.B. Methoxymethoxy, Carbamoyloxymethoxy oder 2-Phthalidyloxymethoxy steht, $R_3$ Phenyl, p-Hydroxy-, p-Acetoxy-, Niederalkylsulfonylamino, p-Carbamoyloxy- oder 3-Chlor-4-hydroxyphenyl, 1-Cyclohexenyl, 1.4-Cyclohexadienyl, 2- oder 3-Thienyl, 2- oder 3-Furyl, oder 2-Amino-4-thiazolyl darstellt, X Sauerstoff oder Schwefel und n 1 oder 2 ist und $R_4$ für Niederalkyl, Hydroxyniederalkyl, Niederalkoxyniederalkyl, Halogenniederalkyl, Carboxyniederalkyl, gegebenenfalls niederalkyliertes

Aminoniederalkyl, Niederalkenyl, Niederalkanoyl, Carbamoyl, Niederalkoxycarbonyl, Thiocarbamoyl, Guanyl, Arylsulfonyl, Niederalkansulfonyl oder Sulfamoyl steht, pharmazeutisch verwendbare Salze von solchen Verbindungen mit salzbildenden Gruppen, sowie Isomere davon.

In erster Linie betrifft die Erfindung Verbindungen der Formel I, worin $R_1$ Wasserstoff, Niederalkoxy mit bis zu 4 Kohlenstoffatomen, z.B. Methoxy, Niederalkylthio mit bis zu 4 Kohlenstoffatomen, z.B. Methylthio, Phenylthio, gegebenenfalls durch Niederalkyl, z.B. Methyl, Carboxyniederalkyl, z.B. Carboxymethyl, Sulfoniederalkyl, z.B. Sulfomethyl, Aminoniederalkyl, z.B. 2-Aminoäthyl, Diniederalkylaminoniederalkyl, z.B. 2-Diäthylaminoäthyl, Niederalkoxyniederalkyl, z.B. 2-Methoxyäthyl, oder Halogenniederalkyl, z.B. 2-Chloräthyl, substituiertes Oxadiazolylthio, Thiadiazolylthio, Triazolylthio oder Tetrazolylthio, z.B. entsprechendes 1.2.4- oder 1.3.4-Oxadiazolylthio, -Thiadiazolylthio oder -Triazolylthio oder entsprechendes 5-Tetrazolylthio, Carboxymethyl, Niederalkoxycarbonylmethyl, z.B. Methoxycarbonylmethyl, Niederalkylcarbonylmethyl, z.B. Methylcarbonylmethyl, oder Carbamoylmethyl bedeutet, $R_2$ Hydroxy, Niederalkoxymethoxy, z.B. Methoxymethoxy, Niederalkanoyloxymethoxy, z.B. Pivaloyloxymethoxy oder 2-Phthalidyloxymethoxy darstellt, $R_3$ für Phenyl, m-Methylsulfonylaminophenyl, p-Hydroxyphenyl, 1.4-Cyclohexadienyl, 2- oder 3-Thienyl, 2- oder 3-Furyl, oder 2-Amino-4-thiazolyl steht, X Sauerstoff oder Schwefel und n 1 oder 2 ist, und $R_4$ für Niederalkyl mit 1-4 Kohlenstoffatomen, z.B. Aethyl oder Isobutyl, gegebenenfalls, z.B. in 1-Stellung, durch Hydroxy, Halogen, z.B. Chlor, Carboxy, Niederalkoxy mit bis zu 4 Kohlenstoffatomen, z.B. Methoxy, oder Amino substituiertes Niederalkyl mit 1-4 Kohlenstoffatomen, z.B. entsprechendes Aethyl oder Isobutyl, Niederalkenyl mit 2-5 Kohlenstoffatomen, wie Isobutenyl, z.B. 1-Isobutenyl, oder Niederalkanoyl, z.B. Acetyl, Carbamoyl, Guanyl,

Niederalkylsulfonyl, z.B. Mesyl, oder Sulfamoyl steht, pharmazeutisch verwendbare Salze von solchen Verbindungen mit salzbildenden Gruppen, sowie Isomere davon.

Die Erfindung betrifft insbesondere die in den Beispielen beschriebenen Verbindungen der Formel I und pharmazeutisch verwendbare Salze dieser Verbindungen, sowie Isomere davon.

Die Verbindungen der Formel I mit isomeriefähigen Gruppen können auch als Isomere oder Gemisch von Isomeren vorliegen, z.B. kann eine 2-Amino-4-thiazolylgruppe $R_3$ auch ganz oder in einem Teil der Moleküle der Formel I als 2-Imino-4-thiazolinylgruppe vorliegen und Verbindungen der Formel I mit einem Asymmetriezentrum können als Racemat oder in Form der reinen optischen oder sterischen Antipoden erhalten werden.

Die Verbindungen der vorliegenden Erfindung können nach an sich bekannten Verfahren erhalten werden.

So können die Verbindungen der Formel I hergestellt werden, indem man

a) in einer Verbindung der Formel

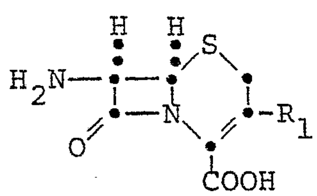

(II),

worin die Aminogruppe gegebenenfalls durch eine die Acylierung erlaubende Gruppe substituiert ist, und worin die 4-Carboxylgruppe und gegebenenfalls im Rest $R_1$ vorhandene weitere funktionelle Gruppen in geschützter Form vorliegen können, die Aminogruppe durch Behandeln mit einem, den Acylrest einer Carbonsäure der Formel

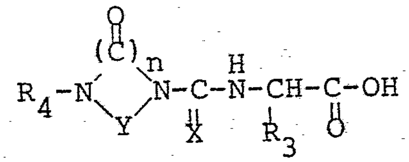

(III)

einführenden Acylierungsmittel, worin gegebenenfalls vorhandene weitere funktionelle Gruppen in geschützter Form vorliegen, acyliert, oder indem man

b) in einer Verbindung der Formel

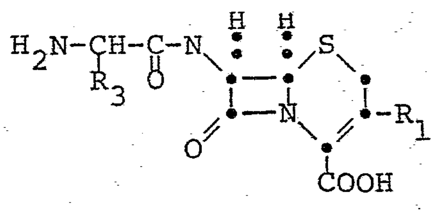

(IV),

worin die Aminogruppe gegebenenfalls durch eine, die Acylierung erlaubende Gruppe substituiert ist, und worin die 4-Carboxylgruppe und gegebenenfalls im Rest $R_1$ und im Rest $R_3$ vorhandene weitere funktionelle Gruppen in geschützter Form vorliegen können, die Aminogruppe durch Behandeln mit einem den entsprechenden Acylrest einer Kohlen- oder Thiokohlensäure der Formel

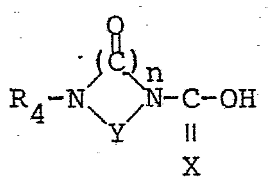

(V)

einführenden Acylierungsmittel, worin im Rest $R_4$ gegebenenfalls vorhandene funktionelle Gruppen in geschützter Form vorliegen können, acyliert oder indem man

c) eine Kohlensäure- oder Thiokohlensäureverbindung der Formel

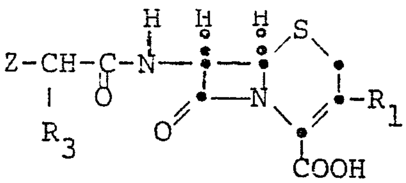

(VI),

worin die 4-Carboxylgruppe und gegebenenfalls in den Resten $R_1$ und $R_3$ vorhandene funktionelle Gruppen in geschützter Form vorliegen können, und worin Z eine von einem Amid der Kohlen- oder Thiokohlensäure abgeleitete, reaktive, mit Aminen unter Ausbildung einer Harnstoff- oder Thioharnstoffgruppierung reagierende Gruppe, z.B. eine Halogencarbonylamino-, Halogenthiocarbonylamino-, Isocyanato- oder Isothiocyanatogruppe ist, mit einem sekundären Amid der Formel

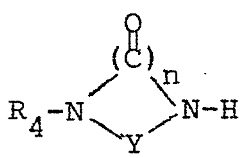

(VII),

worin im Rest $R_4$ gegebenenfalls vorhandene funktionelle Gruppen geschützt sein können und die sekundäre Amidogruppe in einer die Acylierung erlaubenden Form vorliegt, reagieren lässt, oder indem man

d) die unter b) bzw. c) genannte Acylierung in situ vornimmt, indem man eine Aminoverbindung der Formel IV und eine Amidoverbindung der Formel VII, beide mit der oben angegebenen Bedeutung, gleichzeitig oder nacheinander auf ein bivalentes reaktives Derivat der Kohlen- oder Thiokohlensäure einwirken lässt, oder indem man

e) eine Verbindung der Formel

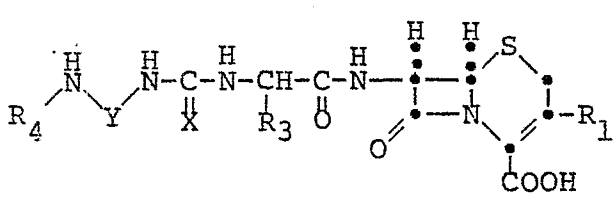

(VIII),

worin die 4-Carboxylgruppe und übrige gegebenenfalls vorhandene funtkionelle Gruppen geschützt sind, und die später den Piperazinring bildende sekundären Amino- bzw. Amidogruppen gegebenenfalls durch Gruppen substituiert sind, die die N-Acylierung erlauben, mit einem bivalenten acylierenden Derivat der Kohlensäure oder Oxalsäure unter Ringschluss diacyliert, oder indem man

f) zur Herstellung von Verbindungen der Formel I, worin $R_1$ eine verätherte Hydroxygruppe oder Halogen bedeutet, und worin funktionelle Gruppen geschützt sind, eine Verbindung der Formel

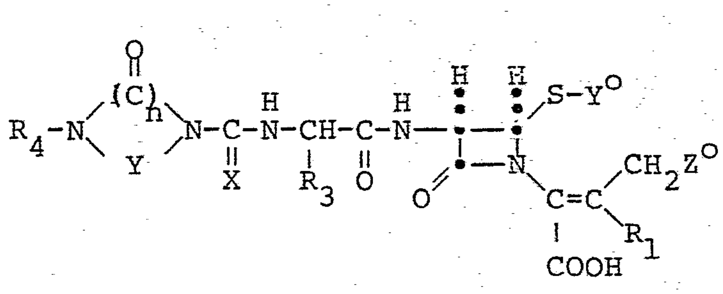

(IX),

worin $R_1$ die vorstehend genannte Bedeutung hat, $Y^o$ eine Abgangsgruppe darstellt und $Z^o$ Wasserstoff oder Halogen bedeutet, und worin funktionelle Gruppen geschützt sind, unter Abspaltung von $Y^o$ und $Z^o$ ringschliesst oder indem man

g) eine 2-Cephemverbindung der Formel

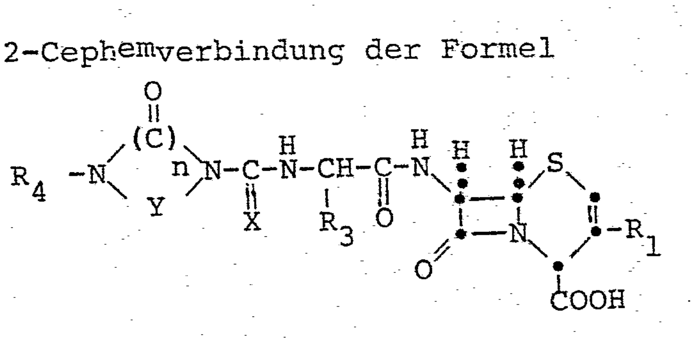

(X),

worin funktionelle Gruppen geschützt sind, zur entsprechenden 3-Cephemverbindung der Formel I isomerisiert, oder indem man

h) in einer Verbindung der Formel

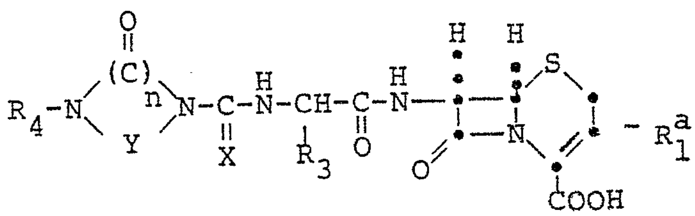

(XI),

worin $R_1^a$ eine freie oder sulfonylierte Hydroxygruppe ist, und worin andere funktionelle Gruppen, insbesondere die 4-Carboxylgruppe, vorzugsweise geschützt sind, $R_1^a$ gegen Halogen ausgetauscht, oder indem man

i) in einer Verbindung der Formel XI, worin $R_1^a$ eine freie Hydroxygruppe ist und worin andere funktionelle Gruppen, insbesondere die 4-Carboxylgruppe, geschützt sind, die Hydroxygruppe veräthert, oder indem man

j) in einer Verbindung der Formel XI, worin funktionelle Gruppen vorzugsweise geschützt sind, eine durch ein Thiol substituierbare veresterte oder verätherte Hydroxygruppe $R_1^a$ durch Behandeln mit einem Thiol der Formel $R_1$-H in eine verätherte Mercaptogruppe $R_1$ überführt, oder indem man

k) in einer Verbindung der Formel XI, worin funktionelle Gruppen vorzugsweise geschützt sind, und worin $R_1^a$ eine Formylgruppe bedeutet, diese in Gegenwart von Decarbonylierungs-Katalysatoren zu einer Verbindung der Formel I, worin $R_1$ Wasserstoff bedeutet, decarbonyliert, oder indem man

l) in einer Verbindung der Formel XI, worin $R_1^a$ freies oder verestertes Hydroxy oder ein gegebenenfalls niederalkyliertes oder durch Niederalkylen oder Heteroniederalkylen cyclisch disubstituiertes Amino ist, und worin funktionelle Gruppen

gegebenenfalls geschützt sind, die Gruppe $R_1^a$ durch Wasserstoff ersetzt, oder indem man

m) eine Verbindung der Formel XI, worin $R_1^a$ eine freie Hydroxygruppe ist und gegebenenfalls vorhandene weitere reaktive Gruppen und insbesondere die 4-Carboxylgruppe in geschützter Form vorliegen, oder ein 1-Oxid davon, mit einem Ylid der Formel

$$\overset{\oplus}{X} — \overset{\ominus}{CH} — C(=O) — R_6 \qquad (XII),$$

worin $R_6$ die unter Formel I genannte Bedeutung hat, wobei funktionelle Gruppen vorzugsweise geschützt vorliegen und $X^{\oplus}$ eine dreifach substituierte Phosphoniumgruppe oder eine zweifach veresterte Phosphonogruppe zusammen mit einem Kation bedeutet, umsetzt und, wie unter näher erläutert, in einer nach einem der Verfahren a) - m) erhaltenen Verbindung der allgemeinen Formel I mit geschützten funktionellen Gruppen, die Schutzgruppen entfernt und/oder, wenn erwünscht, eine erhaltene Verbindung der Formel I in eine andere erfindungsgemässe Verbindung der Formel I umwandelt und/oder, wennn erwünscht, ein erhaltenenes Salz in ein anderes Salz oder die freie Verbindung oder eine erhaltene freie Verbindung in ein Salz umwandelt.

Diese zuletzt genannten Verfahren werden unter "Nachoperationen" beschrieben.

Die in den Resten $R_1$ bis $R_8$ vorhandenen freien funktionellen Gruppen können bei der Herstellung der Verbindungen der Formel I intermediär oder permanent geschützt sein, z.B. durch Silyl-, wie Trimethylsilyl- oder andere, gegen Solvolyse stabilere Schutzgruppen, die, falls (z.B. zwecks Reinigung von Zwischen- oder Endprodukten) erwünscht oder erforderlich, in separaten Reaktionsstufen eingeführt und/oder wieder abgespalten werden können.

Derartige Schutzgruppen, ihre Einführung sowie ihre Abspaltung, sind bekannt und beispielsweise beschrieben in "Protective Groups in Organic Chemistry", Plenum Press, London, New York, 1973, ferner in "The Peptides", Vol. I, Schröder and Lübke, Academic Press, London, New York, 1965, sowie in "Methoden der organischen Chemie", Houben-Weyl, 4. Auflage, Bd. 15/1, Georg Thieme Verlag, Stuttgart, 1974.

Eine geschützte Aminogruppe kann z.B. in Form einer leicht spaltbaren Acylamino-, Mono-, Di- oder Triarylmethylamino-, verätherten Mercaptoamino-, 1-Acyl-2-niederalkylidenamino-, Silyl- oder Stannylaminogruppe vorliegen.

In einer entsprechenden Acylaminogruppe ist Acyl beispielsweise der Acylrest einer organischen Carbonsäure mit z.B. bis zu 18 C-Atomen, insbesondere einer gegebenenfalls, z.B. durch Halogen oder Aryl, substituierten aliphatischen Carbonsäure oder gegebenenfalls, z.B. durch Halogen, Niederalkoxy oder Nitro, substituierten aromatischen Carbonsäure, oder eines Kohlensäurehalbesters. Solche Acylgruppen sind beispielsweise Niederalkanoyl, wie Formyl, Acetyl, Propionyl, Halogenniederalkanoyl, wie 2-Halogenacetyl, insbesondere 2-Chlor-, 2-Brom-, 2-Jod-, 2,2-Dichlor- oder 2,2,2-Trichloracetyl, Phenylacetyl, Phenoxyacetyl, Thienylacetyl, Benzoyl, 4-Chlor-, 4-Methoxy- oder 4-Nitrobenzoyl, Niederalkoxycarbonyl, insbesondere tert.-Niederalkoxycarbonyl, z.B. tert.-Butyloxycarbonyl, Polycycloalkoxycarbonyl, z.B. Adamantyloxycarbonyl, Arylmethoxycarbonyl, worin Aryl vorzugsweise einen oder zwei, gegebenenfalls, z.B. durch Niederalkyl, insbesondere tert.-Niederalkyl, z.B. tert.-Butyl, Niederalkoxy, wie Methoxy, Hydroxy, Halogen, z.B. Chlor, und/oder Nitro, mono- oder polysubstituierte Phenylreste darstellt, wie gegebenenfalls substituiertes Benzyloxycarbonyl, z.B. 4-Nitro-benzyloxycarbonyl,

oder substituiertes Diphenylmethoxycarbonyl, z.B. Benzhydryloxycarbonyl oder Di-(4-methoxyphenyl)-methoxycarbonyl, oder 2-Halogen-niederalkoxycarbonyl, z.B. 2,2,2-Trichloräthoxycarbonyl, 2-Chloräthoxycarbonyl, 2-Bromäthoxycarbonyl oder 2-Jodäthoxycarbonyl, 2-($S_1$)($S_2$)($S_3$)-Silyläthoxycarbonyl, worin die Substituenten $S_1$, $S_2$ und $S_3$ unabhängig voneinander je einen gegebenenfalls substituierten, z.B. durch Niederalkyl, Niederalkoxy, Aryl, Halogen oder Nitro, substituierten, aliphatischen, araliphatischen, cycloaliphatischen oder aromatischen Kohlenwasserstoffrest mit z.B. bis zu 15 C-Atomen, wie einen entsprechenden gegebenenfalls substituierten Niederalkyl-, Arylniederalkyl-, Cycloalkyl- oder Arylrest, bedeuten, z.B. 2-Triniederalkylsilyläthoxycarbonyl, wie 2-Trimethylsilyläthoxycarbonyl oder 2-(Dibutyl-methyl-silyl)-äthoxycarbonyl, oder 2-Triarylsilyläthoxycarbonyl, wie 2-Triphenylsilyläthoxycarbonyl, oder Acylmethoxycarbonyl, ~~wie Triphenylsil~~yläthoxymethoxycarbonyl, worin die Aroylgruppe vorzugsweise gegebenenfalls, z.B. durch Halogen, wie Brom, substituiertes Benzoyl darstellt, z.B. Phenacyloxycarbonyl.

Weitere Acylgruppen in Acylaminogruppen sind Reste organischer Phosphor-, Phosphon- oder Phosphinsäuren der Formel (R')(R'')P(=O)-, worin R' und R'' unabhängig voneinander eine durch einen Kohlenwasserstoffrest verätherte Hydroxygruppe oder einen Kohlenwasserstoffrest bedeuten, wobei die Kohlenwasserstoffreste bevorzugt bis zu 15 C-Atome enthalten, beispielsweise aliphatischer, araliphatischer, cycloaliphatischer oder aromatischer Natur sind, gegebenenfalls z.B. durch Niederalkyl, Niederalkoxy, Aryl, Halogen oder Nitro substituiert sind, und beispielsweise Alkyl, insbesondere Niederalkyl, wie Methyl, Aethyl, Propyl, Isopropyl oder Butyl, Phenylniederalkyl, wie Benzyl, p-Nitro- oder p-Chlorbenzyl, Cycloalkyl, wie Cyclopropyl,

Cyclopentyl, Cyclohexyl oder Cycloheptyl, oder Homoaryl, wie Phenyl, o-, m- oder p-Tolyl, p-Methoxyphenyl, p-Biphenylyl, p-Chlorphenyl oder p-Nitrophenyl, bedeuten. Solche Reste sind beispielsweise Dimethylphosphoryl, Diäthylphosphoryl, Dipropylphosphoryl, Diisopropylphosphoryl, Dibenzylphosphoryl, Di-p-nitrobenzylphosphoryl, Dicyclohexylphosphoryl, Diphenylphosphoryl, Phenoxy-phenyl-phosphonyl, Diäthylphosphinyl und Diphenylphosphinyl.

In einer Mono-, Di- oder Triarylmethylaminogruppe sind die Arylreste insbesondere gegebenenfalls substituierte Phenylreste. Solche Gruppen sind beispielsweise Benzyl-, Diphenylmethyl- oder Tritylamino.

Eine verätherte Mercaptogruppe in einer mit einem solchen Rest geschützten Aminogruppe ist in erster Linie Arylthio oder Arylniederalkylthio, worin Aryl insbesondere gegebenenfalls, z.B. durch Niederalkyl, wie Methyl oder tert.-Butyl, Niederalkoxy, wie Methoxy, Halogen, wie Chlor, und/oder Nitro substituiertes Phenyl ist. Eine entsprechende Aminoschutzgruppe ist z.B. 4-Nitrophenylthio.

In einem als Aminoschutzgruppe verwendbaren 1-Acyl-2-niederalkylidenrest ist Acyl vorzugsweise der entsprechende Rest einer Niederalkancarbonsäure, einer gegebenenfalls, z.B. durch Niederalkyl, wie Methyl oder tert.-Butyl, Niederalkoxy, wie Methoxy, Halogen, wie Chlor, und/oder Nitro substituierten Benzoesäure oder eines Kohlensäurehalbesters, wie eines Kohlensäure-niederalkylhalbesters. Entsprechende Schutzgruppen sind in erster Linie 1-Niederalkanoyl-2-propyliden, z.B. 1-Acetyl-2-propyliden, oder 1-Niederalkoxycarbonyl-2-propyliden, z.B. 1-Aethoxycarbonyl-2-propyliden.

Eine Silyl- oder Stannylaminogruppe ist in erster Linie eine organische Silyl- bzw. Stannylaminogruppe, worin das Silicium- bzw. Zinnatom vorzugsweise Niederalkyl, insbesondere Methyl, ferner Niederalkoxy, z.B. Methoxy, und/oder Halogen, z.B. Chlor, als Substituenten enthält. Entsprechende Silyl- oder Stannylgruppen sind in erster Linie Triniederalkylsilyl, insbesondere Trimethylsilyl, ferner Dimethyl-tert.-butyl-silyl, Niederalkoxy-niederalkyl-halogen-silyl, z.B. Methoxy-methyl-chlor-silyl, oder Diniederalkyl-halogensilyl, z.B. Dimethyl-chlor-silyl, oder entsprechend substituiertes Stannyl, z.B. Tri-n-butylstannyl.

Eine Aminogruppe kann auch in protonierter Form geschützt werden; als Anionen kommen in erster Linie diejenigen von starken anorganischen Säuren, wie von Halogenwasserstoffsäuren, z.B. das Chlor- oder Bromanion, oder von Sulfonsäure, wie p-Toluolsulfonsäure, in Frage.

Bevorzugte Aminoschutzgruppen sind die Acylreste von Kohlensäurehalbestern, insbesondere tert.-Niederalkoxycarbonyl, gegebenenfalls, z.B. wie angegeben substituiertes Benzyloxycarbonyl oder Diphenylmethoxycarbonyl, oder 2-Halogen-niederalkoxycarbonyl, wie 2,2,2-Trichloräthoxycarbonyl.

Hydroxyschutzgruppen sind z.B. Acylreste, wie 2,2-Dichloracetyl oder insbesondere einer der im Zusammenhang mit einer geschützten Aminogruppe genannten Acylreste von Kohlensäurehalbestern, insbesondere 2,2,2-Trichloräthoxycarbonyl, oder organischen Silyl- oder Stannylreste, ferner leicht abspaltbare 2-oxa- oder 2-thiaaliphatische oder -cycloaliphatische Kohlenwasserstoffreste, in erster Linie 1-Niederalkoxy-niederalkyl oder 1-Niederalkylthio-niederalkyl, z.B. 1-Methoxyäthyl, 1-Aethoxy-äthyl, 1-Methylthio-

äthyl oder 1-Aethylthioäthyl, oder 2-Oxa- oder 2-Thiacycloniederalkyl mit 5-7 Ringatomen, z.B. 2-Tetrahydrofuryl oder 2-Tetrahydropyranyl oder entsprechende Thiaanaloge, sowie leicht abspaltbare, gegebenenfalls substituierte α-Phenylniederalkylreste, wie gegebenenfalls substituiertes Benzyl oder Diphenylmethyl, wobei als Substituenten der Phenylreste z.B. Halogen, wie Chlor, Niederalkoxy, wie Methoxy und/oder Nitro in Frage kommen.

Geschützte Carboxylgruppen sind solche, die sich ohne unerwünschte Nebenreaktionen unter vorhersehbaren, möglichst schonenden Bedingungen, beispielsweise solvolytisch, insbesondere hydrolytisch, reduktiv, z.B. hydrogenolytisch oder photolytisch wieder in die freien Carboxylgruppen umwandeln lassen.

Carboxylgruppen sind üblicherweise in veresterter Form geschützt, wobei solche Estergruppierungen unter schonenden Bedingungen entweder in einem separaten Reaktionsschritt abgespalten werden oder die Carboxylgruppe nur intermediär schützen und dann bereits in situ, spätestens bei der Aufarbeitung der Reaktionsprodukte, von selbst abgespalten. Als geeignete geschützte Carboxylgruppen kommen an sich auch die oben definierten, unter physiologischen Bedingungen (z.B. hydrolytisch) spaltbaren geschützten Carboxylgruppen der Formel $-C(=O)-OR_5$ in Frage, beispielsweise Methoxymethoxycarbonyl oder Pivaloyloxymethoxycarbonyl. Ueblicherweise sind für präparative Zwecke geschützte Carboxylgruppen jedoch davon verschieden.

Für präparative Zwecke geschützte Carboxylgruppen können z.B. Niederalkoxycarbonyl, wie tert.-Niederalkoxycarbonyl, z.B. tert.-Butyloxycarbonyl, Polycycloalkoxycarbonyl, z.B. Adamantyloxycarbonyl, Arylmethoxycarbonyl, worin Aryl vorzugsweise einen oder zwei gegebenenfalls, z.B. durch Niederalkyl, insbesondere tert.-Niederalkyl, z.B. tert.-Butyl, Niederalkoxy, wie Methoxy, Hydroxy, Halogen, z.B. Chlor, und/oder Nitro, mono- oder polysubstituierte Phenylreste darstellt, wie gegebenenfalls, z.B. wie oben erwähnt substituiertes Benzyloxycarbonyl, z.B. 4-Nitro-benzyloxycarbonyl oder 4-Methoxybenzyloxycarbonyl, oder gegebenenfalls, z.B. wie oben erwähnt, substituiertes Diphenylmethoxycarbonyl, z.B. Diphenylmethoxycarbonyl oder Di-(4-methoxyphenyl)-methoxycarbonyl, oder 2-Halogenniederalkoxycarbonyl, z.B. 2,2,2-Trichloräthoxycarbonyl sein. Weitere derartige geschützte Carboxylgruppen sind z.B. Aroylmethoxycarbonyl, worin die Aroylgruppe vorzugsweise Benzoyl oder z.B. durch Halogen, wie Brom, substituiertes Benzoyl darstellt, z.B. Phenacyloxycarbonyl, oder Polyhalogenaryloxycarbonyl, wie Pentachlorphenyloxycarbonyl. Durch Veresterung intermediär geschützte Carboxylgruppen sind in erster Linie entsprechende Silyloxycarbonyl-, insbesondere organische Silyloxycarbonylgruppen oder entsprechende Stannyloxycarbonylgruppen. In diesen enthält das Silicium- bzw. Zinnatom vorzugsweise Niederalkyl, insbesondere Methyl, ferner Niederalkoxy, z.B. Methoxy, und/oder Halogen, z.B. Chlor, als Substituenten. Geeignete Silyl- bzw. Stannylschutzgruppen sind in erster Linie Triniederalkylsilyl, insbesondere Trimethylsilyl oder Tri-n-butylsilyl, ferner Dimethyl-tert.-butyl-silyl, Niederalkoxydiniederalkylsilyl, z.B. Methoxydimethylsilyl, oder entsprechend substituierte Stannylverbindungen, z.B. Tri-n-butyl-stannyl.

Bevorzugte geschützte Carboxylgruppen sind tert.-Butyloxycarbonyl, gegebenenfalls, z.B. wie erwähnt substituiertes Benzyloxycarbonyl, z.B. 4-Nitrobenzyloxycarbonyl und Diphenylmethoxycarbonyl sowie Trimethylsilyloxycarbonyl.

Verbindungen der Formel I mit geschützten funktionellen Gruppen sind als Zwischenverbindungen zur Herstellung von Verbindungen der Formel I mit freien funktionellen Gruppen von Bedeutung und werden wie sonstige neue Ausgangs- und Zwischenprodukte im Rahmen dieser Erfindungsanmeldung ebenfalls beansprucht.

Die nachstehenden Ausführungen erläutern die erfindungsgemässen Verfahren a) bis m).

Verfahren a) bis e) (Acylierungen): Gegebenenfalls vorhandene, die Aminogruppe substituierende und deren Acylierung erlaubende Reste in einem Ausgangsmaterial der Formel II, IV, VII oder VIII sind beispielsweise organische Silyl- oder Stannylgruppen, ferner bei primären Aminogruppen auch Ylidengruppen, die zusammen mit der Aminogruppe eine Schiff'sche Base bilden. Die genannten, organischen Silyl- oder Stannylgruppen sind z.B. die gleichen, die auch mit der 4-Carboxylgruppe am Cephemring eine geschützte Carboxylgruppe zu bilden vermögen. Bei der Silylierung oder Stannylierung einer Carboxylgruppe in einem Ausgangsmaterial der Formel II, IV oder VIII können bei Verwendung eines Ueberschusses des Silylierungs- oder Stannylierungsmittels, die Aminogruppe sowie weitere gegebenenfalls vorhandene funktionelle Gruppen in an sich bekannter Weise ebenfalls silyliert oder stannyliert werden.

Die genannten Ylidengruppen sind in erster Linie Arylmethylengruppen, worin Aryl insbesondere für gegebenenfalls, wie durch Nitro oder Hydroxy, substituiertes Phenyl steht.

Die übrigen in den für die Acylierungsreaktionen a)

bis e) verwendeten Ausgangsstoffen der Formeln II bis VIII vorhandenen funktionellen Gruppen können durch die bereits genannten Schutzgruppen geschützt sein. Vorzugsweise sind alle nicht an der Acylierungsaktion teilnehmenden reaktionsfähigen funktionellen Gruppen, insbesondere aber gegebenenfalls vorhandene acylierbare Gruppen, wie Amino-, Hydroxy-, Carboxy- und Mercaptogruppen, geschützt. Die Herstellungsverfahren dieser Zwischen- und Ausgangsprodukte werden im Abschnitt "Herstellung der Ausgangsverbindungen" beschrieben.

Den Acylrest einer Carbonsäure einführende Acylierungsmittel sind beispielsweise die Carbonsäure selbst, wenn diese, wie im Falle der Säure der Formel III, ausreichend stabil ist, oder reaktionsfähige funktionelle Derivate davon.

Falls gemäss Acylierungsverfahren a) eine freie Säure der Formel III, worin alle funktionellen Gruppen ausser der reagierenden Carboxylgruppe geschützt sind, zur Acylierung eingesetzt wird, verwendet man diese üblicherweise in Gegenwart geeigneter Kondensationsmittel, wie Carbodiimide, beispielsweise N,N'-Diäthyl- oder N,N'-Dicyclohexylcarbodiimid oder geeigneter Carbonylverbindungen, wie Carbonyldiimidazol.

Ein reaktionsfähiges, d.h. acylierend wirkendes bzw. amidbildendes, funktionelles Derivat einer der für die Acylierungsreaktionen a) bis e) verwendeten Carbon-, Thiocarbon-, Kohlen-, Thiokohlen- oder Dicarbonsäuren, worin alle funktionellen Gruppen ausser der oder den reagierenden Säuregruppe(n) geschützt sind bzw. sein können, ist in erster Linie ein Anhydrid einer solchen Säure, inklusive und vorzugsweise ein gemischtes Anhydrid, aber auch, wie im Falle der Säure VI, ein inneres Anhydrid eines Kohlen- oder Thiokohlensäureamids, also ein entsprechendes Isocyanat bzw. Isothiocyanat. Gemischte Anhydride

sind z.B. diejenigen mit anorganischen Säuren, wie Halogenwasserstoffsäuren, d.h. die entsprechenden Säurehalogenide, z.B. -chloride oder -bromide, mit einer phosphorhaltigen Säure, z.B. Phosphorsäure oder phosphoriger Säure, oder mit einer schwefelhaltigen Säure, z.B. Schwefelsäure, oder mit Cyanwasserstoffsäure. Weitere gemischte Anhydride sind z.B. diejenigen mit organischen Carbonsäuren, wie mit gegebenenfalls, z.B. durch Halogen, wie Fluor oder Chlor, substituierten Niederalkancarbonsäuren, z.B. Pivalinsäure oder Trichloressigsäure, oder mit Halbestern, insbesondere Niederalkylhalbestern der Kohlensäure, wie dem Aethyl- oder Isobutylhalbester der Kohlensäure, oder mit organischen, insbesondere aliphatischen oder aromatischen Sulfonsäuren, z.B. p-Toluolsulfonsäure. Weitere, zur Reaktion mit der Amino-, Hydroxy- oder Mercaptogruppe geeignete Säurederivate einer der oben genannten Carbon-, Thiocarbon-, Kohlen-, Thiokohlen- oder Dicarbonsäuren, worin alle funktionellen Gruppen ausser der (oder den) reagierenden Carboxylgruppe(n) geschützt sind, bzw. sein können, sind reaktionsfähige, insbesondere sogenannte aktivierte Ester, wie Ester mit vinylogen Alkoholen (d.h. Enolen), wie vinylogen Niederalkenolen, Cyanmethylester, Trichlormethylester, Iminomethylesterhalogenide, wie Dimethyliminomethylesterchlorid (hergestellt aus der Carbonsäure und Dimethyl-chlormethyliden-iminiumchlorid der Formel $[(CH_3)_2N{=}CHCl]^{\oplus}\ Cl^{\ominus}$), oder Arylester, wie 4-Nitrophenyl- oder 2,4-Dinitrophenylester, N-(Acyloxy)-amino-Verbindungen, wie 1-Acyloxy-benztriazol, N-Acyloxyphthalimid, N-Acyloxysuccinimid, N-Acyloxybenzamid oder aktivierte Säureamide, wie N-Acylazole, z.B. N-Acylimidazol, N-Acyltriazol oder N-Acyltetrazol. Beispiele für reaktionsfähige Säurederivate, ihre Herstellung und analoge Anwendung sind z.B. im Handbuch von Houben-Weyl, Band 15/2, Seite 1-364, (Georg Thieme-Verlag, Stuttgart, 1974) ausführlich beschrieben worden.

Die Acylierung mit einem Säurederivat, wie einem Anhydrid, insbesondere mit einem Säurehalogenid, wird bevorzugt in Anwesenheit eines säurebindenden Mittels, beispielsweise eines basischen Mittels, wie einer organischen Base, z.B. eines tertiären Amins, wie Triniederalkylamin, z.B. Trimethylamin, Triäthylamin oder Aethyl-diisopropylamin, oder N,N-Diniederalkyl-anilin, z.B. N,N-Dimethylanilin, oder eines cyclischen tertiären Amins, wie eines N-niederalkylierten Morpholins, wie N-Methylmorpholin, oder einer Base vom Pyridin-Typ, z.B. Pyridin, einer anorganischen Base, beispielsweise eines Alkalimetall- oder Erdalkalimetallhydroxids, -carbonats oder -hydrogencarbonats, z.B. Natrium-, Kalium- oder Calciumhydroxid, -carbonat oder -hydrogencarbonat, durchgeführt. Diese Basen spielen vor allem bei der Acylierung von Aminogruppen, also bei den Acylierungsverfahren a) und b) eine Rolle. Bei diesen Acylierungsreaktionen, insbesondere aber bei der Acylierung von Amidgruppen, also bei den Acylierungsverfahren c), d) und e), verwendet man als säurebindende Mittel vorteilhafter neutrale Protonenfänger, z.B. solche aus der Gruppe der Oxirane, wie ein niederes 1.2-Alkylenoxid, z.B. Aethylenoxid, oder ein reaktionsfähiges Olefin, wie Niederalken , z.B. Propylen, oder ein Molekularsieb, z.B. ein pulverförmiges Typ 4 Å-Molekularsieb und/oder man setzt die (zuvor oder in situ) silylierte oder stannylierte, wie Triniederalkyl-, z.B. Trimethyl-silylierte Amidgruppe um. Bei den genannten Amid-Acylierungsreaktionen setzt man z.B. gemischte Anhydride, insbesondere die entsprechenden Säurechloride, also Säurechloride der Verbindungen der Formel VI, sowie die Mono- oder Disäurechloride der Kohlensäure, Thiokohlensäure bzw. Oxalsäure ein, wie unten näher bezeichnet.

Die obigen Acylierungen werden bevorzugt in einem inerten, vorzugsweise wasserfreien Lösungsmittel oder Lösungsmittelgemisch vorgenommen, z.B. in einem N-alkylierten Carbonsäureamid, wie einem Formamid, z.B. Dimethylformamid, einem halogenierten Kohlenwasserstoff, z.B. Methylenchlorid, Tetrachlorkohlenstoff oder Chlorbenzol, einem Keton, z.B. Aceton, einem Ester, z.B. Essigsäureäthylester, oder einem Nitril, z.B. Acetonitril, oder Mischungen davon, für die Amidacylierungen gemäss c), d) und e) empfiehlt sich die Verwendung unpolarer, z.B. entsprechender halogenierter Lösungsmittel, wie Methylenchlorid.

Die Acylierungen erfolgen bei Raumtemperatur, wenn notwendig, bei erniedrigter oder erhöhter Temperatur, etwa bei -40° bis etwa 100°C, bevorzugt bei -10° bis +40°, und/oder in einer Inertgas-, z.B. Stickstoffatmosphäre.

Bei jedem der oben genannten Acylierungsverfahren kann ein Säurederivat, wenn erwünscht, auch in situ gebildet werden. So erhält man z.B. ein gemischtes Anhydrid durch Behandeln einer Säure der Formel III mit entsprechend geschützten funktionellen Gruppen, oder eines geeigneten Salzes davon, wie eines Ammoniumsalzes, z.B. mit einem tertiären Amin, wie 4-Methylmorpholin, oder eines Metall-, z.B. Alkalimetallsalzes, mit einem geeigneten Säurederivat, wie einem entsprechenden Säurehalogenid einer gegebenenfalls substituierten Niederalkancarbonsäure, z.B. Trichloracetylchlorid, oder mit einem Halbester eines Kohlensäurehalbhalogenids, z.B. Chlorameisensäureäthylester oder -isobutylester, und verwendet das so erhältliche gemischte Anhydrid ohne Isolierung.

Die entsprechenden reaktionsfähigen Säurederivate der Kohlen- bzw. Thiokohlensäuren der allgemeinen Formel V bzw. VI werden sogar vorzugsweise in situ gebildet, indem man auf die entsprechenden Aminoverbindungen der allgemeinen Formel VII bzw. IV ein bivalentes reaktives Derivat der Kohlen- bzw. Thiokohlensäure, wie eines der unten genannten, vorzugsweise ein entsprechendes Säurechlorid, wie Phosgen, Thiophosgen oder ein Chlorameisensäurederivat, vorzugsweise in Gegenwart eines der oben genannten neutralen Protonenfänger, einwirken lässt.

Als bevorzugtes, gemäss Acylierungsverfahren b) den Acylrest einer Kohlen- oder Thiokohlensäure der Formel V einführendes Acylierungsmittel ist daher das entsprechende Carbaminsäure- oder Thiocarbaminsäurehalogenid, insbesondere -chlorid, zu nennen.

Als bevorzugte Kohlen- oder Thiokohlensäureverbindung der Formel VI gemäss Acylierungsverfahren c) ist ebenfalls das entsprechende Carbamin- bzw. Thiocarbaminsäurehalogenid, z.B. das entsprechende -chlorid zu nennen (VI, Z = Cl-C(X)-NH-), das aber im Gegensatz zu dem entsprechenden Derivat der allgemeinen Formel V, als Derivat eines primären Amins leicht unter Abspaltung von Halogenwasserstoff bzw. Chlorwasserstoff das entsprechende Isocyanat bzw. Isothiocyanat (VI, Z = (-N=C=X)) bilden und als solches mit Amiden der Formel VII spontan unter Ausbildung der Harnstoffgruppierung in die erfindungsgemässe Verbindung der Formel I übergehen kann. Es ist zweckmässig, auch bei Verfahren b) und c) unpolare inerte Lösungsmittel, wie Chlorniederaliphaten, z.B. Methylenchlorid, Chloroform oder ein gegebenenfalls chloriertes aromatisches Lösungsmittel wie Benzol, Toluol oder Chlorbenzol oder ein inertes ätherisches Lösungsmittel wie Tetrahydrofuran oder Dioxan zu verwenden und die Reaktion bei oder unterhalb Raumtemperatur, also unter Kühlen, durchzuführen. Bivalente reaktive Derivate der Kohlen-, Thiokohlen- oder Oxalsäure, die für die unter d) und e) genannten Acylierungsverfahren Verwendung finden, sind die entsprechenden Säurehalogenide, wie Säurechloride, z.B.

Phosgen oder Oxalylchlorid, Orthoester, wie Niederalkylorthoester, z.B. Orthokohlensäuretetramethylester, Imidoester, wie Imidokohlensäureniederalkylester, z.B. Imidokohlensäuredimethylester, Esterhalogenide, wie Halogenameisensäureniederalkylester, z.B. Chlorameisensäuremethylester, Imidhalogenide, wie Imidokohlensäurehalogenide, z.B. Kohlensäureimidchlorid, aktivierten Ester, wie entsprechende Enol- oder Arylester, z.B. Bis-p-nitrophenylcarbonat oder aktivierten Amide, wie entsprechende heterocyclische Bisamide der Kohlensäure, z.B. Carbonyldiimidazol. Bevorzugte Verbindungen dieser Art sind Kohlensäure- oder Thiokohlensäurehalogenide, z.B. Phosgen, Thiophosgen oder Kohlensäuredibromid, Tetramethyl-orthocarbonat, Chlorkohlensäureimidchlorid, Chlorcyan, Chlorameisensäureester, wie der entsprechende Methyl-, Trichlormethyl-, Benzyl- oder Phenylester, Imidokohlensäurediäthylester oder -diphenylester, Carbonyldiimidazol, Carbonylditetrazol, oder Kohlensäure-di-(p-nitrophenyl)-ester, sowie entsprechende Verbindungen der Oxalsäure, z.B. Oxalylchlorid.

Die im Acylierungsverfahren e) verwendeten, acylierend wirkenden bivalenten Derivate der Kohlensäure oder Oxalsäure und ebenso die entsprechenden im Verfahren d) verwendeten bivalenten reaktiven Derivate, können à priori in doppelt reaktiver Form, oder zunächst in Form eines monoreaktiven Halbderivats der Kohlen- oder Thiokohlensäure (in dem nur ein Säurerest in reaktiver Form vorliegt) eingesetzt und die zweite reaktive Gruppe erst nachträglich gebildet oder aktiviert und (mit einem zweiten Reaktiónspartner) zur Reaktion gebracht werden.

Vorzugsweise wird beim Verfahren e) Phosgen oder Oxalylchlorid, vor allem in einem 2-10 molaren Ueberschuss, in einem unpolaren inerten Lösungsmittel, wie Methylenchlorid, vorgelegt und darauf die geschützte, an den endständigen Aminogruppen silylierte Verbindung der Formel VIII einwirken gelassen. Als zusätzliche Massnahme kann ein zweites säurebindendes Mittel, wie Propylen oder Propylenoxid, verwendet und die Acylierung unter Kühlung z.B. bei oder unterhalb 0°C, vorgenommen werden.

Verfahren f) (Ringschluss unter Abspaltung von $Y^{O}$ und $Z^{O}$): In einer Ausgangsverbindung der Formel IX ist $Z^{O}$ Wasserstoff oder Halogen, wie Chlor, Brom oder Jod, und $Y^{O}$ ist beispielsweise eine Gruppe $-S-R_a$ eine mit dem Schwefelatom an die Thiogruppe -S- gebundene Gruppe $-SO_2-R_b$ oder auch eine Gruppe $-S-SO_2-R_b$.

In der Gruppe $-S-R_a$ ist $R_a$ ein gegebenenfalls substituierter aromatischer heterocyclischer Rest mit bis zu 15, bevorzugt bis zu 9 Kohlenstoffatomen, und mindestens einem Ringstickstoffatom und gegebenenfalls einem weiteren Ringheteroatom, wie Sauerstoff oder Schwefel, welcher Rest mit einem seiner Ringkohlenstoffatome, das mit einem Ringstickstoffatom durch eine Doppelbindung verbunden ist, an die Thiogruppe -S- gebunden ist, oder ein Acylrest einer organischen Carbon- oder Thiocarbonsäure, wie einer gegebenenfalls substituierten, niederaliphatischen, cycloaliphatischen, arylniederaliphatischen oder aromatischen Carbon- oder Thiocarbonsäure.

Solche Reste $R_a$ sind z.B. fünfgliedrige, heterodiaza-, wie Thiadiaza- oder Oxadiazacyclische oder -triazacyclische Reste aromatischen Charakters, insbesondere aber diaza-cyclische, oxazacyclische und thiazacyclische und in erster Linie entsprechende benzdiazacyclische, benzoxazacyclische oder benzthiazacyclische Reste aromatischen Charakters, worin ein substituierbares Ringstickstoffatom z.B. durch Niederalkyl substituiert sein kann. Repräsentativ für solche Gruppen $R_a$ sind 1-Methyl-imidazol-2-yl, 1,3-Thiazol-

2-yl, 1,3,4-Thiadiazol-2-yl, 1,3-Oxazol-2-yl, 1,3,4-Oxadiazol-2-yl, 2-Chinolyl, 1-Methyl-benzimidazol-2-yl, Benzoxazol-2-yl und insbesondere Benzthiazol-2-yl.

Bevorzugte Acyl- oder Thioacylreste $R_a$ sind beispielsweise Niederalkanoyl, z.B. Acetyl, Thioniederalkanoyl, z.B. Thioacetyl, Benzoyl, Thiobenzoyl, heterocyclisches Carbonyl, wie Pyridylcarbonyl oder Thenoyl, oder entsprechende substituierte, beispielsweise durch Niederalkyl, wie Methyl, Halogen, wie Fluor oder Chlor, Niederalkoxy, wie Methoxy, Phenyl oder Phenoxy, mono- oder polysubstituierte Acyl- oder Thioacylgruppen.

In den Gruppen $-SO_2-R_b$ und $-S-SO_2-R_b$ ist $R_b$ ein gegebenenfalls substituierter, insbesondere niederaliphatischer, cycloaliphatischer, arylniederaliphatischer oder aromatischer Kohlenwasserstoffrest. Geeignete Gruppen $R_b$ sind beispielsweise gegebenenfalls substituierte, wie durch Niederalkoxy, wie Methoxy, Halogen, wie Fluor, Chlor oder Brom, Aryl, wie Phenyl, Aryloxy, wie Phenyloxy, mono- oder polysubstituierte Alkyl-, insbesondere Niederalkyl-, Niederalkenyl- oder Cycloalkylgruppen oder gegebenenfalls durch Niederalkyl, wie Methyl, Niederalkoxy, wie Methoxy, Halogen, wie Fluor, Chlor oder Brom, Aryl, wie Phenyl, Aryloxy, wie Phenyloxy, oder Nitro, mono- oder polysubstituierte Aryl-, wie Phenyl- oder Naphthylgruppen, beispielsweise Phenyl, p-Tolyl, p-Methoxyphenyl, o-, m- oder p-Chlorphenyl, p-Biphenylyl, p-Phenoxyphenyl, p-Nitrophenyl oder 1- oder 2-Naphthyl.

In einer Verbindung der Formel IX kann die Gruppe $R_1$ in trans- (Crotonsäurekonfiguration) oder in cis-Stellung (Isocrotonsäurekonfiguration) zur Carboxylgruppe stehen. Der Ringschluss unter Abspaltung von $Y^o$ und $Z^o$ wird

durch eine geeignete Base, insbesondere eine starke organische oder anorganische Base bewirkt. Hervorzuheben sind bicyclische Amidine, wie Diazabicycloalkene, z.B. 1,5-Diazabicyclo[4.3.0]non-5-en oder 1,5-Diazabicyclo[5.4.0]-undec-5-en, substituierte, z.B. durch Niederalkyl mehrfach substituierte Guanidine, wie Tetramethylguanidin, ferner Metallbasen, wie Hydride, Amide oder Alkoholate von Alkalimetallen, insbesondere von Lithium, Natrium oder Kalium, beispielsweise Natriumhydrid, Lithiumdiniederalkylamide, wie Lithiumdiisopropylamid, Kalium-niederalkanolate, wie Kalium-tert.-butylat, sowie tertiäre organische Stickstoffbasen, z.B. Triniederalkylamine, wie Triäthylamin.

Die Reaktion wird in einem geeigneten inerten Lösungsmittel durchgeführt, beispielsweise in einem niederaliphatischen, cycloaliphatischen oder aromatischen Kohlenwasserstoff, wie Hexan, Cyclohexan, Benzol oder Toluol, einem halogenierten Kohlenwasserstoff, wie Methylenchlorid, einem Aether, wie einem Diniederalkyläther, z.B. Diäthyläther, einem Diniederalkoxyniederalkan, z.B. 1.2-Dimethoxyäthan oder einem cyclischen Aether, z.B. Dioxan oder Tetrahydrofuran, einem Niederalkanol, z.B. Methanol oder Aethanol, oder in einem Gemisch dieser Lösungsmittel bei Raumtemperatur oder unter leichtem Erwärmen auf 40 bis 50°C, gewünschtenfalls in einer Inertgas-, wie Stickstoffatmosphäre.

<u>Verfahren g) (Isomerisierung)</u>: In einer 2-Cephem-Verbindung der Formel X weist die gegebenenfalls geschützte Carboxylgruppe in 4-Stellung vorzugsweise die α-Konfiguration auf.

2-Cephem-Verbindungen der Formel X werden isomerisiert, indem man sie mit einem schwach-basischen Mittel behandelt und die entsprechende 3-Cephem-Verbindung aus dem normalerweise entstehenden Isomerengemisch isoliert. Geeignete Isomerisierungsmittel sind z.B. organische, stickstoffhaltige Basen, insbesondere tertiäre heterocyclische Basen aromatischen Charakters, in erster Linie Basen des Pyridin-Typs, wie Pyridin selber, sowie Collidine oder Lutidine, ferner Chinolin, tertiäre aromatische Basen, z.B. solche des Anilin-Typs, wie N,N-Diniederalkylaniline, z.B. Dimethylanilin oder, Triniederalkylamine, z.B. Trimethylamin oder Diisopropyläthylamin, sowie Gemische davon, wie das Gemisch einer Base vom Pyridintyp und eines Tri-niederalkylamins, z.B. Pyridin und Triäthylamin. Ferner können auch anorganische oder organische Salze von Basen, sowie Gemische von solchen basischen Mitteln verwendet werden.

Bei Isomerisierung mit basischen Mitteln arbeitet man vorzugsweise in wasserfreiem Medium, in An- oder Abwesenheit eines Lösungsmittels, wie eines gegebenenfalls halogenierten, z.B. chlorierten, niederaliphatischen, cycloaliphatischen oder aromatischen Kohlenwasserstoffs, oder eines Lösungsmittelgemisches, wobei als Reaktionsmittel verwendete, unter den Reaktionsbedingungen flüssige Basen gleichzeitig auch als Lösungsmittel dienen können, unter Kühlen, bei Zimmertemperatur oder unter Erhitzen, vorzugsweise in einem Temperaturbereich von etwa -30°C bis etwa +100°C, in einer Inertgas-, z.B. Stickstoffatmosphäre, und/oder in einem geschlossenen Gefäss.

Die so erhältlichen 3-Cephem-Verbindungen lassen sich in an sich bekannter Weise, z.B. durch Adsorption und/oder Kristallisation, von gegebenenfalls noch vorhandenen 2-Cephem-verbindungen abtrennen.

Die Isomerisierung von 2-Cephem-verbindungen der Formel X kann auch durchgeführt werden, indem man den Ringschwefel (d.h. die 1-Stellung) oxidiert, wobei sich die Doppelbindung aus der 2-Stellung grösstenteils in die 3-Stellung verschiebt und, wenn erwünscht, ein so erhaltenes Isomerengemisch der 1-S-Oxide trennt, und die so erhältlichen 1-Oxide von 3-Cephemverbindungen zu den reinen 3-Cephem-verbindungen der Formel I reduziert.

Geeignete Oxidationsmittel für die Oxidation des Ringschwefels von 2-Cephem-verbindungen sind Persäuren, wie anorganische oder organische Persäuren oder Gemische aus Wasserstoffperoxid und Säuren, insbesondere organischen Carbonsäure mit einer Dissoziationskonstante von wenigstens $10^{-5}$. Geeignete anorganische Persäuren sind Perjod- und Perschwefelsäure. Organische Persäuren sind entsprechende Percarbon- und Persulfonsäuren, die als solche zugesetzt oder durch Verwendung von wenigstens einem Aequivalent Wasserstoffperoxyd und einer Carbonsäure in situ gebildet werden könne. Dabei ist es zweckmässig, einen grossen Ueberschuss der Carbonsäure zu verwenden, wenn z.B. Essigsäure als Lösungsmittel verwendet wird. Geeignete organische Persäuren sind z.B. Trifluorperessigsäure, gegebenenfalls substituierte aromatische Persäuren, wie Perbenzoesäure, 3-Chlorperbenzoesäure oder Monoperphthalsäure oder p-Toluolpersulfonsäure.

Die obige Oxidation kann in Gegenwart von geeigneten Katalysatoren durchgeführt werden. So kann z.B. die Oxidation mit Percarbonsäuren durch die Anwesenheit einer Säure mit einer Dissoziationskonstante von wenigstens $10^{-5}$ katalysiert werden, wobei ihre Wirksamkeit von ihrer Stärke abhängt. Als Katalysatoren geeignete Säuren sind z.B. Essigsäure, Perchlorsäure und Trifluoressigsäure. Ueblicherweise verwendet man mindestens äquimolare Mengen des Oxidationsmittels, vorzugsweise einen geringen Ueberschuss von etwa 10 % bis etwa 20 %. Die Oxidation wird unter milden Bedingungen, z.B. bei Temperaturen von etwa -50°C bis etwa +100°C, vorzugsweise von etwa -10°C bis etwa +40°C durchgeführt.

Die Reduktion der 1-S-Oxide von 3-Cephem-Verbindungen kann in an sich bekannter Weise, z.B. durch Behandeln mit einem Reduktionsmittel durchgeführt werden. Geeignete Reduktionsmittel sind beispielsweise katalytisch aktivierter Wasserstoff, wobei Edelmetallkatalysatoren verwendet werden, welche Palladium, Platin oder Rhodium enthalten, und die man gegebenenfalls zusammen mit einem geeigneten Trägermaterial, wie Kohle oder Bariumsulfat, einsetzt, reduzierende Schwermetallsalze oder -Komplexe oder reduzierende trivalente anorganische oder organische Phosphorverbindungen, wie Phosphine, Ester, Amide und insbesondere Halogenide der phosphinigen, phosphonigen oder phosphorigen Säure, worin organische Reste in erster Linie gegebenenfalls substituierte Niederalkyl-, Phenyl oder Phenylniederalkylgruppen darstellen, wie z.B. Triphenylphosphin, Diphenylchlorphosphin, Phenyl dichlorphosphin, Phosphorigsäuretriphenylester, Phosphortrichlorid, Phosphortribromid, oder reduzierende Halogensilanverbindungen, die mindestens ein an das Siliciumatom gebundenes Wasserstoffatom aufweisen, und die ausser Halogen, wie Chlor, Brom oder Jod, auch organische Reste, wie Niederalkyl- oder Phenylgruppen aufweisen können, wie Mono-, Di- oder Trichlorsilan, Diphenylchlorsilan, Dimethylchlorsilan oder auch Halogensilanverbindungen, worin alle Wasserstoffe durch organische Reste ersetzt sind, wie Triniederalkylhalogensilan, z.B. Trimethylchlor- oder Trimethyljodsilan.

Als aktivierende Mittel, die zusammen mit denjenigen der obgenannten Reduktionsmittel verwendet werden, welche selber nicht Lewissäure-Eigenschaften aufweisen, d.h. die in erster Linie zusammen mit Schwermetall- und den nichthalogenhaltigen trivalenten Phosphor-Reduktionsmitteln oder bei der katalytischen Reduktion eingesetzt werden, sind insbesondere organische Carbon- und Sulfonsäurehalogenide, ferner Schwefel-, Phosphor- oder Siliciumhalogenide mit gleicher oder grösserer Hydrolysenkonstante zweiter Ordnung als Benzoylchlorid, z.B. Phosgen, Oxalylchlorid, Phosphortrichlorid oder Phosphortribromid, zu erwähnen.

Die Reduktion wird vorzugsweise in Gegenwart von Lösungsmitteln oder Gemischen davon durchgeführt, deren Auswahl in erster Linie durch die Löslichkeit der Ausgangsstoffe und die Wahl des Reduktionsmittels bestimmt wird, so z.B. Niederalkancarbonsäuren oder Ester davon, wie Essigsäure und Essigsäureäthylester, bei der katalytischen Reduktion, und z.B. gegebenenfalls halogenierte oder nitrierte niederaliphatische, cycloaliphatische, aromatische oder arylniederaliphatische Kohlenwasserstoffe, z.B. Benzol, Methylenchlorid, Chloroform oder Nitromethan, geeignete Säurederivate, wie Niederalkancarbonsäureester oder -nitrile, z.B. Essigsäureäthylester oder Acetonitril, aprotische Säureamide, wie Dimethylformamid oder Hexamethylphosphoramid, Aether, z.B. Tetrahydrofuran oder Dioxan, Ketone, z.B. Aceton, oder Sulfone wie Tetramethylensulfon, etc., zusammen mit den chemischen Reduktionsmitteln, wobei diese Lösungsmittel vorzugsweise kein Wasser enthalten. Dabei arbeitet man gewöhnlich bei Temperaturen von etwa -20° bis etwa 100°C, wobei bei Verwendung von sehr reaktionsfähigen Aktivierungsmitteln die Reaktion auch bei tieferen Temperaturen durchgeführt werden kann.

Verfahren h) (Austausch von freiem oder sulfonyliertem Hydroxy gegen Halogen):

Der Austausch der freien Hydroxygruppe $R_1^a$ durch Halogen wird auf an sich bekannte Weise, beispielsweise durch Behandeln mit einem halogenierenden, insbesondere chlorierenden Mittel, durchgeführt. Geeignete Halogenierungsmittel zur Einführung von Chlor oder Brom sind beispielsweise Phosphor-Reagentien, wie Di-halogen-triorganyl-phosphorane, Trihalogendiorganyl-phosphorane oder ein Gemisch bestehend aus einem Triorganylphosphin und einem Tetrahalogenmethan. Repräsentative Vertreter der genannten Phosphorane sind Dichlor-triphenyl-, Trichlor-diphenyl- oder Dibromtriphenylphosphoran. Repräsentative Vertreter der genannten Phosphine sind Triäthyl-, Tris-(dimethylamino)- und insbesondere Triphenylphosphin. Tetrahalogenmethane sind z.B. Tetrabrom- und insbesondere Tetrachlorkohlenstoff.

Die Reaktion mit den halogenierenden Phosphorreagentien findet auf an sich bekannte Weise in einem inerten, aprotischen, bevorzugt polaren Lösungsmittel, wie einem chlorierten Kohlenwasserstoff, z.B. Methylenchlorid, Chloroform, Tetrachlorkohlenstoff, 1,2-Dichloräthan, einem Nitril, wie Acetonitril oder Benzonitril, oder einem N,N-disubstituierten Carbonsäureamid, wie Dimethylformamid oder N,N-Dimethylacetamid, oder Mischungen davon, je nach Reaktivität des eingesetzten Reagenses unter Kühlen oder Erwärmen, d.h. bei Temperaturen zwischen etwa -60°C bis zur Rückflusstemperatur des verwendeten Lösungsmittel, gegebenenfalls in einer Inertgas-, wie Stickstoffatmosphäre, statt. Bei Verwendung von Triniederalkyl- oder Tris-(diniederalkylamin)-phosphinen und Tetrachlorkohlenstoff oder Tetrabromkohlenstoff ist gewöhnlich Kühlen notwendig, etwa auf -60° bis -20°C. Beim Halogenieren mit den genannten Phosphoranen kann dem Reaktionsmedium zum Abfangen entstehenden Halogenwasserstoffs eine schwache Base, wie

Pyridin oder ein Diniederalkylanilin, wie Dimethylanilin oder ein neutraler Protonenfänger, wie Propylen, zugesetzt werden.

Weitere wichtige Halogenierungsmittel sind entsprechende N,N-diniederalkylierte Halogen-iminiumhalogenid-Verbindungen, insbesondere solchen der Formel $[(CH_3)_2N{=}CH\text{-}Hal]^{\oplus}$ $Hal^{\ominus}$, worin Hal Chlor bzw. Brom ist. Die obigen Reagentien werden üblicherweise in situ hergestellt, indem man ein geeignetes N,N-diniederalkyliertes Amid, in erster Linie Dimethylformamid, mit einem Chlorierungs- bzw. Bromierungsmittel, z.B. Phosgen, Carbonyldibromid, Oxalylchlorid, Oxalylbromid, Thionylbromid, Phosphoroxychlorid, Phosphoroxybromid, Phosphorpentachlorid oder insbesondere mit Phosphortrichlorid, Phosphortribromid oder Thionylchlorid behandelt.

Die obige Reaktion wird üblicherweise in Gegenwart eines Lösungs- oder Verdünnungsmittels durchgeführt, wobei man neben dem normalerweise im Ueberschuss vorliegenden und als Lösungsmittel dienenden Amid, üblicherweise Dimethylformamid, ferner auch Dimethylacetamid, auch Aether z.B. Tetrahydrofuran oder Dioxan, Halogenkohlenwasserstoffe, z.B. Methylenchlorid, oder Sulfoxide, z.B. Dimethylsulfoxid, verwenden kann.

Die Chlorierungs- und Bromierungsmittel werden üblicherweise in Mengen verwendet, die zwei Aequivalenten des 3-Hydroxy-3-cephem-Ausgangsmaterials entsprechen. Die Reaktion kann man z.B. so durchführen, dass man das Chlorierungs- bzw. Bromierungsmittel unter Kühlen zu einer Lösung des 3-Hydroxy-3-cephem-Ausgangsmaterials in Dimethylformamid zugibt, worauf man das Reaktionsgemisch während einigen Stunden bei Raumtemperatur stehen lässt.

Die Chlorierung oder Bromierung kann auch so durchgeführt werden, dass man zunächst das Chlorierungs- bzw. Bromierungsmittel mit dem Amid, insbesondere dem Dimethylformamid, mischt und so das Halogeniminiumhalogenid bildet, worauf man die Lösung des 3-Hydroxy-3-cephem-Ausgangsmaterials im Amid, insbesondere in Dimethylformamid, dem noch ein zusätzliches Lösungsmittel zugegeben werden kann, oder in einem anderen Lösungsmittel, z.B. Tetrahydrofuran, zufügt.

Die Umwandlung der freien Hydroxygruppe $R_1^a$ in Fluor kann z.B. durch Behandeln mit einem Reagens der Formel $F_3S$-Am erfolgen, worin Am eine disubstituierte Aminogruppe darstellt; solche Reagentien sind u.a. von Markovskij et al., Synthesis, Bd. 1973, S. 787 beschrieben worden. Die Gruppe Am steht in erster linie für Diniederalkylamino, z.B. Dimethylamino, Diäthylamino oder Diisopropylamino, Niederalkylphenyl-amino, z.B. Methylanilino, Niederalkylenamino, z.B. Pyrrolidino oder Piperidino, Oxaniederalkylenamino, z.B. Morpholino, oder gegebenenfalls N-niederalkyliertes Azaniederalkylenamino, z.B. 4-Methyl-piperazino.

Die Reaktion wird vorzugsweise in einem geeigneten inerten Lösungsmittel, wie einem gegebenenfalls chlorierten Kohlenwasserstoff, z.B. Cyclopentan, Cyclohexan, Benzol, Toluol, Xylol, Chlorbenzol, Dichlorbenzol, Pentan, Hexan, Chloroform, und vor allem Methylenchlorid oder einem Aether, wie Diäthyläther, Tetrahydrofuran oder vor allem Dioxan, falls notwendig, unter Kühlen oder Erwärmen, z.B. in einem Temperaturbereich von etwa -20°C bis etwa 80°C, vorzugsweise von etwa 0°C bis etwa 30°C und/oder unter einer Inertgasatmosphäre durchgeführt.

Eine sulfonylierte Hydroxygruppe $R_1^a$ kann durch Behandeln mit einem anorganischen Fluorid in Gegenwart eines Kronenäthers in Fluor übergeführt werden. Eine sulfonylierte Hydroxygruppe $R_1^a$ ist in erster Linie Niederalkylsulfonyloxy, insbesondere Methylsulfonyloxy, kann aber auch Arylsulfonyloxy sein, worin Aryl vorzugsweise gegebenenfalls, z.B. durch Niederalkyl, wie Methyl, Halogen, z.B. Brom oder Nitro substituiertes Phenyl ist, z.B. 4-Methylphenylsulfonyloxy. Ein anorganisches Fluorid ist in erster Linie ein Metallfluorid, wobei man insbesondere ein Alkalimetallfluorid, z.B. Natriumfluorid, oder ein Schwermetallfluorid, z.B. Silberfluorid verwendet. Die zusammen mit dem anorganischen Fluorid verwendeten Kronenäther sind gegebenenfalls substituierte 18-Kronen-6-äther, wie der Dicyclohexyl-18-kronen-6-äther.

Die Reaktion wird in Gegenwart eines inerten Lösungsmittels, insbesondere eines Nitrils, z.B. Acetonitril oder Propionnitril, oder eines Nitroniederalkans, z.B. Nitromethan oder Nitroäthan, unter im wesentlichen wasserfreien Bedingungen und, falls notwendig, unter Kühlen, z.B. in einem Temperaturbereich von etwa -20°C bis etwa 20°C, vorzugsweise bei etwa Raumtemperatur und gegebenenfalls in einer Inertgasatmosphäre durchgeführt. Entsprechende Verfahrensweisen sind beispielsweise in der DE-OS 2.636.962 beschrieben worden.

<u>Verfahren i) (Verätherung der 3-Hydroxygruppe):</u> Die Verätherung der freien Hydroxygruppe $R_1^a$ wird mit einem den gewünschten Kohlenwasserstoff einführenden konventionellen Verätherungsmittel durchgeführt.

Geeignete Verätherungsmittel sind beispielsweise entsprechende Diazoverbindungen, wie gegebenenfalls substituierte Diazoniederalkane, z.B. Diazomethan, Diazo-n-butan oder Diphenyldiazomethan. Diese Reagentien werden in Gegenwart eines geeigneten inerten Lösungsmittels, wie eines niederaliphatischen, cycloaliphatischen oder aromatischen Kohlenwasserstoffs, wie Hexan, Cyclohexan, Benzol oder Toluol, eines halogenierten niederaliphatischen Kohlenwasserstoffs, z.B. Methylenchlorid, oder eines Aethers, wie eines Diniederalkyläthers, z.B. Diäthyläther, oder eines cyclischen Aethers, z.B. Tetrahydrofuran oder Dioxan, oder eines Lösungsmittelgemisches, und je nach Diazoreagens unter Kühlen, bei Raumtemperatur oder unter leichtem Erwärmen, ferner, wenn notwendig, in einem geschlossenen Gefäss und/oder unter einer Inertgas-, z.B. Stickstoffatmosphäre, zur Anwendung gebracht.

Weitere geeignete Verätherungsmittel sind Ester entsprechender Alkohole, in erster Linie solche mit starken anorganischen oder organischen Säuren, wie Mineralsäuren, z.B. Halogenwasserstoffsäuren, wie Chlorwasserstoff-, Bromwasserstoff- oder Jodwasserstoffsäure, ferner Schwefelsäure oder Halogen-schwefelsäuren, z.B. Fluorsulfonsäure, oder starken organischen Sulfonsäuren, wie gegebenenfalls, z.B. durch Halogen, wie Fluor, substituierten Niederalkansulfonsäuren, oder aromatischen Sulfonsäuren, wie z.B. gegebenenfalls, z.B. durch Niederalkyl, wie Methyl, Halogen, wie Brom, und/oder Nitro substituierten Benzolsulfonsäuren, z.B. Methansulfon-, Trifluormethansulfon- oder p-Toluolsulfonsäure, z.B. Diniederalkylsulfate, wie Dimethylsulfat, ferner Fluorsulfonsäureniederalkylester z.B. Fluorsulfonsäuremethylester oder gegebenenfalls Halogen-substituierte Methansulfonsäure-niederalkylester, z.B. Trifluormethansulfonsäuremethylester. Sie werden üblicherweise in Gegenwart eines inerten Lösungsmittels, wie

eines gegebenenfalls halogenierten, wie chlorierten aliphatischen, cycloaliphatischen oder aromatischen Kohlenwasserstoffs, z.B. Methylenchlorid, eines Aethers, wie Dioxan oder Tetrahydrofuran, oder eines Gemisches verwendet. Dabei wendet man vorzugsweise geeignete Kondensationsmittel, wie Alkalimetallcarbonate oder -hydrogencarbonate, z.B. Natrium- oder Kaliumcarbonat oder -hydrogencarbonat (üblicherweise zusammen mit einem Sulfat), oder organische Basen, wie, üblicherweise sterische gehinderte, Triniederalkylamine, z.B. N,N-Diisopropyl-N-äthyl-amin (vorzugsweise zusammen mit Niederalkyl-halogensulfaten oder gegebenenfalls halogensubstituierten Methansulfonsäure-niederalkylestern) an, wobei unter Kühlen, bei Raumtemperatur oder unter Erwärmen, z.B. bei Temperaturen von etwa -20°C bis etwa 50°C und, wenn notwendig, in einem geschlossenen Gefäss und/oder in einer Inertgas-, z.B. Stickstoffatmosphäre gearbeitet wird.

Durch Phasentransfer-Katalyse (s. E.V. Dehmlow, Angewandte Chemie 5, 187 (1974)) kann die letztgenannte Verätherungsreaktion wesentlich beschleunigt werden. Als Phasentransfer-Katalysatoren können quartäre Phosphoniumsalze und insbesondere quartäre Ammoniumsalze, wie gegebenenfalls substituierte Tetraalkylammoniumhalogenide, z.B. Tetrabutylammoniumchlorid, -bromid oder -jodid, oder auch Benzyl-triäthylammoniumchlorid in katalytischen oder bis zu äquimolaren Mengen verwendet werden. Als organische Phase kann irgendeines der mit Wasser nicht mischbaren Lösungsmittel dienen, beispielsweise einer der gegebenenfalls halogenierten, wie chlorierten niederaliphatischen, cycloaliphatischen oder aromatischen Kohlenwasserstoffe, wie Tri- oder Tetrachloräthylen, Tetrachloräthan, Tetrachlorkohlenstoff, Chlorbenzol oder auch Toluol oder Xylol. Die als Kondensationsmittel geeigneten Alkalimetallcarbonate oder

-hydrogencarbonate, z.B. Kalium- oder Natriumcarbonat oder -hydrogencarbonat, Alkalimetallphosphate, z.B. Kaliumphosphat und Alkalimetallhydroxide, z.B. Natriumhydroxid, können bei basenempfindlichen Verbindungen der Reaktionsmischung titrimetrisch, z.B. mittels eines Titrierautomaten zugesetzt werden, damit der pH-Wert während der Verätherung zwischen etwa 7 und 8.5 bleibt.

Weitere Verätherungsmittel sind entsprechende Acetale, z.B. gem-Niederalkoxyniederalkane, wie 2,2-Dimethoxypropan, die in Gegenwart einer starken organischen Sulfonsäure, wie p-Toluolsulfonsäure, und eines geeigneten Lösungsmittels, wie eines Diniederalkyl- oder Niederalkylensulfoxyds, z.B. Dimethylsulfoxyd, zur Anwendung gelangen, oder Orthoester, z.B. Orthoameisensäure-triniederalkylester, z.B. Orthoameisensäure-triäthylester, die in Gegenwart einer starken Mineralsäure, z.B. Schwefelsäure, oder einer starken organischen Sulfonsäure, wie p-Toluolsulfonsäure, und eines geeigneten Lösungsmittels, wie eines Aethers, z.B. Dioxan, verwendet werden. Wichtige Verätherungsmittel sind auch entsprechende Tri-$R_1^o$-oxoniumsalze (sogenannte Meerweinsalze), worin $R_1^o$ den einzuführenden Rest darstellt, beispielsweise Triniederalkyloxoniumsalze, insbesondere die entsprechenden Salze mit komplexen, fluorhaltigen Säuren, wie die entsprechenden Tetrafluorborate, Hexafluorphosphate, Hexafluorantimonate oder Hexachlorantimonate, beispielsweise Trimethyloxonium-tetrafluorborat. Man verwendet diese Verätherungsmittel vorzugsweise in einem inerten Lösungsmittel, wie einem Aether oder einem halogenierten niederaliphatischen Kohlenwasserstoff, z.B. Tetrahydrofuran oder Methylenchlorid, oder in einem Gemisch davon, wenn notwendig, in Gegenwart einer Base, wie einer organischen Base, z.B. eines, vorzugsweise sterisch gehinderten, Triniederalkylamins, z.B. N,N-Diisopropyl-N-äthyl-amin, und unter Kühlen, bei Raumtemperatur oder unter leichtem Erwärmen, z.B. bei etwa -20°C bis etwa 50°C, wenn notwendig, in einem geschlossenen Gefäss und/oder in einer Inertgas-, z.B. Stickstoffatmosphäre.

Weitere Verätherungsmittel sind schliesslich entsprechende 1-$R_1^o$-3-Aryltriazenverbindungen, worin $R_1^o$ den einzuführenden Rest und Aryl vorzugsweise einen gegebenenfalls substituierten Phenylrest, z.B. Niederalkylphenyl, wie 4-Methylphenyl bedeutet. Charakteristische Beispiele für solche Triazenverbindungen sind 3-Aryl-1-niederalkyl-triazene, z.B. 3-(4-Methylphenyl)-1-äthyl-triazen. Diese Reagentien werden üblicherweise in Gegenwart von inerten Lösungsmitteln, wie gegebenenfalls halogenierten niederaliphatischen Kohlenwasserstoffen, Aethern oder Gemischen davon, unter Kühlen, bei Raumtemperatur und vorzugsweise bei erhöhter Temperatur, z.B. bei etwa 20°C bis etwa 100°C, verwendet. Entsprechende Verfahrensweisen sind beispielsweise in der DE-OS 2.636.962 beschrieben worden.

<u>Verfahren j) (Austausch einer veresterten oder verätherten Hydroxylgruppe $R_1^a$ gegen eine Thioäthergruppe):</u> Eine durch ein Thiol substituierbare veresterte Hydroxygruppe $R_1^a$ ist durch eine Halogenwasserstoffsäure, wie Chlorwasserstoffsäure oder eine vom Schwefel abgeleitete starke Säure, wie Schwefel- oder eine Sulfonsäure, verestert. Eine durch ein Thiol substituierbare verätherte Hydroxygruppe $R_1^a$ ist durch einen niederaliphatischen, cycloaliphatischen, aromatischen oder arylniederaliphatischen Rest, vorzugsweise durch $C_1$-$C_4$-Niederalkyl, z.B. Methyl oder Aethyl, $C_2$-$C_6$-Niederalkenyl, z.B. Vinyl oder Propenyl, $C_3$-$C_7$-Cycloalkyl, z.B. Cyclopropyl oder Cyclohexyl, Phenyl, oder Phenylniederalkyl, wie Benzyl, veräthert.

Besonders leicht gegen eine Thioäthergruppe $R_1$ austauschbare Reste $R_1^a$ sind Chlor, Brom, Niederalkansulfonyloxy, wie Methansulfonyloxy, und Arylsulfonyloxy, wie p-Toluolsulfonyloxy.

Die Substitution dieser Gruppe $R_1^a$ durch die verätherte Mercaptogruppe findet in an sich bekannter Weise unter Kühlen, bei Raumtemperatur oder unter Erwärmen, d.h. bei Temperaturen zwischen 0° bis etwa 40°C, in einem geeigneten inerten Lösungsmittel und in Gegenwart einer Base statt. Geeignete Basen sind insbesondere sterisch gehinderte Amine, wie Triniederalkylamine, z.B. N,N-Diisopropyl-N-äthylamin, bicyclische Amidine, wie Diazabicycloalkane, z.B. 1,5-Diazabicyclo[4.3.0]non-5-en oder 1.5-Diazabicyclo-[5.4.0]undec-5-en, sowie Alkalimetallhydride, -amide oder -niederalkanolate, wie Natriumhydrid, Natrium- oder Lithiumamid, Natriumäthylat oder Kalium-tert.-butylat. Die Reaktion kann in einer Inertgas-, wie Stickstoffatmosphäre und, wenn notwendig, in einem geschlossenen Gefäss unter Druck durchgeführt werden.

Falls $R_1^a$ im Ausgangsmaterial eine verätherte Hydroxygruppe ist, kann unter den basischen Bedingungen zunächst eine Addition des Thiols $R_1$-H an die Doppelbindung stattfinden, worauf der Alkoholrest $R_1^a$ -H abgespalten werden muss.

Die Abspaltung des Alkohols $R_1^a$-H findet in Gegenwart einer Säure, in An- oder Abwesenheit eines geeigneten, inerten Lösungsmittels und unter Kühlen, bei Raumtemperatur oder unter Erwärmen, d.h. bei Temperaturen zwischen etwa -70° und +100°C, bevorzugt bei etwa 5° bis etwa 40°C, statt. Zur Abspaltung des Alkohols $R_1$-H geeignete Säuren sind starke Protonensäuren, insbesondere entsprechende Mineralsäuren, wie Salzsäure, ferner Sulfonsäuren, wie Niederalkansulfonsäuren, z.B. Methansulfonsäure, oder aromatische Sulfonsäuren, z.B. Benzol- oder Toluolsulfonsäure, halogenierte Niederalkancarbonsäuren, wie Trifluor- oder Trichloressigsäure oder auch Ameisensäure. Geeignete Lösungsmittel sind die bei der basischen Addition genannten. Entsprechende Verfahrensweisen sind beispielsweise in der DE-OS 2.537.974 beschrieben worden.

Verfahren k) (Decarbonylierung): Die Decarbonylierung der Formylgruppe wird in erster Linie durch Behandeln mit einem Kohlenmonoxyd-aufnehmenden Schwermetallkomplex durchgeführt. Solche Schwermetallkomplexe sind insbesondere Platinmetall-Komplexe, wobei man unter einem Platinmetall ausser Platin auch Iridium, Rhodium, Palladium und Osmium versteht.

Vorzugsweise verwendet man bis-trisubstituierte Phosphin-platinhalogenide, bis-trisubstituierte Phosphincarbonyliridiumhalogenide tris-trisubstituierte Phosphin-iridiumhalogenide und in erster Linie tris-trisubstituierte Phosphin-rhodiumhalogenide, worin die Substituenten des Phosphins vorzugsweise Niederalkyl, z.B. n-Butyl, und in erster Linie Phenyl darstellen, und die Halogenide in erster Linie Chloride sind. Solche Phosphinplatinmetall-Komplexe, welche Kohlenmonoxyd in kovalenter Bindung aufzunehmen vermögen, sind z.B. Bis-triphenylphosphin-platin-II-chlorid $[(C_6H_5)_3P]_2PtCl_2$, oder Bis-triphenyl-phosphincarbonyliridium-II-chlorid, $[(C_6H_5)_3P]_2\ Ir(CO)Cl$, sowie Tris-triphenylphosphin-iridium-I-chlorid, $[(C_6H_5)_3P]_3Ir\ Cl$, in erster Linie aber Tris-triphenyl-phosphin-rhodium-I-chlorid, $[(C_6H_5)_3P]_3RhCl$.

Wenn erwünscht oder notwendig, kann die Decarbonylierung mit den obengenannten Schwermetallkomplexen in Gegenwart von geeigneten Katalysatoren oder Aktivierungsmitteln, z.B. Lewissäuren, wie Bortrifluorid (das man z.B. zusammen mit dem Bis-triphenylphosphin-platinchlorid verwenden kann), ferner eines Perchlorats, wie Alkalimetallperchlorates, z.B. Natriumperchlorat (das man z.B. zusammen mit Bis-triphenylphosphin-carbonyliridiumchlorid verwenden kann), durchgeführt werden.

Vorzugsweise arbeitet man in Gegenwart von inerten Lösungsmitteln, insbesondere von Kohlenwasserstoffen, wie aliphatischen oder cycloaliphatischen, insbesondere aromatischen Kohlenwasserstoffen, z.B. Benzol, Toluol oder Xylol, halogenierten, wie chlorierten Kohlenwasserstoffen, wie entsprechenden niederaliphatischen oder aromatischen Kohlenwasserstoffen, z.B. Methylenchlorid oder Chlorbenzol, Aethern, wie niederaliphatischen, niederaliphatisch-aromatischen, ferner cyclischen, Aethern, z.B. Di-n-butyläther, Anisol oder Dioxan, Nitrilen, wie niederaliphatischen Nitrilen, z.B. Acetonitril oder Ketonen, wie Niederalkanonen, z.B. Aceton, oder Gemischen von solchen Lösungsmitteln. Dabei wird die Reaktion unter Kühlen, bei Zimmertemperatur oder unter Erwärmen, z.B. bei etwa 10° bis etwa 150°C, wie bei etwa 40° bis etwa 120°C, ferner, wenn notwendig, in einem geschlossenen Gefäss und/oder in einer inerten Gasatmosphäre, z.B. unter Stickstoff oder Argon, durchgeführt. Entsprechende Verfahrensweisen sind beispielsweise in der DE-OS 2.151.567 beschrieben worden.

Verfahren 1) (Austausch einer freien oder veresterten Hydroxy- oder gegebenenfalls substituierten Aminogruppe gegen Wasserstoff): Eine gegen Wasserstoff austauschbare veresterte Hydroxygruppe $R_1^a$ ist vorzugsweise eine durch eine starke organische oder anorganische Säure, wie eine starke Mineralsäure, z.B. einen Halogenwasserstoff, wie Chlorwasserstoff oder eine organische Sulfonsäure, wie eine entsprechende niederaliphatische oder aromatische Sulfonsäure, veresterte Hydroxygruppe. So stellt $R_1^a$ z.B. Halogen, wie Chlor, Niederalkylsulfonyloxy, z.B. Methylsulfonyloxy oder Aethylsulfonyloxy oder Arylsulfonyloxy, z.B. p-Toluolsulfonyloxy dar. Ein gegebenenfalls niederalkylierter, durch Niederalkylen oder Heteroniederalkylen cyclisch disubstituierter Aminorest $R_1^a$ hat die oben für diese Reste angegebene Bedeutung und ist beispielsweise Amino, Methylamino, Dimethylamino, Diäthylamino, Pyrrolidino, Piperidino oder Morpholino.

Der Ersatz der Gruppe $R_1^a$ in einer Verbindung der Formel XI durch Wasserstoff erfolgt entweder in zwei Stufen durch Reduktion der $C_3$-$C_4$-Doppelbindung und anschliessende Abspaltung von $R_1^a$-H oder einstufig, durch unmittelbare hydrierende Abspaltung von $R_1^a$.

Beim zweistufigen Austausch von $R_1^a$ gegen Wasserstoff erfolgt die Reduktion der $C_3$-$C_4$-Doppelbindung in Ausgangsstoffen der Formel XI z.B. mittels geeigneter Hydrid-Reduktionsmittel. Geeignete Hydrid-Reduktionsmittel sind in erster Linie komplexe Metallhydride, vorzugsweise entsprechende Borhydride, wie Diboran oder Alkalimetallborhydride, z.B. Natriumborhydrid oder Lithiumborhydrid, ferner Zinkborhydrid, sowie organische Alkalimetallaluminiumhydride, wie Tri-Niederalkoxy-alkalialuminiumhydride, z.B. Tri-(tert.-

butyloxy)-lithiumaluminiumhydrid, die man üblicherweise in Gegenwart von Lösungsmitteln, insbesondere von relativ polaren Lösungsmitteln, wie Alkoholen, z.B. Niederalkanolen, wie Methanol oder Aethanol, oder Aethern, wie aliphatischen Aethern, z.B. Glycol- und Polyglycoläthern, wie Aethylenglycoldimethyläther oder Diäthylenglycoldimethyläther, oder cyclischen Aethern, wie Tetrahydrofuran oder Dioxan, oder Lösungsmittelgemischen, auch von wässrigen Lösungsmitteln, anwendet, wobei man bei Temperaturen von etwa -20° bis etwa 80°C, wenn notwendig, in einem geschlossenen Gefäss und/oder in einer Inertgas-, z.B. Stickstoffatmosphäre arbeitet. Falls diese Reduktion in Gegenwart geeigneter Wasser-, Säure- oder Amin-abspaltender Mittel durchführt wird, kann die erwünschte 3-unsubstituierte 3-Cephem-Verbindung unmittelbar erhalten werden. Es kann aber auch zunächst eine 3-$R_1^a$-Cepham-Verbindung entstehen, die entweder isoliert oder in situ weiterverarbeitet werden kann. (Diese 3-$R_1^a$-Cepham-Ausgangsverbindungen lassen sich auch auf anderem Wege, z.B. durch Acylierung einer entsprechenden 7-β-Amino-3-$R_1^a$-cepham-Verbindung, erhalten, vergl. unter Formel XXIV.

In der zweiten Verfahrensstufe erfolgt die Abspaltung der Elemente einer Verbindung der Formel $R_1^a$-H, d.h. einer Säure, eines Amins, oder insbesondere von Wasser, aus einer erhaltenen 3-$R_1^a$-Cepham-Verbindung, wobei man diese vorzugsweise mit geeigneten Wasser-, Säure- oder Amin-abspaltenden Mitteln behandelt. Wasser und Amine werden vorzugsweise mittels einer starken organischen Carbon-, wie einer Halogen-niederalkancarbonsäure oder Sulfonsäure, z.B. p-Toluolsulfonsäure oder Methansulfonsäure, abgespalten. Wasser kann insbesondere auch mittels eines geeigneten wasserbindenden Säurederivats, wie eines Anhydrids oder insbesondere Säurehalogenids, wie Säurechlorids, einer vom Phosphor oder

Schwefel abgeleiteten Säure, wie einer entsprechenden anorganischen Säure, z.B. mittels Phosphoroxychlorid oder Thionylchlorid oder einer Sulfonsäure, wie einer niederaliphatischen oder aromatischen Sulfonsäure, z.B. mittels Tosylchlorid oder Mesylchlorid, abgespalten werden. Man arbeitet dabei vorzugsweise in Gegenwart einer Base, wie eines tertiären , z.B. Triniederalkylamins oder heterocyclischen Amins, z.B. von Triäthylamin oder Pyridin, oder eines geeigneten Ionenaustauschers, z.B. eines mit Kationen beladenen sulfonierten Polystyrol-Ionenaustauschers.

Ferner kann man zur Wasserabspaltung auch dehydratisierende Carbodiimidverbindungen, z.B. Dicyclohexylcarbodiimid, oder dehydratisierende, über Stickstoffatome disubstituierte Carbonylverbindungen, z.B. Carbonyldiimidazol, verwenden. Dabei verwendet man diese Mittel üblicherweise in Gegenwart eines Lösungsmittels, wie eines gegebenenfalls halogenierten niederaliphatischen oder aromatischen Kohlenwasserstoffs, z.B. Benzol oder Toluol, oder eines Lösungsmittelgemisches, wobei man geeignete saure Mittel, wie Trifluoressigsäure, gleichzeitig auch als Lösungsmittel verwenden kann.Wenn notwendig, verwendet man zusätzlich ein wasser-adsorbierendes Mittel oder einen Wasserabscheider und arbeitet unter Kühlen oder Erwärmen und/oder in einer Inertgas-, z.B. Stickstoffatmosphäre.

Die einstufige hydrierende Abspaltung einer Gruppe $R_1^a$ aus einer Verbindung der Formel XI gelingt am besten, wenn $R_1^a$ Halogen, wie Chlor oder eine veresterte Hydroxygruppe, wie eine durch eine Sulfonsäure, wie eine niederaliphatische oder aromatische Sulfonsäure, z.B. Methansulfonsäure oder Toluolsulfonsäure, veresterte Hydroxygruppe ist. In diesem Falle hydriert man vorzugsweise mit Hilfe von metallischen Reduktionsmitteln ("naszierendem Wasserstoff"), z.B. von

Aluminium oder insbesondere von Zink, oder von reduzierenden Metallverbindungen, z.B. entsprechenden Metallegierungen, Amalgamen oder Salzen, z.B. Aluminiumamalgam, die man üblicherweise in Gegenwart von schwachen Protonendonatoren, wie Wasser, Alkohol (z.B. Methanol oder Aethanol) oder einer niederaliphatischen Carbonsäure, z.B. Essigsäure oder Ameisensäure anwendet. Zink wendet man beispielsweise in Gegenwart von Eisessig oder Ameisensäure, ein Amalgam z.B. in Gegenwart eines wasserhaltigen inerten organischen Lösungsmittels, wie eines Aethers, an.

Gewisse veresterte Hydroxygruppen $R_1^a$, insbesondere Sulfonyloxy, z.B. Methylsulfonyloxygruppen, lassen sich in Form einer Säure der Formel $R_1^a$ -H auch durch Adsorption, z.B. an Silikagel, Aluminiumoxyd, etc., und Elution (Chromatographie), abspalten.

Bevorzugt wird in einer Verbindung der Formel XI eine veresterte Hydroxygruppe $R_1^a$, wie eine 3-Halogen-, wie 3-Chlor- oder 3-Brom-, eine 3-Toluolsulfonyloxy- oder eine 3-Methansulfonyloxygruppe durch Behandlung mit Zink in Eisessig oder Ameisensäure und eine tertiäre Aminogruppe $R_1^a$, wie 3-Morpholino oder 3-Pyrrolidino, durch Behandlung mit Diboran und anschliessend mit Eisessig, durch Wasserstoff ersetzt. Entsprechende Verfahrensweisen sind z.B. in der DE-OS 2.620.308 beschrieben.

Die Abspaltung von $R_1^a$-H wird üblicherweise in Gegenwart eines Lösungsmittels durchgeführt. Bei Anwendung von anorganischen Basen kann man dabei in Wasser, einem wasserlöslichen organischen Lösungsmittel, wie Aceton oder Dioxan oder wässrigen Gemischen davon und bei einem pH von höchstens 9, bei Anwendung von Aminen vorzugsweise auch in einem gegebenenfalls halogenierten, wie chlorierten, niederaliphatischen, cycloaliphatischen oder aromatischen Kohlenwasserstoff, wie Methylenchlorid, einem Niederalkanon, z.B. aceton, oder Aether, z.B. Tetrahydrofuran oder Dioxan, oder einem entsprechendem Lösungsmittelgemisch inkl. einem wässrigen Gemisch, wenn notwendig unter Kühlen oder Erwärmen und/oder in einer Inertgas-, z.B. Stickstoffatmosphäre, arbeiten.

Verfahren m) (Einführung eines Carbonylmethylrestes): Die 3-Hydroxy-Ausgangsverbindung der Formel XI ($R_1^a$ = OH, die 4-Carboxylgruppe liegt in geschützter Form vor) kann mit der dazu tautomeren 3-Oxo-cepham-4-carbonsäure-verbindung im Gleichgewicht stehen, wobei letztere durch Reaktion mit dem Ylid laufend aus dem Gleichgewicht entfernt wird.

In einem Ylid der Formel XII ist die Gruppe $X^{\oplus}$ eine der bei Kondensationsreaktionen analog Wittig gebräuchlichen dreifach substituierten Phosphonium- oder Phosphonogruppen, insbesondere eine Triaryl-, wie Triphenyl-, oder Triniederalkyl-, wie Tributylphosphoniumgruppe, oder eine durch Niederalkyl, wie Aethyl, zweifach verätherte Phosphonogruppe, wobei Triphenylphosphonium und Diäthylphosphono bevorzugt sind, und wobei das Symbol $X^{\oplus}$ für den Fall der Phosphonogruppe noch das Kation einer starken Base umfasst.

In Phosphoniumverbindungen der Formel XII, die in der isomeren Ylenform auch als Phosphorane bezeichnet werden, z.B. Carbomethoxymethylentriphenylphosphoran, wird die negative Ladung durch die positive geladene Phosphoniumgruppe neutralisiert. Phosphonoverbindungen der Formel XII, die in ihrer isomeren Form auch als Phosphonate bezeichnet werden, werden von einem Kation einer starken Base neutralisiert, das je nach Herstellungsweise, beispielsweise ein Alkalimetall-, wie Kalium-, Natrium- oder Lithium-ion sein kann. Die Phosphonate werden in dieser Form, d.h. als Salze,

in die Reaktion eingesetzt.

Die obige Kondensationsreaktion wird in einem geeigneten inerten Lösungsmittel durchgeführt, beispielsweise in einem gegebenenfalls halogenierten niederaliphatischen, cycloaliphatischen oder aromatischen Kohlenwasserstoff, wie Hexan, Cyclohexan, Benzol, Toluol oder Methylenchlorid, einem Aether, wie einem Diniederalkyläther, z.B. Diäthyläther, einem Diniederalkoxyniederalkan, wie Dimethoxyäthan, einem cyclischen Aether, wie Dioxan oder Tetrahydrofuran, einem Diniederalkylamid, wie Dimethylformamid, einem Diniederalkylsulfoxid, wie Dimethylsulfoxid, oder auch einem Niederalkanol, z.B. Methanol oder Aethanol oder in einem Gemisch davon, bei erhöhter Temperatur, wie bei etwa 50° bis 150°C, bevorzugt bei etwa 60° bis 100°C, gewünschtenfalls in einer Inertgas-, wie Stickstoffatmosphäre.

In der obigen Wittig-Reaktion kann man je nach Ausgangsmaterial und Reaktionsbedingungen einheitliche Verbindungen der Formel I oder deren einheitliche Isomere, worin $R_1$ den Exomethylenrest der Teilformel (B) darstellt, oder Gemische der beiden isomeren Formen erhalten. Erhaltene Gemische können in an sich bekannter Weise, z.B. mit Hilfe von geeigneten Trennmethoden, z.B. durch Adsorption und fraktionierte Elution, inkl. Chromatographie (Säulen-, Papier- oder Plattenchromatographie) unter Verwendung von geeigneten Adsorptionsmitteln, wie Silikagel oder Aluminiumoxyd, und Elutionsmitteln, ferner durch fraktioniertes Kristallisieren, Lösungsmittelverteilung, etc. aufgetrennt werden. Entsprechende Verfahrensweisen sind beispielsweise in der DE-OS 2.637.176 beschrieben.

Nachoperationen

1. Abspaltung und Umwandlung von Schutzgruppen: In einer erhaltenen Verbindung der Formel I, worin mindestens eine der funktionellen Gruppen in geschützter Form vorliegt, können diese Schutzgruppen auf übliche, an sich bekannte Weise abgespalten oder gegen andere Schutzgruppen ausgetauscht werden. Geschützte funktionelle Gruppen, wie entsprechende Carboxyl-, Amino-, Hydroxy- und/oder Mercaptogruppen kann man beispielsweise mittels Solvolyse, insbesondere Hydrolyse, Alkoholyse oder Acidolyse, mittels Reduktion, insbesondere Hydrogenolyse oder chemische Reduktion, gegebenenfalls schrittweise oder gleichzeitig freisetzen. So kann man z.B. eine geschützte Carboxylgruppe, wie eine gegebenenfalls in 2-Stellung durch Silylgruppen substituierte Niederalkoxycarbonylgruppe, eine Polycycloalkoxycarbonyl- oder eine Diarylmethoxycarbonylgruppe, durch Behandeln mit einem geeigneten sauren Mittel, wie Ameisensäure oder Trifluoressigsäure, gegebenenfalls unter Zugabe einer nucleophilen Verbindung, wie Phenol oder Anisol, in die freie Carboxylgruppe überführen. Eine gegebenenfalls substituierte Benzyloxycarbonylgruppe kann z.B. mittels Hydrogenolyse durch Behandeln mit Wasserstoff in Gegenwart eines Hydrierkatalysators, wie eines Palladiumkatalysators, freigesetzt werden. Ferner kann man bestimmte substituierte Benzyloxycarbonylgruppen, wie 4-Nitrobenzyloxycarbonyl, auch mittels chemischer Reduktion, z.B. durch Behandeln mit einem Alkalimetall-, z.B. Natriumdithionit, oder mit einem reduzierenden Metall, z.B. Zink, oder Metallsalz, wie einem Chrom-IIsalz, z.B. Chrom-II-chlorid, üblicherweise in Gegenwart eines wasserstoffabgebenden Mittels, das zusammen mit dem Metall nascierenden Wasserstoff zu erzeugen vermag, wie einer Säure, in erster Linie einer Carbonsäure oder α-Hydroxycarbonsäure, wie insbesondere Essigsäure, Ameisensäure, Glycolsäure, Milchsäure oder Weinsäure, oder eines vorzugsweise

wässrigen Niederalkanols, in die freie Carboxylgruppe überführen. Durch Behandeln mit einem reduzierenden Metall oder Metallsalz, wie oben beschrieben, kann man auch eine 2-Halogen-niederalkoxycarbonylgruppe (gegebenenfalls nach Umwandlung einer 2-Brom-niederalkoxycarbonylgruppe in eine 2-Iodniederalkoxycarbonylgruppe) oder eine Acylmethoxycarbonylgruppe in die freie Carboxylgruppe umwandeln. Eine Aroylmethoxycarbonylgruppe kann auch durch Behandeln mit einem nucleophilen Reagens, wie Natriumthiophenolat oder Natriumjodid, gespalten werden. Eine substituierte Silyläthoxycarbonylgruppe kann auch durch Behandeln mit einem dissoziierbaren, löslichen Salz der Fluorwasserstoffsäure, wie einem Alkalimetallfluorid, z.B. Natrium- oder Kaliumfluorid, in Anwesenheit eines macrocyclischen Polyäthers ("Kronenäther"), oder mit dem Fluorid einer organischen quaternären Base, wie Tetraalkylammoniumfluorid oder Trialkylarylammoniumfluorid, z.B. Tetraäthylammoniumfluorid oder Tetrabutylammoniumfluorid, in Gegenwart eines aprotischen polaren Lösungsmittels, wie Dimethylsulfoxid oder N,N-Dimethylacetamid, in die freie Carboxylgruppe übergeführt werden. Eine Polyhalogenaryloxycarbonylgruppe, wie die Pentachlorphenyloxycarbonylgruppe, wird unter milden basischen Bedingungen, wie durch verdünnte Natronlauge oder organische Basen in Gegenwart von Wasser, zur freien Carboxylgruppe verseift.

Eine z.B. durch Silylierung oder Stannylierung geschützte Carboxylgruppe kann in üblicher Weise, z.B. durch Behandeln mit Wasser oder einem Alkohol, freigesetzt werden, was normalerweise bereits bei der Aufarbeitung der Reaktionslösung geschieht.

Eine geschützte Aminogruppe wird in an sich bekannter und je nach Art der Schutzgruppe in verschiedenartiger Weise, z.B. mittels Solvolyse oder Reduktion, freigesetzt.

Eine 2-Halogen-niederalkoxycarbonylaminogruppe (gegebenenfalls nach Umwandlung einer 2-Brom-niederalkoxycarbonylgruppe in eine 2-Jod-niederalkoxycarbonylgruppe), eine Acylmethoxycarbonylaminogruppe oder eine 4-Nitrobenzyloxycarbonylaminogruppe kann z.B. durch Behandeln mit einem geeigneten chemischen Reduktionsmittel, wie Zink in Gegenwart einer Carbon- oder α-Hydroxycarbonsäure, wie wässriger Essigsäure, eine Aroylmethoxycarbonylaminogruppe auch durch Behandeln mit einem nucleophilen, vorzugsweise salzbildenden Reagens, wie Natriumthiophenolat, und eine 4-Nitrobenzyloxycarbonylaminogruppe auch durch Behandeln mit einem Alkalimetall-, z.B. Natriumdithionit, eine Diphenylmethoxycarbonylamino-, tert.-Niederalkoxycarbonylamino-, 2-$(S_1)(S_2)(S_3)$-Silyläthoxycarbonylamino- oder Polycycloalkoxycarbonylaminogruppe durch Behandeln z.B. mit Ameisen- oder Trifluoressigsäure, eine gegebenenfalls substituierte Benzyloxycarbonylaminogruppe z.B. mittels Hydrogenolyse durch Behandeln mit Wasserstoff in Gegenwart eines Hydrierkatalysators, wie eines Palladiumkatalysators, eine Triarylmethylgruppe z.B. durch Behandeln mit wässriger Mineralsäure, und eine mit einer organischen Silyl- oder Stannylgruppe geschützte Aminogruppe z.B. mittels Hydrolyse oder Alkoholyse freigesetzt werden. Eine durch 2-Halogenacetyl, wie 2-Chloracetyl, geschützte Aminogruppe kann durch Behandeln mit Thioharnstoff in Gegenwart einer Base, oder mit einem Thiolatsalz, wie einem Alkalimetallthiolat des Thioharnstoffs und anschliessende Solvolyse, wie Alkoholyse oder Hydrolyse, des entstandenen Kondensationsproduktes freigesetzt werden. Eine durch eine substituierte-Silyläthoxycarbonylgruppe geschützte Aminogruppe kann auch durch Behandeln mit einem Salz der Fluorwasserstoffsäure, das Fluoridanionen liefert, wie oben bei der Freisetzung einer entsprechend geschützten Carboxylgruppe angegeben, in die freie Aminogruppe übergeführt werden. Eine Phosphor-, Phosphon- oder Phosphinamidogruppe kann durch Behandeln mit

einer Phosphor-haltigen Säure, wie einer Phosphor-, Phosphon- oder Phosphinsäure, z.B. Orthophosphorsäure oder Polyphosphorsäure, einem sauren Ester davon, z.B. Monomethyl-, Monoäthyl-, Dimethyl- oder Diäthylphosphat oder Monomethylphosphonsäure, oder einem Anhydrid davon, wie Phosphorpentoxid, und dergleichen, in die freie Aminogruppe übergeführt werden.

Eine in Form einer Azidogruppe geschützte Aminogruppe wird in an sich bekannter Weise, durch Reduktion, in die freie Aminogruppe übergeführt, beispielsweise durch katalytische Hydrierung mit Wasserstoff und einem Hydrierkatalysator, wie Platinoxid, Palladium, oder auch Raney-Nickel, oder auch durch Zink und Säure, wie Essigsäure. Die katalytische Hydrierung wird bevorzugt in einem inerten Lösungsmittel, wie einem halogenierten Kohlenwasserstoff, z.B. Methylenchlorid, oder auch in Wasser oder einem Gemisch von Wasser und einem organischen Lösungsmittel, wie einem Alkohol oder Dioxan, bei etwa 20 bis 25°, oder auch bei erniedrigter oder erhöhter Temperatur, durchgeführt.

Eine durch eine Acylgruppe, eine Silyl- oder Stannylgruppe oder durch einen gegebenenfalls substituierten α-Phenylniederalkylrest geschützte Hydroxygruppe wird wie eine entsprechend geschützte Aminogruppe freigesetzt. Eine durch 2,2-Dichloracetyl geschützte Hydroxygruppe wird durch basische Hydrolyse und eine durch einen 2-oxa- oder 2-thiaaliphatischen oder -cycloaliphatischen Kohlenwasserstoffrest geschützte Hydroxygruppe wird durch Acidolyse freigesetzt.

Bevorzugt werden die Schutzgruppen bereits beim Syntheseverfahren so gewählt, dass sie später alle gleichzeitig abgespalten werden können, beispielsweise acidolytisch, wie durch Behandeln mit Trifluoressigsäure oder Ameisensäure, oder reduktiv, wie durch Behandeln mit Zink und Eisessig, oder mit Wasserstoff und einem Hydrierkatalysator, wie einem Palladium/Kohle-Katalysator.

Die beschriebenen Spaltungsreaktionen werden unter an sich bekannten Bedingungen durchgeführt, wenn notwendig unter Kühlen oder Erwärmen, in einem geschlossenen Gefäss und/oder in einer Inertgas-, z.B. Stickstoffatmosphäre.

Ueber die vorgenannten Schutzgruppenumwandlungen hinaus können beispielsweise mittels der nachstehenden Verfahren erfindungsgemässe Verbindungen der Formel I durch Umwandlung einer erfindungsgemäss definierten Gruppe $R_1$ bis $R_4$ in eine andere erfindungsgemäss definierte Gruppe $R_1$ bis $R_4$ in andere erfindungsgemässe Verbindungen der Formel I umgewandelt werden.

2. Veresterung der 4-Carboxylgruppe: Die Ueberführung einer freien Carboxylgruppe $-C(=O)-R_2$ in einer erhaltenen Verbindung der Formel I in eine veresterte Carboxylgruppe $-C(=O)OR_5$, die unter physiologischen Bedingungen spaltbar ist, erfolgt nach an sich bekannten Veresterungsmethoden. Beispielsweise kann man eine Verbindung der Formel I, worin andere funktionelle Gruppen, wie Amino-, Hydroxy- oder weitere gegebenenfalls vorhandene Carboxylgruppen, vorzugsweise in geschützter Form vorliegen und worin die zu veresternden Carboxylgruppe in freier Form, als Salz oder als reaktionsfähige funktionelle Gruppe vorliegt, mit einem entsprechenden Alkohol oder einem reaktionsfähigen funktionellen Derivat davon, verestern. Beispiele für die dabei übliche Verfahrensweise finden sich in den bei der Definition der physiologisch spaltbaren Estergruppen genannten Patentschriften.

3. Austausch einer Gruppe $R_6$ in einem Rest der Teilformel (A) oder (B) gegen einen anderen Rest $R_6$: In einer Verbindung der Formel I, worin $R_1$ als gegebenenfalls substituierter Carbonylmethylrest eine Gruppe der Teilformel (A) oder (B), worin $R_6$ eine freie Hydroxygruppe ist, bedeutet, kann eine solche in an sich bekannter Weise in eine Niederalkoxygruppe übergeführt werden. Man erhält solche Ester z.B. durch Behandeln mit einer geeigneten Diazoverbindung, wie einem Diazoniederalkan, z.B. Diazomethan oder Diazobutan, wenn notwendig, in Gegenwart einer Lewissäure, wie z.B. Bortrifluorid, oder durch Umsetzen mit einem zur Veresterung geeigneten Alkohol in Gegenwart eines Veresterungsmittels, wie eines Carbodiimids, z.B. Dicyclohexylcarbodiimid, sowie Carbonyldiimidazol, ferner mit einem N,N'-disubstituierten O- bzw. S-substituierten Isoharnstoff oder Isothioharnstoff, worin ein O- und S-Substituent z.B. Niederalkyl, insbesondere tert.-Butyl, und N- bzw. N'-Substituenten z.B. Niederalkyl, insbesondere Isopropyl, Cycloalkyl oder Phenyl sind, oder nach irgendeinem anderen bekannten und geeigneten Veresterungsverfahren, wie Reaktion eines Salzes der Säure mit einem reaktionsfähigen Ester eines Alkohols und einer starken anorganischen Säure, sowie einer starken organischen Sulfonsäure. Ferner können z.B. intermediär erhaltene Säurehalogenide, wie -chloride (hergestellt z.B. durch Behandeln mit Oxalylchlorid), aktivierte Ester (gebildet z.B. mit N-Hydroxystickstoffverbindung, wie N-Hydroxy-succinimid) oder gemischte Anhydride (erhalten z.B. mit Halogenameisensäureniederalkylestern, wie Chlorameisensäureäthyl- oder Chlorameisensäureisobutylester, oder mit Halogenessigsäurehalogeniden, wie Trichloressigsäurechlorid) durch Umsetzen mit Alkoholen, gegebenenfalls in Gegenwart einer Base, wie Pyridin, in eine veresterte Carboxylgruppe übergeführt werden.

In einer erhaltenen Verbindung der Formel I, worin $R_1$ eine Gruppe der Teilformel (A) oder (B) ist, worin $-C(=O)-R_6$ eine freie Carboxylgruppe bedeutet, kann diese direkt mit einer äquivalenten oder leicht überschüssigen Menge eines sekundären Amins der Formel $R_6$-H, worin $R_6$ ein wie definiert, disubstituierter Aminorest ist, in Gegenwart von vorzugsweise äquivalenten Mengen eines Kondensationsmittels, wie Cyclohexylcarbodiimid, in einem inerten Lösungsmittel, wie den oben genannten, beispielsweise in Tetrahydrofuran oder Benzol oder in Gemischen davon, in eine entsprechende disubstituierte Carbamoylgruppe $-C(=O)-R_6$ umgewandelt werden. Die genannten Amine sowie auch primäre Amine der Formel $R_6$-H und auch Ammoniak lassen sich in bekannter Weise in entsprechende Amide überführen, wenn man statt der freien Säure einen Ester, wie den Niederalkylester oder ein reaktionsfähiges, funktionelles Derivat der Carbonsäure, wie deren gemischtes Anhydrid, z.B. ein Säurehalogenid oder einen aktivierten Ester, mit ihnen zur Reaktion bringt. Entsprechende Verfahrensweisen sind beispielsweise in der DE-OS 2.637.176 beschrieben worden.

<u>4. Substitution eines Wasserstoffatoms $R_4$:</u> In einer erhaltenen Verbindung der Formel I kann ein Wasserstoffatom $R_4$ in an sich bekannter Weise durch einen gegebenenfalls substituierten niederaliphatischen Rest $R_4$ (z.B. durch Alkylierung gemäss 4.1) oder einen Acylrest $R_4$ (z.B. durch Acylierung gemäss 4.2) ersetzt werden.

<u>4.1. Der Austausch des sekundären Amid-Wasserstoffatoms $R_4$</u> durch einen gegebenenfalls substituierten niederaliphatischen Rest $R_4$ kann in an sich bekannter Weise durch ein reaktives, diesen Rest $R_4$ übertragendes z.B. alkylierendes Mittel, wie ein entsprechendes Halogenid, Sulfat oder einen anderen reaktiven Ester, wie durch eine entsprechende

"Ortho"-verbindung, z.B. ein Acetal, Ketal oder Orthoester, oder durch entsprechende Oniumsalze, z.B. Trialkyloxoniumborfluorid, erfolgen, wobei gleichzeitig einer der oben genannten neutralen Protonenfänger, wie Propylen, mit Vorteil Anwendung finden kann. Die Alkylierung findet in inerten, polaren Lösungsmitteln, wie Methylenchlorid, Tetrahydrofuran oder Aceton statt. Für die N-Alkylierung kann als Ausgangsprodukt auch die entsprechende N-silylierte, wie Trimethyl-silylierte oder die N-Alkalimetall-, wie N-Lithium-Verbindung eingesetzt werden, allerdings darf im letzteren Fall kein Ueberschuss des Reagenses verwendet werden.

4.2 Der Austausch des Amidswasserstoffatoms $R_4$ durch einen Acylrest gelingt leicht mit entsprechenden, den Acylrest $R_4$ übertragenden Acylierungsmitteln, wie entsprechenden Anhydriden oder Säurehalogeniden, z.B. Säurechloriden, wenn die bei den Acylierungsverfahren c), d) und insbesondere e) genannten Bedingungen für die Acylierung von Amid-Stickstoffatomen eingehalten werden. Beispielsweise lässt sich ein H-Atom $R_4$ zuerst durch einen Silyl-, wie Trimethylsilylrest und dieser durch den gewünschten Acylrest ersetzen, wobei vorzugsweise ein weiterer Protonenfänger, wie Propylen, eingesetzt wird.

5. Sonstige Nachoperationen, Salzumwandlungen: Man kann auch weitere gegebenenfalls vorhandene freie funktionelle Gruppen, wie eine freie Hydroxygruppe im Phenylrest $R_3$ oder in einem der Reste $R_4$ gegebenenfalls vorhandene freie Amino,-, Hydroxy-, Carboxy- oder Mercaptogruppen nach an sich bekannten Verfahren, z.B. durch Acylieren, Alkylieren oder Substituieren, funktionell abwandeln. So lässt sich die p-Hydroxyphenylgruppe $R_3$ in einer Verbindung der Formel I, worin andere funktionelle Gruppen geschützt sind, mit Acetanhydrid oder Keten in die p-Acetoxyphenylgruppe oder mit

einem Carbamoylierungsmittel, wie einem reaktiven Derivat der Carbaminsäure, z.B. Carbamoylchlorid, in die p-Carbamoyloxyphenylgruppe $R_3$ umwandeln.

Salze von Verbindungen der Formel I können in an sich bekannter Weise hergestellt werden. So kann man Salze von Verbindungen der Formel I mit sauren Gruppen, z.B. durch Behandeln mit Metallverbindungen, wie Alkalimetallsalzen von geeigneten Carbonsäuren, z.B. mit Natrium-α-Aethylcapronat oder Natriumbicarbonat, oder mit Ammoniak oder einem geeigneten organischen Amin bilden, wobei man vorzugsweise stöchiometrische Mengen oder nur einen kleinen Ueberschuss des salzbildenden Mittels verwendet. Säureadditionssalze von Verbindungen der Formel I erhält man in üblicher Weise, z.B. durch Behandeln mit einer Säure oder einem geeigneten Anionenaustauschreagens. Innere Salze von Verbindungen der Formel I, welche eine freie Carboxylgruppe enthalten, können z.B. durch Neutralisieren von Salzen, wie Säureadditionssalzen, auf den isoelektrischen Punkt, z.B. mit schwachen Basen, oder durch Behandeln mit flüssigen Ionenaustauschern gebildet werden.

Salze können in üblicher Weise in die freien Verbindungen übergeführt werden, Metall- und Ammoniumsalze z.B. durch Behandeln mit geeigneten Säuren, und Säureadditionssalze z.B. durch Behandeln mit einem geeigneten basischen Mittel.

Erhaltene Gemische von Isomeren können nach an sich bekannten Methoden in die einzelnen Isomeren getrennt werden. Gemische von diastereomeren Isomeren können z.B. durch fraktioniertes Kristallisieren, Adsorptionschromatographie (Kolonnen- oder Dünnschichtchromatographie) oder andere geeignete Trennverfahren aufgetrennt werden. Erhaltene

Racemate können in üblicher Weise, gegebenenfalls nach Einführen von geeigneten salzbildenden Gruppierungen, z.B durch Bilden eines Gemisches von diastereomeren Salzen mit optisch aktiven salzbildenden Mitteln, Trennen des Gemisches in die diastereomeren Salze und Umwandlung der Salze in die freien Verbindungen oder durch fraktioniertes Kristallisieren aus optisch aktiven Lösungsmitteln, in die Antipoden getrennt werden.

Das Verfahren umfasst auch diejenigen Ausführungsformen, bei denen als Zwischenprodukte anfallende Verbindungen als Ausgangsstoffe verwendet und die restlichen Verfahrensschritte mit diesen durchgeführt werden, oder das Verfahren auf irgendeiner Stufe abgebrochen wird; ferner können Ausgangsstoffe in Form von Derivaten verwendet oder während der Reaktion (intermediär) gebildet werden.

Vorzugsweise werden solche Ausgangsstoffe verwendet und die Reaktionsbedingungen so gewählt, dass man zu den vorstehend als besonders bevorzugt aufgeführten Verbindungen gelangt.

Herstellung der Ausgangsverbindungen

Die Ausgangsverbindungen der Formel II, III, VI, VII und XII sind bekannt oder können in analoger Weise erhalten werden.

Neue Ausgangsverbindungen der Formel IV, V, VIII, IX, X und XI und ihre geschützten Derivate sowie solche erfindungsgemässen Verbindungen der Formel I, worin mindestens eine funktionelle Gruppe eine Schutzgruppe trägt und/oder in denen die 4-Carboxylgruppe durch eine (von einer physiologisch spaltbaren Schutzgruppe $R_5$ verschiedene)

Schutzgruppe geschützt vorliegt, bzw. Verfahren zu ihrer Herstellung, sind ebenfalls Gegenstand der vorliegenden Erfindung. Alle diese Verbindungen können nach an sich bekannten Verfahren hergestellt werden.

In die genannten Verbindungen können z.B. auf an sich bekannte Weise Schutzgruppen eingeführt werden. So kann in einer Verbindung der Formel I-XI eine freie Aminogruppe durch Einführen einer an sich bekannten Aminoschutzgruppe, wie einer Triarylmethylgruppe, z.B. durch Behandeln mit einem reaktionsfähigen Ester eines Triarylmethanols, wie Tritylchlorid, vorzugsweise in Gegenwart eines basischen Mittels, wie Pyridin, geschützt werden.

In einer Verbindung der Formel II oder IV kann eine Aminogruppe z.B. auch in reaktionsfähiger Form, beispielsweise durch Einführen einer Silyl- und Stannylgruppe geschützt werden. Solche Gruppen werden in an sich bekannter Weise eingeführt , z.B. durch Behandeln mit einem geeigneten Silylierungsmittel, wie mit einem Dihalogen-diniederalkylsilan, Niederalkoxy-niederalkyl-dihalogen-silan oder Triniederalkylhalogen-silan (bzw. Triniederalkylsilyl-halogenid) z.B. Dichlor-dimethylsilan, Methoxy-methyl-dichlor-silan, Trimethylsilylchlorid oder Dimethyl-tert.-butyl-silylchlorid, wobei man solche Silylhalogenid-Verbindungen vorzugsweise in Gegenwart einer Base, z.B. Pyridin, verwendet, mit einem gegebenenfalls niederalkylierten, wie N,N-Diniederalkyl, N-Niederalkyl-N-triniederalkylsilyl oder N-Niederalkyl-N-triniederalkylsilyl-N-(Tri-niederalkylsilyl)-amin (siehe z.B. britisches Patent Nr. 1.073.530), oder mit einem silylierten Carbonsäureamid oder Harnstoffe, wie einer entsprechenden Bis-triniederalkylsilyl-Verbindung, z.B. Bistrimethyl-silyl-acetamid, N-Trimethylsilyl-trifluoracetamid oder N,N'-Bis-(trimethylsilyl)-Harnstoff, ferner mit

einem geeigneten Stannylierungsmittel, wie Tri-n-butylzinnchlorid.

In einer erfindungsgemässen erhältlichen Verbindung der Formel I mit freier 4-Carboxylgruppe oder in einem Ausgangsprodukt der Formel II-XI, das eine (oder mehrere) freie Carboxylgruppe(n), z.B. in 4-Stellung des Cephem-Gerüstes (und)oder in einem der Reste $R_1$ oder $R_4$ trägt, kann eine solche in an sich bekannter Weise in eine geschützte Carboxylgruppe übergeführt werden. So erhält man Ester z.B. durch Behandeln mit einer geeigneten Diazoverbindung, wie einem Diazoniederalkan, z.B. Diazomethan oder Diazobutan, oder einem phenyl-substituierten Diazoniederalkan, z.B. Diphenyldiazomethan, wenn notwendig, in Gegenwart einer Lewissäure wie Bortrifluorid, oder auch durch Umsetzen mit einem zur Veresterung geeigneten Alkohol in Gegenwart eines Veresterungsmittels, wie eines Carbodiimids, z.B. Dicyclohexylcarbodiimid, sowie Carbonyldiimidazol. Die Esterschutzgruppe kann ferner mit einem N,N'-disubstituierten, O- bzw. S-substituierten Isoharnstoff bzw. Isothioharnstoff, worin ein O- oder S-Substituent z.B. Niederalkyl, insbesondere tert.-Butyl, Phenylniederalkyl oder Cycloalkyl, und N- bzw. N'-Substituenten z.B. Niederalkyl, insbesondere Isopropyl, Cycloalkyl oder Phenyl ist, oder nach irgendeinem anderen bekannten und geeigneten Veresterungsverfahren, wie durch Reaktion eines Salzes der Carbonsäure mit dem reaktionsfähigen Ester eines Schutzgruppenalkohols mit einer starken anorganischen Säure oder einer starken organischen Sulfonsäure eingeführt werden. Ferner können die freien Carboxylgruppen über die Säurehalogenide, wie -chloride (hergestellt z.B. durch Behandeln mit Oxalylchlorid), die aktivierten Ester (gebildet z.B. mit N-Hydroxystickstoffverbindung, wie N-Hydroxy-succinimid)

oder die gemischten Anhydride (erhalten z.B. mit Halogenameisensäureniederalkylestern, wie Chlorameisensäureäthyl- oder Chlorameisensäureisobutylester, oder mit Halogenessigsäurehalogeniden, wie Trichloressigsäurechlorid) durch Umsetzen mit Schutzgruppen-Alkoholen, vorzugsweise in Gegenwart einer Base, wie Pyridin oder eines Protonenfängers, wie Propen, in eine veresterte Carboxylgruppe übergeführt werden.

In einer erhaltenen Verbindung mit einer veresterten Gruppierung der Formel $-C(=O)-R_2$ kann diese in eine andere veresterte Carboxygruppe dieser Formel übergeführt werden. So kann man z.B. 2-Chloräthoxy- oder 2-Bromäthoxycarbonyl-Reste durch Behandeln mit einem Jodid-übertragenden Salz, wie einem Alkalijodid, z.B. Natriumjodid, in Gegenwart eines geeigneten Lösungsmittels, wie Aceton, in einen 2-Jodäthoxycarbonylrest umwandeln.

Gemischte Anhydride können hergestellt werden, indem man eine Verbindung der Formel I mit einer freien Carboxylgruppe der Formel $-C(=O)-R_2$, vorzugsweise ein Salz, insbesondere ein Alkalimetall-, z.B. Natrium-, oder Ammonium-, z.B. Triäthylammoniumsalz davon, mit einem reaktionsfähigen Derivat, wie einem Halogenid, z.B. dem Chlorid, einer Säure, z.B. einem Halogenameisensäure-niederalkylester oder einem Niederalkancarbonsäurechlorid, umsetzt.

Eine durch eine organische Silyl- oder Stannylgruppe geschützte Carboxylgruppe kann in der oben für eine entsprechend silylierte oder stannylierte Aminogruppe beschriebenen Weise gebildet werden, z.B. indem man Verbindungen der Formeln I, worin $R_2$ für Hydroxy steht, oder Ausgangsprodukte der Formel II-XI, die freie Carboxylgruppen aufweisen, oder entsprechende Salze, wie Alkalimetall-, z.B. Natriumsalze

davon, mit einem geeigneten Silylierungs- oder Stannylierungsmittel, wie einem der obgenannten Silylierungs- oder Stannylierungsmittel behandelt.

Ferner kann man andere funktionelle Gruppen in den Resten $R_1$, $R_3$ und $R_4$, wie freie Hydroxy- oder Mercaptogruppen, in üblicher Weise mit den oben genannten geeigneten Schutzgruppen, vorzugsweise intermediär, schützen. Bevorzugte Schutzgruppen sind die oben genannten Silyl-, sowie ferner entsprechende Stannylgruppen, die auf die bereits angegebene Weise eingeführt werden.

Neue Aminoverbindungen der Formel IV erhält man beispielsweise aus den entsprechenden Verbindungen der Formel II durch Acylierung mittels eines, den Rest einer α-Aminocarbonsäure der Formel

$$H_2N-\underset{\displaystyle R_3}{\underset{|}{CH}}-COOH \qquad (XIII),$$

einführenden Acylierungsmittels, wie eines der unter Verfahren a) oder b) genannten, als Acylierungsmittel geeigneten Carbonsäurederivate, z.B. des entsprechenden Säurechlorids oder eines anderen gemischten Anhydrids, oder durch direkte Umsetzung der Carbonsäure XIII, worin die Aminogruppe geschützt ist, mit einer 7β-Amino-Verbindung der Formel II in Gegenwart eines oben genannten Kondensationsmittels, z.B. Dicyclohexylcarbodiimid.

Erhaltene Verbindungen der Formel IV kann man in an sich bekannter Weise in Verbindungen der Formel VI umwandeln, worin Z eine mit Aminen unter Bildung einer Harnstoff- oder Thioharnstoffgruppierung reagierende Gruppe, wie oben definiert, beispielsweise eine Halogenformylamino-, Isocyanato- oder Isothiocyanatogruppe, ist. Diese Umwandlung geschieht vorteilhaft durch Einwirkung eines aktivierten Derivats der Kohlen- oder Thiokohlensäure, gemäss den unter Verfahren c) beschriebenen in situ-Bedingungen. Vorzugsweise werden Phosgen, Thiophosgen oder ein übliches Phosgen-bildendes Mittel in Gegenwart von Protonenfängern auf IV einwirken gelassen.

Das den Acylrest einer Kohlen- oder Thiokohlensäure der Formel V einführende Acylierungsmittel (vorstehend auch "reaktives Derivat der Carbaminsäure V" genannt) kann durch Umsetzung einer Verbindung der Formel VII, z.B. mit Phosgen, Chlorameisensäuretrichlormethylester, Thiophosgen oder einem anderen, oben genannten aktiven Kohlen- oder Thiokohlensäurederivat, erhalten werden. Vorzugsweise verwendet man dabei das Säurechlorid der Formel

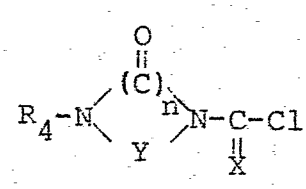 (XV),

worin X O oder S bedeutet und $R_4$ Acyl oder ein gegebenenfalls substituierter niederaliphatischer Rest ist. Verbindungen der Formel XV, worin n=1 ist (Aethylenharnstoff-Typ), sind z.B. nach den in den DE-OS 1.057.126 und 2.720.579 angegebenen Verfahren zugänglich. Die entsprechenden Verbindungen, worin n=2 ist (Dioxopiperazin-Typ), erhält man beispielsweise gemäss den in den DE-OS 2.519.400, 2.657.559 oder 2.702.552 sowie von J.L. Riebsomer, J.Org. Chem. 15, 68-73 (1950), angegebenen Verfahren. So kann man entsprechend substituierte Aethylendiamine mit einem reaktionsfähigen Derivat der Kohlensäure oder Oxalsäure ringschliessen. Als reaktionsfähige Derivate dieser Säuren verwendet man beispielsweise die entsprechenden Säurechloride, wie Phosgen oder Oxalylchlorid (z.B. in Gegenwart eines Protonenfängers) oder Oxalsäure-dimethylester, in welchem Falle freigesetztes Methanol laufend aus dem Reaktionsgemisch abdestilliert wird. Die Reaktionstemperatur hängt u.a. von der Empfindlichkeit des Substituenten $R_4$ ab und liegt im allgemeinen zwischen 0 und 100°C. Bei den vorstehenden Kondensationsreaktionen können saure Katalysatoren, wie Protonensäuren, saure Ionenaustauscher oder auch Lewissäuren zur Reaktionsbeschleunigung dienen. Vorzugsweise werden dazu Schwefelsäure, p-Toluolsulfonsäure oder saure Ionenaustauscher verwendet. Als Verdünnungsmittel eignen sich polare, inerte Lösungsmittel wie Niederalkanole, Acetonitril, Dimethylformamid, Dioxan, Tetrahydrofuran, Pyridin, Benzol, Toluol oder Essigsäure.

Die Verbindung VIII kann in analoger Weise durch Einwirken eines Diamins der Formel

$$R_4 - \underset{\displaystyle H}{\underset{|}{N}} - Y - NH_2 \qquad \text{(XIV)},$$

worin Y die unter Formel I genannte Bedeutung hat, und $R_4$ vorzugsweise Wasserstoff oder eine die Reaktionsfähigkeit der Aminogruppe mindernde Gruppe $R_4$, z.B. eine Acylgruppe, ist, beispielsweise eines gegebenenfalls durch den Rest $R_4$ monosubstituierten oder geschützten, z.B. silylierten, Aethylendiamins, z.B. Aethylendiamin, N-Niederalkyl-, z.B. N-Aethyl- oder N-Niederalkanoyl, z.B. N-Acetyläthylendiamins, auf eine vorzugsweise weniger als 1-molare Menge der Verbindung VI erhalten werden.

Ausgangsverbindungen der Formel IX mit geschützten funktionellen Gruppen können auf an sich bekannte Weise hergestellt werden, z.B. analog der DE-OS 2.506.330 oder

den belgischen Patentschriften 838.656 oder 844.344. Als Startmaterial verwendet man, beispielsweise aus der DE-OS 2.506.330 bekannte oder in dazu analoger Weise erhältliche Penicillansäure-Sulfoxide der Formel

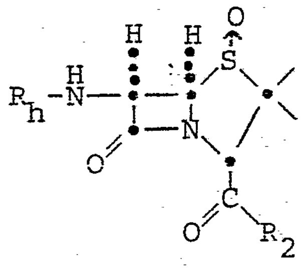

(XVI),

worin $R_h$ Wasserstoff oder einer der in Verbindungen der Formel I, IV, VI, VIII oder X vorkommenden, an die 7β-Aminogruppe gebundenen Acylreste, wobei alle funktionellen Gruppen vorzugsweise geschützt sind, bedeutet. Diese an sich bekannten oder in dazu analoger Weise erhältlichen Sulfoxide der Formel XVI werden (z.B. gemäss DE-OS 2.506.330) vorzugsweise zunächst mit Mercaptobenzthiazol und dann mit einer Sulfinsäure $HSO_2-R_9$, einem Sulfonylcyanid der Formel $N{\equiv}C-SO_2-R_9$ oder einer Thiosulfonsäure der Formel $HS-SO_2-R_9$, vorzugsweise in einem inerten Verdünnungsmittel, wie Methylenchlorid, einem Alkohol, wie Methanol, oder einem Keton, wie Aceton, einem Aether, wie Glykoldimethyläther, Methyl-tert.-butyläther, Dioxan oder Tetrahydrofuran, oder einem polaren, aprotischen Lösungsmittel, wie Dimethylformamid, bei oder wenig oberhalb Raumtemperatur umgesetzt. Dabei können Katalysatoren, wie Halogenionenliefernde Verbindungen, z.B. quaternäre, insbesondere phasentransferierende Ammoniumverbindungen, wie Tetrabutylammoniumchlorid oder Benzyltriäthylammoniumchlorid, Verwendung finden. Als Zwischenprodukte entstehen dabei Exomethylenverbindungen beispielsweise der Formel

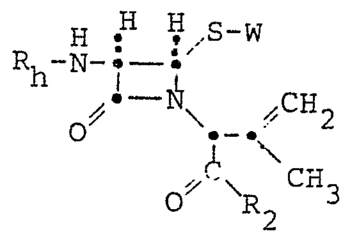

(XVII),

worin W die Gruppen - $SO_2R_9$ oder $-S-SO_2-R_9$ umfasst.
Auch auf dieser Stufe kann der Rest $R_h$ gegen einen anderen Rest $R_h$ ausgetauscht, z.B. eine 7β-Aminogruppe acyliert werden. Die Exomethylenverbindungen XVII lassen sich unter oxidativem Abbau der Exomethylengruppe ozonisieren. Die oxydative Abspaltung der Methylengruppe in Verbindungen der Formel XVII mit Ozon verläuft unter Bildung einer nicht-isolierten Ozon-Zwischenverbindung. Man verwendet Ozon üblicherweise in Anwesenheit eines Lösungsmittels, wie eines Alkohols, z.B. eines Niederalkanols, wie Methanol oder Aethanol, eines Ketons, z.B. eines Niederalkanons, wie Aceton, eines gegebenenfalls halogenierten aliphatischen, cycloaliphatischen oder aromatischen Kohlenwasserstoffs, z.B. eines Halogenniederalkans, wie Methylenchlorid oder Tetrachlorkohlenstoff, oder eines Lösungsmittelgemisches, inkl. eines wässrigen Gemisches, sowie unter Kühlen oder leichtem Erwärmen, z.B. bei Temperaturen von etwa -90° bis etwa +40°C. Das gebildete Ozonid kann, vorzugsweise ohne vorherige Isolierung, reduktiv zu einer Verbindung der Formel

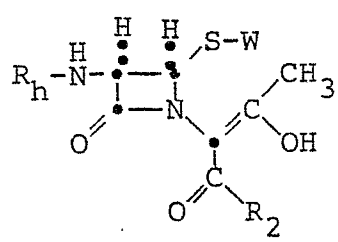

(XVIII),

gespalten werden. Als Reduktionsmittel verwendet man beispielsweise katalytisch aktivierten Wasserstoff, z.B. Wasserstoff in Gegenwart eines Schwermetall-Hydrierkatalysators, wie Raney-Nickel oder feinverteiltes Palladium, vorzugsweise auf einem geeigneten Trägermaterial, wie Calciumcarbonat oder Kohle, oder chemische Reduktionsmittel, wie reduzierende Schwermetalle inkl. Schwermetalllegierungen oder -amalgame, z.B. Zink, in Gegenwart eines Wasserstoffdonators, wie einer Säure, z.B. Essigsäure, oder eines Alkohols, z.B. Niederalkanols. Ferner eignen sich hierfür reduzierende anorganische Salze, wie Alkalimetalljodide, z.B. Natriumjodid, in Gegenwart eines Wasserstoffdonators, wie einer Säure, z.B. Essigsäure, oder reduzierende Sulfidverbindung, wie ein Diniederalkylsulfid, eine reduzierende organische Phosphorverbindung, wie ein Phosphin, das gegebenenfalls substituierte aliphatische oder aromatische Kohlenwasserstoffreste als Substituenten enthalten kann, wie Triniederalkyl-phosphin, z.B. Tri-n-butyl-phosphin, oder Triarylphosphin, z.B. Triphenylphosphin, ferner Phosphite, welche gegebenenfalls substituierte aliphatische Kohlenwasserstoffereste als Substituenten enthalten, wie Triniederalkylphosphite, üblicherweise in der Form von entsprechenden Alkoholadduktverbindungen, wie Trimethylphosphit, oder Phosphorigsäure-triamide, welche gegebenenfalls substituierte aliphatische Kohlenwasserstoffreste als Substituenten enthalten, wie Hexaniederalkyl-phosphorigsäuretriamide, z.B. Hexamethylphosphorigsäuretriamid, letzteres vorzugsweise in der Form eines Methanoladdukts, oder Tetracyanäthylen. Die Spaltung des üblicherweise nicht isolierten Ozonids erfolgt normalerweise unter Bedingungen, die man zu seiner Herstellung anwendet, d.h. in Gegenwart eines geeigneten Lösungsmittels oder Lösungsmittelgemisches, sowie unter Kühlen oder leichtem Erwärmen.

Enolverbindungen der Formel XVIII können auch in der tautomeren Ketoform vorliegen.

In einer erhaltenen Verbindung der Formel XVIII kann eine Gruppe $R_h$ oder $R_2$ in eine andere Gruppe $R_h$ oder $R_2$ übergeführt werden, wobei z.B. analoge Reaktionen angewendet werden können, wie sie oben für die Umwandlung dieser Gruppen bei Verbindungen der Formel I beschrieben werden.

In der nächsten Stufe wird eine erhaltene Enolverbindung der Formel XVIII durch Verätherung in eine Verbindung der Formel IX übergeführt, worin $R_1$ eine verätherte Hydroxygruppe ist. Zur Herstellung von solchen Niederalkyl- und gegebenenfalls substituierten α-Phenylniederalkyl-enoläthern der Formel IX verwendet man als Verätherungsreagens die unter Verfahren i) beschriebenen Verätherungsmittel, beispielsweise eine entsprechende Diazoverbindung, z.B. ein gegebenenfalls α-phenylsubstituiertes Diazoniederalkan, wie Diazomethan oder Diphenyldiazomethan. Diese Reagentien werden in Gegenwart eines geeigneten inerten Lösungsmittels, wie eines niederaliphatischen, cycloaliphatischen oder aromatischen Kohlenwasserstoffs, wie Hexan, Cyclohexan, Benzol oder Toluol, eines halogenierten niederaliphatischen Kohlenwasserstoffs, z.B. Methylenchlorid, eines Niederalkanols, z.B. Methanol oder eines Aethers, z.B. Methyl-tert.-butyläther oder Tetrahydrofuran, je nach Diazoregenz unter Kühlen, bei Raumtemperatur oder leichtem Erwärmen, angewendet.

Ferner kann man Verbindungen der Formel IX, worin $R_1$ z.B. Niederalkoxy oder gegebenenfalls substituiertes α-Phenylniederalkoxy ist, durch Behandeln einer Enolverbindung der Formel XVIII mit einem reaktionsfähigen Ester eines entsprechenden Alkohols der Formel $R_1$-H bilden. Ge-

eignete Ester sind in erster Linie solche mit starken anorganischen oder organischen Säuren, wie Mineralsäuren, z.B. Halogenwasserstoffsäuren, wie Chlorwasserstoffsäure, ferner Schwefelsäure oder Halogen-schwefelsäuren, z.B. Fluorschwefelsäure, oder starken organischen Sulfonsäuren, wie gegebenenfalls, z.B. durch Halogen, wie Fluor, substituierten Niederalkansulfonsäuren, oder aromatischen Sulfonsäuren, wie z.B. gegebenenfalls substituierten Benzolsulfonsäuren, z.B. Methansulfon-, Trifluormethansulfon- oder p-Toluolsulfonsäure. Dabei wendet man vorzugsweise geeignete Kondensationsmittel, wie Alkalimetallcarbonate oder -hydrogencarbonate (üblicherweise zusammen mit einem Sulfat), oder organischen Basen, wie, üblicherweise sterisch gehinderte, Triniederalkylamine, z.B. N,N-Diisopropyl-N-äthylamin (vorzugsweise zusammen mit Niederalkyl-halogensulfaten oder gegebenenfalls halogensubstituierten Methansulfonsäureniederalkylestern) an, wobei unter Kühlen, bei Raumtemperatur oder unter Erwärmen, z.B. bei Temperaturen von etwa -20° bis etwa +50°C gearbeitet wird. Durch Phasentransfer-Katalyse (vgl. unter Verfahren i) kann die Verätherungsreaktion wesentlich beschleunigt werden.

2-Cephem-Ausgangsverbindungen der Formel X sind neu. Sie können, analog den Acylierungs- und Kondensationsverfahren a) bis e), ausgehend von den bekannten oder auf an sich bekannte Weise herstellbaren 2-Cephemverbindungen der Formel

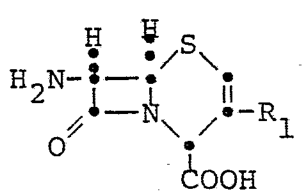

(XIX),

durch entsprechende Acylierung der 7-Aminogruppe hergestellt werden. Ausserdem können sie als Nebenprodukte bei anderen erfindungsgemässen Verfahren, z.B. bei den Verfahren a) bis

f) entstehen, insbesondere wenn unter basischen Bedingungen gearbeitet wird.

Ausgangsverbindungen der Formel XI sind ebenfalls neu. Ausgangsverbindungen der Formel XI, worin $R_1^a$ eine freie oder veresterte, z.B. sulfonierte Hydroxygruppe ist, können z.B. aus einer Verbindung der Formel XX

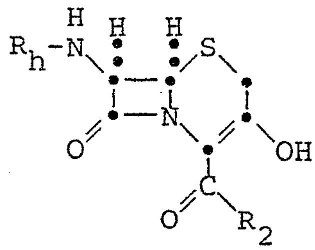

(XX),

worin $R_h$ die unter Formel XVI angegebene Bedeutung hat, durch Umwandeln des Restes $R_h$ in einen unter Formel I definierten 7β-N-Acylrest erhalten werden.

Ausgangsprodukte der Formel XX können beispielsweise hergestellt werden, indem man in einer an sich bekannten oder in analoger Weise erhältlichen Cephemverbindung der Formel

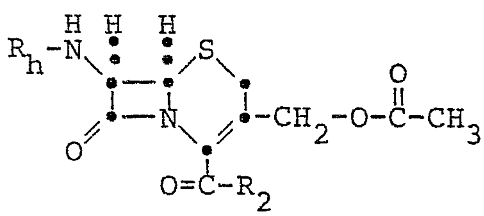

(XXI),

worin $R_h$ die unter Formel XVI angegebene Bedeutung hat und vorzugsweise für eine Aminoschutzgruppe steht und worin $R_2$ Hydroxy bedeutet, das auch geschützt sein kann, die Acetoxymethylgruppe hydrolysiert, in der erhaltenen 3-Hydroxymethyl-cephem-Verbindung gegebenenfalls eine freie Carboxylgruppe der Formel $-C(=O)-R_2$ in geeigneter Weise z.B. durch Behandeln mit einer Diazoverbindung, wie Diphenyldiazomethan,

durch Veresterung schützt, die Hydroxymethylgruppe, z.B. durch Behandeln mit einem Halogenierungsmittel, wie Chlorierungsmittel, z.B. Thionylchlorid, in eine Halogenmethyl-, z.B. Chlormethylgruppe und die Chlormethylverbindung unter Abspaltung von Halogenwasserstoff, z.B. HCl, in die Exomethylen-cepham-Verbindung der Formel

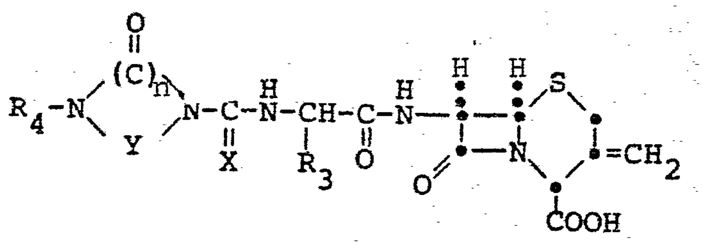

(XXII),

worin die 4-Carboxylgruppe und übrige gegebenenfalls vorhandene funktionelle Gruppen geschützt sind, und $R_3$, $R_4$, X und Y die unter Formel I angegebene Bedeutung haben, umwandelt.

Die Hydrolyse der Verbindung XXI kann z.B. in einem schwach-basischem Medium, wie mit einer wässrigen Natriumhydroxydlösung bei pH 9-10, oder durch Behandeln mit einer geeigneten Esterase, wie einem entsprechenden Enzym aus Rhizobium tritolii, Rhizobium lupinii, Rhizobium japonicum oder Bacillus subtilis erfolgen. Die Abspaltung von Halogenwasserstoff kann entweder direkt, z.B. durch Behandeln mit einer zu Reduktionszwecken geeigneten Chrom-II-Verbindung, wie einem entsprechenden Salz, z.B. Chrom-II-chlorid, in einem inerten Lösungsmittel, wie Dimethylsulfoxid, oder indirekt, z.B. unter katalytischer Mitwirkung eines Metalljodids, wie Natriumjodid, in einem geeigneten Lösungsmittel, wie Aceton, durch Behandeln mit einem geeigneten, z.B. metallischen Reduktionsmittel, wie Zink, in Gegenwart einer schwachen Säure, wie einer niederaliphatischen Carbonsäure, z.B. Essig- oder Ameisensäure, vorgenommen werden.

Eine Methylenverbindung der Formel

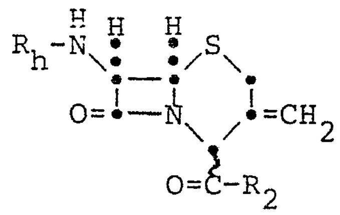

(XXIII)

ist aus Verbindungen der Formel XXI auch durch elektrochemische Reduktion oder durch Reduktion mit Aluminiumamalgam erhältlich. Entsprechende Verfahrensweisen sind beispielsweise in der DE-OS 2.331.133 beschrieben worden.

Eine Verbindung der Formel XXII mit geschützten funktionellen Gruppen kann, sofern sie keine weiteren isolierten, ozonisierbaren Doppelbindungen enthält, auch direkt unter oxidativer Abspaltung der Methylengruppe ozonisiert und so in eine entsprechende 3-Oxo-cepham- (bzw. eine dazu isomere 3-Hydroxy-3-cephem-Verbindung) der Formel XX umgewandelt werden.

Ein als Zwischenprodukt gebildetes Ozonid wird reduktiv gespalten, wobei man die bei der Reduktion des aus der Exomethylen-Verbindung der Formel XVII erhaltenen Ozonids verwendbaren, oben genannten Reduktions- und Lösungsmittel und die gleichen Reaktionsbedingungen anwenden kann.

Je nach Durchführung der Oxydationsreaktion erhält man eine Verbindung der Formel XX oder das entsprechende 1-Oxid oder ein Gemisch der beiden Verbindungen. Ein solches Gemisch kann in die Verbindung der Formel XX und das entsprechende 1-Oxyd aufgetrennt oder als solches verwendet werden, oder man kann es nach der unter Verfahren g) aufgeführten Methode reduktiv, z.B. mit $PCl_3$, in eine Verbindung der Formel XX überführen. Ein Gemisch einer Verbindung der Formel XX mit dem entsprechenden 1-Oxyd kann in üblicher Weise, z.B. durch fraktioniertes Kristallisieren

oder durch Chromatographieren (z.B. Säulenchromatographie, Dünnschichtchromatographie), in die Einzelkomponenten aufgetrennt werden.

Andere Ausgangsverbindungen der Formel XI, worin $R_1^a$ eine andere veresterte Hydroxygruppe oder eine der oben definierten Aminogruppen ist, können durch Veresterung einer 3-Hydroxy-cephem-Verbindung bzw. durch Umsetzung einer 3-Hydroxy-cephem-Verbindung mit einem Salz eines Amins der Formel $R_1^a$-H oder, ausgehend von Verbindungen der Formeln II, IV, VI, VIII oder IX, in denen $R_1$ die Bedeutung einer entsprechenden veresterten Hydroxy- oder Aminogruppe hat, gegebenenfalls unter Schutz nicht an den Reaktionen teilnehmender Gruppen, analog den Verfahren a), b), c), d), e) und f), erhalten werden.

Die Veresterung der freien Hydroxygruppe $R_1^a$ wird mit einem entsprechenden organischen Acylierungsmittel durchgeführt. Als solche verwendet man die entsprechende organische Carbonsäure oder ein reaktionsfähiges Säurederivat davon, wie ein Halogenid, z.B. Fluorid oder Chlorid, ferner ein Pseudohalogenid, wie eine der Carbonsäure entsprechende Cyancarbonylverbindung, oder ein Anhydrid (worunter auch ein inneres Anhydrid dieser Säure, z.B. im Falle der Carbamin- oder Thiocarbaminsäure ein Isocyanat, oder ein gemischtes Anhydrid, wie ein solches, das sich z.B. mit einem Halogenameisensäureniederalkyl-, wie Chlorameisensäureäthylester oder -isobutylester, oder mit Trichloressigsäurechlorid bilden lässt, zu verstehen ist), oder einen aktivierten Ester, wobei man, wenn notwendig, in Gegenwart von geeigneten Kondensationsmitteln, bei Verwendung von Säuren z.B. von Carbodiimidverbindungen, wie Dicyclohexylcarbodiimid, oder Carbonylverbindungen, wie Carbonyldiimidazol, bei Verwendung von reaktionsfähigen Säurederivaten z.B. von basischen

Mitteln, wie Triniederalkylaminen, z.B. Triäthylamin, oder heterocyclischen Basen, z.B. Pyridin, arbeitet. Die Acylierungsreaktion kann in Abwesenheit oder in Gegenwart eines Lösungsmittels oder Lösungsmittelgemisches, unter Kühlen, bei Raumtemperatur oder unter Erwärmen, und, wenn notwendig, in einem geschlossenen Gefäss und/oder in einer Inertgas-, z.B. Stickstoffatmosphäre durchgeführt werden. Geeignete Lösungsmittel sind z.B. gegebenenfalls substituierte, insbesondere gegebenenfalls chlorierte, niederaliphatische oder aromatische Kohlenwasserstoffe, wie Methylenchlorid oder Toluol. Durch organische Sulfonsäuren, z.B. Niederalkansulfonsäuren, wie Methansulfonsäure, oder aromatische Sulfonsäuren, wie p-Toluolsulfonsäure, veresterte Hydroxygruppen kann man vorzugsweise durch Behandeln mit einem reaktionsfähigen Sulfonsäurederivat, wie einem entsprechenden Halogenid, z.B. Chlorid, wenn notwendig, in Gegenwart eines Protonenfängers oder basischen Mittels, z.B. einer anorganischen oder organischen Base, z.B. in analoger Weise wie die Ester mit Carbonsäuren, bilden.

Die Einführung einer gegebenenfalls substituierten Aminogruppe $R_1^a$ in eine Verbindung der Formel XI, worin $R_1^a$ eine gegebenenfalls veresterte Hydroxygruppe, insbesondere freies Hydroxy bedeutet, und worin die 4-Carboxylgruppe und weitere gegebenenfalls vorhandene funktionelle Gruppen geschützt sind, kann in Gegenwart eines tertiären aromatischen Amins mit einem Salz eines Amins der Formel $R_1^a$-H, worin $R_1^a$ die Bedeutung des oben definierten Aminorestes $R_1^a$ hat, erfolgen und die erhaltene 3-Aminocephemverbindung in die freie Verbindung überführt werden.

Geeignete Salze eines Amins der Formel $R_1^a$-H sind beispielsweise von organischen Säuren, wie solche von gegebenenfalls substituierten niederaliphatischen oder aromatischen

Carbon- oder Sulfonsäuren, wie Niederalkancarbonsäuren, z.B. Essigsäure, Niederalkansulfonsäuren, wie Methansulfonsäure, Aryl-, wie p-Toluolsulfonsäure, oder von anorganischen Säuren, insbesondere Mineralsäuren, wie Schwefel oder Phosphor enthaltenden Sauerstoffsäuren, z.B. Schwefel- oder Phosphorsäure, oder insbesondere von Halogenwasserstoffsäuren, wie Chlorwasserstoffsäure, abgeleitet. Bevorzugt geeignete Säureadditionssalze von Aminen der Formel $R_1^a$-H sind deren Hydrochloride.

Geeignete tertiäre aromatische Amine sind beispielsweise entsprechende heteroaromatische Amine, wie gegebenenfalls durch Niederalkyl, z.B. Methyl, Halogen, z.B. Chlor, Niederalkoxy, z.B. Methoxy, oder Phenyl, substituiertes Pyridin, beispielsweise Picolin, Lutidin, Collidin, Chlorpyridin, Methoxypyridin oder Phenylpyridin, oder bevorzugt unsubstituiertes Pyridin.

Die Säureadditionssalze von Aminen der Formel $R_1^a$-H werden in mindestens äquimolaren Mengen, bevorzugt im Ueberschuss, d.h. in etwa 2 bis 10-fach molaren Mengen, eingesetzt. Auch das bei der Reaktion anwesende tertiäre aromatische Amin wird in mindestens äquimolaren Mengen, bevorzugt aber im Ueberschuss eingesetzt, wobei die Reaktion in einem geeigneten organischen Lösungsmittel z.B. in einem Niederalkanol, wie Methanol, ätherischen Lösungsmittel, wie Dioxan, Tetrahydrofuran, niederen Carbonsäureamid, wie Dimethylformamid, Niederalkansäurenitril, wie Acetonitril oder niederen Sulfoxid, wie Dimethylsulfoxid, oder in einem der genannten tertiären aromatischen Amine, wie Pyridin, oder Mischungen davon, bei Raumtemperatur oder erhöhter Temperatur, z.B. zwischen etwa 20 und etwa 100°C, bevorzugt zwischen etwa 30 und 60°C, durchgeführt wird.

Beispielsweise erhält man Verbindungen der Formel XI, worin $R_1^a$ z.B. die primäre Aminogruppe ist, indem man eine 3-Hydroxyverbindung der Formel XI mit Ammoniumchlorid in Gegenwart von Pyridin in einem Niederalkanol, wie Aethanol, auf etwa 50°C erwärmt. Entsprechende Verfahrensweisen sind z.B. in der DE-OS 2.606.192 beschrieben worden.

Die gegebenenfalls als Zwischenprodukte bei der Herstellung von Verbindungen der Formel XX auftretenden Cephamverbindungen können auch analog den Acylierungsverfahren a) bis e), ausgehend von den bekannten oder auf analoge Weise herstellbaren Cephamverbindungen der Formel

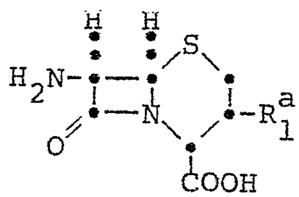

(XXIV),

erhalten werden. Entsprechende Verfahrensweisen sind beispielsweise in der DE-OS 2.537.974 beschrieben worden.

Die vorliegende Erfindung betrifft ebenfalls pharmazeutische Präparate, welche Verbindungen der Formel I oder pharmazeutisch verwendbare Salze von solchen Verbindungen mit salzbildenden Gruppen enthalten.

Die pharmakologisch verwendbaren Verbindungen der vorliegenden Erfindung können z.B. zur Herstellung von antibakteriell wirksamen Präparaten verwendet werden, welche eine wirksame Menge der Aktivsubstanz zusammen oder im Gemisch mit anorganischen oder organischen, festen oder flüssigen, pharmazeutisch verwendbaren Trägerstoffen enthalten, die sich vorzugsweise zur parenteralen Verabreichung eignen.

Vorzugsweise verwendet man die pharmakologisch wirksamen Verbindungen der vorliegenden Erfindung in Form von injizierbaren, z.B. subcutan verabreichbaren Präparaten oder von Infusionslösungen. Solche Lösungen sind vorzugsweise isotonische wässrige Lösungen oder Suspensionen, wobei diese z.B. aus lyophilisierten Präparaten, welche die Wirksubstanz allein oder zusammen mit einem Trägermaterial, z.B. Mannit, enthalten, vor Gebrauch hergestellt werden können. Die pharmazeutischen Präparate können sterilisiert sein und/oder Hilfsstoffe, z.B. Konservier-, Stabilisier-, Netz-, und/oder Emulgiermittel, Löslichkeitsvermittler, Salze zur Regulierung des osmotischen Druckes und/oder Puffer enthalten. Die vorliegenden pharmazeutischen Präparate, die, wenn erwünscht, weitere pharmakologisch wertvolle Stoffe enthalten können, werden in an sich bekannter Weise, z.B. mittels konventioneller Lösungs- oder Lyophilisierungsverfahren, hergestellt und enthalten von etwa 0,1 % bis 100 %, insbesondere von etwa 1 % bis etwa 50 %, Lyophilisate bis zu 100 % des Aktivstoffes. Je nach Art der Infektion und Zustand des infizierten Organismus verwendet man tägliche Dosen von etwa 0,3 bis etwa 5,0 g s.c. zur Behandlung von Warmblütern von etwa 75 kg Gewicht.

Die antibiotisch wirksamen Verbindungen der Formel I, insbesondere solche, in denen $-C(=O)-R_2$ eine physiologisch spaltbare Gruppe der Formel $-C(=O)-OR_5$ ist, können auch oral verabreicht werden, z.B. in Form üblicher oral applizierbarer pharmazeutischer Präparate, wie Tabletten, Kapseln oder Lösungen, die eine wirksame Menge der Verbindung und gegebenenfalls übliche pharmazeutisch akzeptable Zusatz- oder Trägerstoffe enthalten.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister unter Verwendung z.B. von Mais-, Weizen-, Reis- oder Kartoffelstärke, Gelatine, Traganth, Methylcellulose, Hydroxypropyl-methylcellulose, Natriumcarboxymethylcellulose und/oder Polyvinylpyrrolidon, und/oder wenn erwünscht, Sprengmittel, wie die obgenannten Stärken, ferner Carboxymethylstärke, quervernetztes Polyvinylpyrrolidon, Agar, Alginsäure oder Salzen davon, wie Natriumalginat. Hilfsmittel sind in erster Linie Fliessregulier- und Schmiermittel, z.B. Kieselsäure, Talk, Stearinsäure oder Salze davon, wie Magnesium- oder Calciumstearat, und/oder Polyäthylenglykol. Dragée-Kerne werden mit geeigneten, gegebenenfalls Magensaft-resistenten Ueberzügen versehen, wobei man u.a. konzentrierte Zuckerlösungen, welche gegebenenfalls arabischen Gummit, Talk, Polyvinylpyrrolidon, Polyäthylenglycol und/oder Titandioxid enthalten, Lacklösungen in geeigneten organischen Lösungsmitteln oder Lösungsmittelgemischen oder, zur Herstellung von Magensaft-resistenten Ueberzügen, Lösungen von geeigneten Cellulosepräparaten, wie Acetylcellulosephthalat oder Hydroxypropylmethylcellulosephthalat, verwendet. Den Tabletten oder Dragée-Ueberzügen können Farbstoffe oder Pigmente, z.B. zur Identifizierung oder zur Kennzeichnung verschiedener Wirkstoffen, beigefügt werden.

Weitere, oral anwendbare pharmazeutische Präparate sind Steckkapseln aus Gelatine, sowie weiche, geschlossene Kapseln aus Gelatine und einem Weichmacher, wie Glycerin oder Sorbitol. Die Steckkapseln können den Wirkstoff in Form eines Granulats, z.B. im Gemisch mit Füllstoffen,wie Lactose, Bindemitteln, wie Stärken, und/oder Gleitmitteln,

wie Talk oder Magnesiumstearat, und gegebenenfalls von Stabilisatoren, enthalten. In weichen Kapseln ist der Wirkstoff vorzugsweise in geeigneten Flüssigkeiten, wie fetten Oelen, Paraffinöl oder flüssigen Polyäthylenglykolen, gelöst oder suspendiert, wobei ebenfalls Stabilisatoren zugefügt sein können.

Zur parenteralen Verabreichung eignen sich in erster Linie wässrige Lösungen eines Wirkstoffs in wasserlöslicher Form, z.B. eines wasserlöslichen Salzes, ferner Suspensionen des Wirkstoffs, wie entsprechende ölige Injektionssuspensionen, wobei man geeignete lipophile Lösungsmittel oder Vehikel, wie fette Oele, z.B. Sesamöl, oder synthetische Fettsäureester, z.B. Aethyloleat oder Triglyceride, verwendet, oder wässrige Injektionssuspensionen, welche viskositätserhöhende Stoffe, z.B. Natrium-Carboxymethylcellulose, Sorbit und/oder Dextran und gegebenenfalls auch Stabilisatoren enthalten.

Die nachfolgenden Beispiele illustrieren die oben beschriebene Erfindung; sie sollen jedoch diese in ihrem Umfang in keiner Weise einschränken. Temperaturen werden in Celciusgraden angegeben.

Beispiel 1. 7β-[D-α-(4-Aethyl-2.3-dioxo-1-piperazinocarbonylamino)-phenylacetamido]-3-methoxy-3-cephem-4-carbonsäure.

a.) Eine Suspension von 3,18 g (22,4 mMol) 1-Aethyl-2,3-dioxo-piperazin in 32 ml Acetonitril wird zusammen mit 5,9 ml (24 mMol) O.N-Bis(trimethylsilyl)-acetamid 1 Stunde unter Rückfluss gekocht. Das Gemisch wird auf 20°C gekühlt und zu 17,2 ml einer 20 %igen Lösung von Phosgen (35 mMol) in Toluol getropft. Nach 2 Stunden Rühren bei 20°C dampft man die klare Lösung im Vakuum ein und löst den Rückstand in 25 ml Methylenchlorid (=Lösung A).

b.) Eine Suspension von 7,48 g (18,7 mMol) 7β-[D-α-(4-"Aethyl-phenylacetamido -3-methoxy-3-cephem-4-carbonsäure (Dihydrat) in 50 ml Methylenchlorid wird bei -12°C mit 6,8 ml (56,1 mMol) Dimethyldichlorsilan und anschliessend langsam bei 0°C, unter Abführung der Reaktionswäme, mit 14,2 ml (112 mMol) N,N-Dimethylanilin versetzt und noch 1 Stunde bei 0°C gerührt. Nach Zugabe von 2 ml N,O-Bis-(trimethyl-silyl)-acetamid wird eine fast klare Lösung erhalten (=Lösung B).

c.) Zur Lösung B werden bei -15° bis -20°C 14,5 ml (131 mMol) N-Methylmorpholin und anschliessend die Lösung A getropft. Man rührt 2 Std. bei 20°C. Das rötliche Gemisch wird auf 400 ml 0,33-molaren Phosphat-Puffer von pH 7 gerührt und im Vakuum vom Methylenchlorid befreit. Die wässrige Phase wird 3 mal mit je 200 ml Essigester gewaschen, mit konzentrierter Salzsäure auf pH 2,0 angesäuert, mit Kochsalz gesättigt und bei 0°C 3 mal mit je 300 ml Essigester extrahiert. Die über Natriumsulfat getrockneten Extrakte geben beim Eindampfen 4,01 g rohe 7β-[D-α-(4-Aethyl 2,3-dioxo-1-piperazinocarbonylamino)-phenylacetamido]-3-methoxy-3-cephem-4-carbonsäure.

Im Dünnschichtchromatogramm an Merck Kieselgel 60 im Laufmittel n-Butanol-Eisessig-Wasser (67:10:23) wird, nach Entwicklung mit Joddampf, $R_f$ 0,19 und an einem durch Silylieren hydrophobierten Kieselgel (Macherey-Nagel SIL G-25 HR) im Laufmittel 3%ige wässrige Kochsalzlösung: Aceton (50:1) $R_f$ 0,40 gefunden; IR-Spektrum (in Nujol): Banden bei 3.03, 5.65, 5.80, 5.95, 6.07, 6.24, 6.61, 7.77, 8.45 μm u.a.

Beispiel 2. 7β-[D-α-(4-Aethyl-2.3-dioxo-1-piperazinocarbonylamino)-phenylacetamido-3-cephem-4-carbonsäure-(Natriumsalz)

Eine Suspension von 4.35 g (13,1 mMol) 7β-[D-α-Aminophenylacetamido]-3-cephem-4-carbonsäure in 40 ml absolutem Methylenchlorid wird bei 20° mit 6,4 ml (26.2 mMol) N,O-Bis-(trimethylsilyl)-acetamid versetzt und 1 Stunde bei 20°C gerührt. Bei -10° werden 1,5 ml (13,1 mMol) 2,6-Lutidin und 7,6 ml einer Lösung von 17,7 mMol 4-Aethyl-2,3-dioxo-1-piperazinocarbonylchlorid (s. Beispiel 1a) in Methylenchlorid zugetropft. Man rührt 1 Stunde bei 0° und 1/2 Stunde bei 20°C. Der Ansatz wird in eine Mischung von 500 ml Essigester und 500 ml 0,3 molarem Phosphat-Puffer vom pH 7 eingerührt. Man trennt die Phasen, verwirft die organische Phase und überschichtet die wässrige Phase mit 1 Liter Essigester. Nach Ansäuern mit konzentrierter Salzsäure auf pH 2 gibt man Eis zu, trennt die Phasen und extrahiert die wässrige Phase nochmals mit 500 ml Essigester. Die vereinigten organischen Phasen werden über Natriumsulfat getrocknet und im Vakuum eingedampft. Eine Lösung des Rückstandes (5,8 g) in 50 ml Essigester-Methanol (3:1) wird mit 15 ml einer Lösung, die 15 mMol α-Aethylhexansäure-Natriumsalz in Essigester enthält, versetzt und das kristallin ausfallende 7β-[D-α-(4-Aethyl-2,3-dioxo-1-piperazinocarbonylamino)-phenylacetamido]-3-cephem-4-carbonsäure-Natriumsalz abgesaugt. Im Dünnschichtchromatogramm an Merck Kieselgel 60 im Laufmittel n-Butanol-Eisessig-Wasser (67:10:23) wird, nach Entwicklung mit Joddampf, $R_f$ 0,22 und an einem durch Silylieren hydrophobierten Kieselgel (Macherey-Nagel SIL G-25 HR) im Laufmittel 3%ige wässrige Kochsalzlösung: Aceton (50:1) $R_f$ 0,44 gefunden; NMR-Spektrum in deuteriertem Dimethylsulfoxid (100 MHz); Banden (ppm) bei 1,10 (t, J=7, $-CH_3$); 3,49 (m, 3x $-NCH_2-$); 3,92 (m, $-S-CH_2$); 4,87 (d,J = 5, H-6); 5,66 (m, H-7 und Phenyl -CH< ); 6,08 (S, H-3); 7,40 (m, 5 aromat. H); 9,43 (d,J = 8, -CONH-); 9,84 (d,J = 7, -CONH-).

Beispiel 3. 7β-[D-α-(4-Aethyl-2.3-dioxo-1-piperazinocarbonylamino)-phenylacetamido]-3-methoxy-3-cephem-4-carbonsäure (Natriumsalz).

a.) Eine Suspension von 8,8 g (58 mMol) D-α-Phenylglycin in 40 ml Methylenchlorid wird zusammen mit 28,5 ml (117 mMol) N.O-Bis-(trimethylsilyl)-acetamid 1 1/2 Stunden bei 20°C gerührt. Man gibt bei -5°/0°C 6,8 ml (58 mMol) 2,6-Lutidin sowie 50 ml einer Lösung, die 70 mMol 4-Aethyl-2,3-dioxo-1-piperazinocarbonylchlorid in Methylenchlorid enthält, zu und rührt 2 Stunden bei 0°C. Das Gemisch wird in 100 ml 0,5-molaren Phosphatpuffer vom pH 7 eingerührt und zweimal mit je 150 ml Essigester gewaschen. Die organische Phase wird verworfen. Die wässrige Phase wird mit 300 ml Essigester-Aceton (8:2) überschichtet, mit konzentrierter Phosphorsäure auf pH 2 angesäuert und mit Natriumchlorid gesättigt. Man trennt die Phasen, extrahiert zweimal mit je 125 ml Essigester-Aceton (8:2) und trocknet die vereinigten organischen Phasen über Natriumsulfat. Nach Eindampfen erhält man amorphe D-α-(4-Aethyl-2,3-dioxo-1-piperazinocarbonylamino)-phenylessigsäure.

b.) Zu einer Suspension von 5,27 g (16,5 mMol) D-α-(4-Aethyl-2,3-dioxo-1-piperazinocarbonylamino)-phenylessigsäure in 37 ml Methylenchlorid gibt man bei -15°C 2,47 ml (19,5 mMol) N,N-Dimethylanilin und 2,05 (15,8 mMol) Chlorameisensäure-isobutylester. Man rührt 100 Min. bei -5°C und gibt dann bei -5°C eine Lösung zu, die man durch 1-stündiges Rühren bei 20°C einer Mischung von 3,45 g (15 mMol) 7β-Amino-3-methoxy-3-cephem-4-carbonsäure und 7,35 ml (30 mMol) N,O-Bis-(trimethylsilyl)-acetamid in 21 ml Methylenchlorid erhalten hat. Nach erfolgter Zugabe wird das Gemisch 2 Stunden bei 0°C gerührt, wonach die Aufarbeitung und Ueberführung in das Natriumsalz wie in Beispiel 2 erfolgt. Das erhaltene 7β-[D-α-(4-Aethyl-2,3-dioxo-1-piperazinocarbonylamino)-phenylacetamido]-3-methoxy-3-cephem-4-carbonsäure - Natriumsalz ist gemäss Dünnschichtchromatogramm identisch mit der gemäss Beispiel 1 erhaltenen Verbindung.

Beispiel 4. 7β-[D-α-(4-Aethyl-2,3-dioxo-1-piperazinocarbonylamino)-p-hydroxyphenylacetamido]-3-methoxy-3-cephem-4-carbonsäure (Natriumsalz).

a.) Analog Beispiel 3a werden aus 86,9 g D-α-p-Hydroxyphenylglycin 66 g kristalline D-α-(4-Aethyl-2,3-dioxo-1-piperazinocarbonylamino)-p-hydroxyphenylessigsäure erhalten. Die Einheitlichkeit des Produktes wird durch Dünnschichtchromatographie nachgewiesen.

b.) 5,53 g (16,5 mMol) D-α-(4-Aethyl-2,3-dioxo-1-piperazinocarbonylamino)-p-hydroxyphenylessigsäure werden analog Beispiel 3b umgesetzt und das Produkt wie dort aufgearbeitet. Man erhält 7β-[D-α-(4-Aethyl-2,3-dioxo-1-piperazinocarbonylamino)-p-hydroxyphenylacetamido]-3-methoxy-3-cephem-4-carbonsäure-Natriumsalz; Dünnschichtchromatographie auf

Merck Kieselgel 60 (Laufmittel: n-Butanol-Eisessig-Wasser 67:10:25) $R_f$ = 0,10; NMR-Spektrum in deuteriertem Dimethylsulfoxid (100 MHz); Banden (ppm) bei 1,08 (t, J=7, C-$CH_3$); 3,52 (m 3x -N-$CH_2$-), 3,70 (s, -O-$CH_3$); 3,92 (m, -S-$CH_2$-); 4,98 (d, J=4, H-6); 5,51 (m, H-7); 6,75 (d, J=8, 2 aromat. H); 7,23 (d, J=8, 2 aromat. H); 9,34 (d, J=8, -CO-NH-); 9,72 (d, J=7, -CONH-).

Beispiel 5. 7β-[D-α-(4-Aethyl-2,3-dioxo-1-piperazinocarbonylamino)-phenylacetamido]-3-chlor-3-cephem-4-carbonsäure-Natriumsalz.

Zu einer Suspension von 5,35 g (16,5 mMol) D-α-(4-Aethyl-2,3-dioxo-1-piperazinocarbonylamino)-phenylessigsäure in 40 ml Methylenchlorid gibt man bei -5° 2,85 ml (22,5 mMol) N,N-Dimethylanilin und 2,05 ml (15,8 mMol) Chlorameisensäure-isobutylester. Man rührt 45 Min. bei -5° und gibt dann bei -5° eine Lösung von 6,00 g (15,0 mMol) 7β-Amino-3-chlor-3-cephem-4-carbonsäure-benzhydrylester in 25 ml Methylenchlorid zu. Man rührt 2 Stunden bei 0°. Das hellgelbe Gemisch wird am Vakuum vom Methylenchlorid befreit. Der Rückstand wird in 100 ml Essigester gelöst und mit je 2mal 100 ml 5%iger Natriumbicarbonat-Lösung, 1mal 100 ml Wasser, 2mal 100 ml 0,1n Salzsäure und 2mal 100 ml gesättigter Kochsalzlösung gewaschen. Die wässrigen Lösungen werden mit 2mal je 100 ml Essigester nachextrahiert. Die über Natriumsulfat getrockneten Extrakte geben beim Eindampfen 8,03 g Rückstand, welcher auf einer Säule vom Durchmesser 3 cm mit 150 g Kieselgel in Essigester gereinigt wird. Die im Dünnschichtchromatogram an Kieselgel (System Essigester) einheitlichen Fraktionen ($R_f$-Wert 0,25) ergeben nach dem Eindampfen amorphen 7β-[D-α-(4-Aethyl-2,3-dioxo-1-piperazinocarbonylamino)-phenylacetamido]-3-chlor-3-cephem-4-carbonsäure-benzhydrylester.

Zur Entesterung und Darstellung des Natriumsalzes löst man 3,80 g (5,3 mMol) des Rückstandes in 70 ml Methylenchlorid und gibt bei 0° 3,8 ml (35 mMol) Anisol und 20 ml (260 mMol) Trifluoressigsäure zu. Man rührt 30 Min. bei 0°. Das hellgelbe Reaktionsgemisch wird am Vakuum eingedampft und der Rückstand mit Aether suspendiert. Der abfiltrierte und mit Aether gewaschene weisse Niederschlag ergibt nach dem Trocknen 7β-[D-α-(4-Aethyl-2,3-dioxo-1-piperazinocarbonylamino)-phenylacetamido]-3-chlor-3-cephem-4-carbonsäure.

2,80 g (3,8 mMol) obiger Säure werden in 30 ml Essigester-Methanol 3:1 gelöst und mit 10 ml einer Lösung enthaltend 5 mMol α-Aethylhexansäure-Natriumsalz in Essigester versetzt. Durch Filtration erhält man kristallines 7β-[D-α-(4-Aethyl-2,3-dioxo-1-piperazinocarbonylamino)-phenylacetamido]-3-chlor-3-cephem-4-carbonsäure-Natriumsalz. Dünnschichtchromatogramm an Kieselgel, System n-Butanol-Pyridin-Eisessig-Wasser 42:24:4:30, $R_f$ = 0,29.

Beispiel 6. 7β-[D-α-(4-Aethyl-2,3-dioxo-1-piperazinocarbonylamino)-p-hydroxyphenylacetamido]-3-(1-methyl-1H-tetrazol-5-yl-mercapto)-3-cephem-4-carbonsäure-Natriumsalz

Zu einer Suspension von 3,84 g (11,5 mMol) D-α-(4-Aethyl-2,3-dioxo-1-piperazinocarbonylamino)-phenylessigsäure in 28 ml Methylenchlorid gibt man bei -5° 2,0 ml (15,8 mMol) N,N-Dimethylanilin und 1,42 ml (10,9 mMol) Chlorameisensäure-isobutylester. Man rührt 45 Min. bei -5° und gibt dann bei -5° eine Lösung von 5,00 g (10,4 mMol) 7β-Amino-3-(1-methyl-1H-tetrazol-5-yl-thio)-ceph-3-em-4-carbonsäure-benzhydrylester in 18 ml Methylenchlorid zu. Man rührt 2 Stunden bei 0°. Nach Aufarbeitung und Reinigung an Kieselgel wie in Beispiel 5 beschrieben erhält man

amorphen 7β-[D-α-(4-Aethyl-2,3-dioxo-1-piperazinocarbonylamino)-phenylacetamido]-3-(1-methyl-1H-tetrazol-5-yl-thio)-ceph-3-em-4-carbonsäure-benzhydrylester. (Ausbeute 27% d.Th.) Dünnschichtchromatogramm an Kieselgel Merck, System Essigester $R_f$-Wert 0,20 . Esterspaltung und Darstellung des Natriumsalzes wie in Beispiel 5 beschrieben ergeben kristallines 7β-[D-α-(4-Aethyl-2,3-dioxo-1-piperazinocarbonylamino)-phenylacetamido]-3-(1-methyl-1H-tetrazol-5-yl-thio)-ceph-3-em-4-carbonsäure-Natriumsalz. Dünnschichtchromatogramm an Kieselgel System n-Butanol-Pyridin-Eisessig-Wasser 42:24:4:30, $R_f$ = 0,5).

Beispiel 7. 7β-[D-α-(4-Aethyl-2,3-dioxo-1-piperazinocarbonylamino)-m-methylsulfonylaminophenylacetamido]-3-cephem-4-carbonsäure.

Zu einer eisgekühlten 20-proz. Lösung von 11,9 ml Phosgen in Toluol werden nacheinander eine Lösung von 2,84 g 1-Aethyl-2,3-dioxo-piperazin in 28 ml Methylenchlorid und 3,35 ml Triäthylamin gegeben, wobei die Temperatur bei 5-10° gehalten wird. Das Gemisch wird 30 Min. bei Eiskühlung und 2 Stunden bei Raumtemperatur nachgerührt.
(Lösung A)

4,88 g D-α-(3-Mesylamino)-phenylglycin in 40 ml Methylenchlorid werden mit 14,7 ml N,O-Bis(trimethylsilyl)-acetamid (BSA) 1 1/4 Stunden bei Raumtemperatur gerührt.

(Lösung B)

Lösung B wird mit 2,31 ml 2,6-Lutidin versetzt, auf 0° gekühlt und zur eisgekühlten Lösung A gegeben. Das Reaktionsgemisch wird noch 2 Stunden bei 0° und 1 Stunde bei Raumtemperatur weitergerührt und anschliessend mit Phosphat-Puffer von pH 7 extrahiert. Die wässrige Phase wird mit Phosphorsäure auf pH~2 eingestellt, mit Essigester zweimal extrahiert, die organische Phase mit gesättigter Kochsalzlösung ausgeschüttelt, über Natriumsulfat getrocknet und eingedampft. Digerieren des Rückstandes mit Aether ergibt D-α-(4-Aethyl-2,3-dioxo-1-piperazino-carbonylamino)- m-methylsulfonylamino-phenylessigsäure als einheitliches, farbloses Pulver.

1,9 g D-α-(4-Aethyl-2,3-dioxo-1-piperazino-carbonylamino)-m-methylsulfonylaminophenylessigsäure gelöst in 14 ml Methylenchlorid/Dimethylformamid (6:1) werden bei -10° mit 0,57 ml Chlorameisensäureisobutylester und 0,76 ml Dimethylanilin 1 1/2 Stunden umgesetzt. Anschliessend wird das Reaktionsgemisch auf -25° gekühlt, mit einer Lösung von 1,68 g 7β-Amino-3-cephem-4-carbonsäurediphenylmethylester in 12 ml Methylenchlorid versetzt und 1 Stunde bei -10°, sowie 2 Stunden bei ca. 0° weitergerührt.

Zur Aufarbeitung wird das Gemisch im Vakuum eingeengt, in Essigsäureäthylester aufgenommen und mit gesättigter Kochsalzlösung ausgeschüttelt. Trocknen über Natriumsulfat und Eindampfen ergibt das Rohprodukt, welches an 110 g Kieselgel chromatographiert wird. Mit Essigsäureäthylester/Toluol

(3:1) wird ein einheitliches Produkt eluiert und nach Digerieren mit Aether zu 7β-[D-α-(4-Aethyl-2,3-dioxo-1-piperazino-carbonylamino)-m-methylsulfonylaminophenylacetamido]-3-cephem-4-carbonsäurediphenylmethylester als farbloses Pulver erhalten.
Schmelzpunkt: ~160-165° (Zersetzung); $R_f$ ~ 0,12 (Silicagel, Essigester); $IR_{(CH_2Cl_2)}$: 3300, 1765, 1710 str., 1680, 1604, 1500 $cm^{-1}$.

1,7 g 7β-[D-α-(4-Aethyl-2,3-dioxo-1-piperazino-carbonylamino)-3-methylsulfonylaminophenylacetamido]-3-cephem-4-carbonsäurediphenylmethylester werden in 17 ml Methylenchlorid gelöst und bei 0° 30 Min. mit 1,75 ml Trifluoressigsäure und 0,38 ml Anisol gerührt. Nach Zugabe von ca. 50 ml kaltem Toluol wird das Gemisch im Vakuum eingedampft und getrocknet. Zweimalige Umfällung ergibt die Titelverbindung als hellbeiges Pulver.
Schmelzpunkt: 168-175° (Zersetzung); $R_f$ ~ 0,08 (Silicagel, n-Butanol-Essigsäure-$H_2O$; $UV_{(Phosphat-Puffer\ pH\ 7,4)}$: $\gamma_{max}$ = 262 nm (~ 8000); $IR_{(Nujol)}$: 3300, 1780 b, 1715, 1678, 1610, 1510.

<u>Beispiel 8</u>. <u>7β-[D-α-(2-Oxo-imidazolidinocarbonylamino)-m-methylsulfonylaminophenylacetamido]-3-cephem-4-carbonsäure</u>.

2,44 g D-α-3-Mesylaminophenylglycin suspendiert in 20 ml Methylenchlorid werden 1 1/4 Stunden bei Raumtemperatur mit 5,3 ml N,O-Bis(trimethylsilyl)-acetamid (BSA) gerührt, wobei eine trübe Lösung entsteht. Darauf wird das Gemisch im Eisbad gekühlt und nacheinander mit 1,15 ml 2,6-Lutidin und einer

Suspension von 1,49 g von 2-Oxo-1-imidazolidinocarbonylchlorid in 30 ml Methylenchlorid versetzt. Das Reaktionsgemisch wird 1 1/4 Stunden bei 0°, sowie 1 Stunde bei Raumtemperatur weitergerührt und dann mit Phosphat-Puffer-pH 7 zweimal extrahiert. Die organische Phase wird verworfen, die wässrige Phase mit Phosphorsäure auf pH 1,9 eingestellt und mit Essigsäureäthylester extrahiert. Nach Ausschütteln mit gesättigter Kochsalzlösung wird der Essigsäureäthylesterextrakt über Natriumsulfat getrocknet und eingedampft. Man erhält die rohe D-α-(2-Oxo-1-imidazolidinocarbonylamino)-m-methylsulfonylaminophenylessigsäure, welche mit Aether digeriert und getrocknet wird.

Eine Suspension von 1,4 g D-α-(2-Oxo-1-imidazolidinocarbonylamino)-3-methylsulfonylaminophenylessigsäure in 12 ml Methylenchlorid/Dimethylformamid (5:1) wird bei ca. -12° mit 0,65 ml N,N-Dimethylanilin und 0,52 ml Chlorameisensäureisobutylester versetzt und 1 1/2 Stunden weitergerührt. Anschliessend wird das Gemisch auf -25° abgekühlt und nach Zugabe einer Lösung von 1,44 g 7β-Amino-3-cephem-4-carbonsäurediphenylmethylester in 10 ml Methylenchlorid 1 Stunde bei ca. -10° und 1 1/2 Stunden bei Eiskühlung weitergerührt. Zur Aufarbeitung verdünnt man mit Methylenchlorid und extrahiert mit gesättigter Kochsalzlösung. Trocknen der organischen Phase über Natriumsulfat und Eindampfen ergibt obiges Rohprodukt, welches an 110 g Kieselgel chromatographiert wird. Elution mit Essigsäureäthylester/Toluol (3:1) ergibt den 7β-[D-α-(2-Oxo-1-imidazolidinocarbonylamino)-m-methylsulfonylaminophenylacetamido]-3-cephem-4-carbonsäurediphenylmethylester, welcher mit Aether zu einem festen, farblosen Pulver digeriert wird.
Schmelzpunkt: 158-162° (Zersetzung); $R_f$: ca. 0,14 (Silicagel, Essigester); $IR_{(CH_2Cl_2)}$ : 3300, 3200, 1782, 1728, 1675, 1603, 1520 $cm^{-1}$.

1,6 g 7β-[D-α-(2-Oxo-1-imidazolidinocarbonylamino)-m-methylsulfonylaminophenylacetamido]-3-cephem-4-carbonsäurediphenylmethylester werden mit 1,75 ml Trifluoressigsäure und 0,38 ml Anisol 30 Min. bei 0° behandelt, anschliessend mit Toluol versetzt, im Vakuum eingedampft und getrocknet. Zweimalige Umfällung aus Aceton/Aether ergibt die Titelverbindung als beiges Pulver.
Schmelzpunkt: 170° (Zersetzung); $R_f$ 0,13 (Silicagel, n-Butanol, Essigsäure, Wasser ; $UV_{(Phosphat\text{-}Puffer\text{-}pH\ 7,4)}$: 250 nm (5500), $IR_{(Nujol)}$: ca. 3300, 1775, 1720, 1653 sh, 1530 $cm^{-1}$.

<u>Beispiel 9.</u> <u>7β-[D,L-α-(2-Oxo-1-imidazolidinocarbonylamino)-α-(2-amino-4-thiazolyl)-acetamido]-3-cephem-4-carbonsäure-Natriumsalz.</u>

7 g 7β-D,L-α-(2-Trichloräthoxycarbonylamino-4-thiazolyl)-glycin suspendiert in 100 ml absolutem Tetrahydrofuran werden bei Raumtemperatur mit 6 ml N,O-Bis(trimethylsilyl)-acetamid während 45 Min. gerührt, wobei eine leicht trübe Lösung entsteht. Nach Zugabe von 2,2 ml N-Methylmorpholin und 3,27 g 2-Oxo-1-imidazolidinocarbonylchlorid wird das Reaktionsgemisch während 5 Stunden bei Raumtemperatur gerührt. Nach Entfernung des Lösungsmittels wird der Rückstand in Essigester gelöst und mit Phosphat-Puffer-pH 7 zweimal extrahiert. Nach Trocknen mit Magnesiumsulfat des Essigesterextraktes und Entfernung des Lösungsmittels am Rotationsverdampfer erhält man die rohe D,L-α-(2-Oxo-1-imidazolidinocarbonylamino)-α-(2-trichloräthoxycarbonylamino-4-

thiazolyl)-essigsäure, die ohne weitere Reinigung im nächsten Reaktionsschritt eingesetzt wird.

Eine auf -20° gekühlte Lösung von 4,61 g D,L-α-(2-Oxo-1-imidazolidinocarbonylamino)-α-(2-trichloräthoxycarbonylamino-4-thiazolyl)-essigsäure und 1,1 ml N-Methylmorpholin in 100 ml absolutem Tetrahydrofuran wird unter Rühren und Feuchtigkeitsausschluss mit 1,3 ml Chlorameisensäureisobutylester versetzt. Nach einer Stunde werden bei -20° 3,66 g 7β-Amino-3-cephem-4-carbonsäurediphenylmethylester zugegeben und das Reaktionsgemisch je eine Stunde bei 0° und Raumtemperatur weitergerührt. Nach Entfernung des Lösungsmittels am Rotationsverdampfer wird der Rückstand in Essigester gelöst, je dreimal mit Eiswasser und gesättigter Natriumchloridlösung gewaschen, über Magnesiumsulfat getrocknet und unter vermindertem Druck vom Lösungsmittel befreit. Darauf wird das Rohprodukt an 500 g Kieselgel mit Essigester als Eluiermittel gereinigt, woraus 7β-[D,L-α-(2-Oxo-1-imidazolidinocarbonylamino-α-(2-trichloräthoxycarbonylamino-4-thiazolyl)-acetamido]-3-cephem-4-carbonsäurediphenylmethylester als farbloses Pulver isoliert wird.
$R_f \sim$ 0,1 und 0,14, DL-Formen (Silicagel, Essigester).

Eine auf 0° gekühlte Lösung von 4,125 g 7β-[D,L-α-(2-Oxo-1-imidazolidinocarbonylamino)-α-(2-trichloräthoxycarbonylamino-4-thiazolyl)-acetamido]-3-cephem-4-carbonsäurediphenylmethylester in 45 ml Essigsäure-Acetonitril (1:1) wird unter Rühren mit 4,6 g Zinkstaub versetzt. Nach 1 1/2 Stunden wird der Zinkstaub abfiltriert und das mit Essigester verdünnte Filtrat je zweimal mit Eiswasser, gesättigter Natriumhydrogencarbonat-Lösung und gesättigter Natriumchlorid-Lösung gewaschen. Nach dem Trocknen der organischen Phase über Magnesiumsulfat und Entfernung des Lösungsmittels am Rotationsverdampfer wird

das Rohprodukt an Kieselgel (300 g) mit Tetrahydrofuran als Eluiermittel gereinigt, woraus 7β-[D,L-α-(2-Oxo-1-imidazolidinocarbonylamino)-α-(2-amino-4-thiazolyl)-acetamido]-3-cephem-4-carbonsäurediphenylmethylester als farbloses amorphes Pulver erhalten wird.
$R_f$: ca. 0,31 (Silicagel, Tetrahydrofuran).

Eine auf 0° gekühlte Lösung von 3,25 g 7β-[D,L-α-(2-Oxo-1-imidazolidinocarbonylamino)-α-(2-amino-4-thiazolyl)-acetamido]-3-cephem-4-carbonsäurediphenylmethylester und 3 ml Anisol in 15 ml Methylenchlorid werden mit 15 ml vorgekühlter Trifluoressigsäure versetzt und 30 Min. gerührt. Nach der Zugabe von 200 ml Petroläther-Diäthyläther (1:1) wird der beige Niederschlag abfiltriert. Das so erhaltene Trifluoressigsäuresalz wird in 40 ml Wasser gelöst, mit Essigsäureäthylester extrahiert und die wässrige Phase mit 2N Natriumhydroxidlösung auf pH 7,5 gebracht, woraus nach der chromatographischen Reinigung am Amberlite XAD-2 mit Wasser-Isopropanol (88:12) als Eluiermittel die Titelverbindung als farbloses amorphes Pulver isoliert werden kann.
$R_f$: 0,18 (Silicagel, n-Butanol-Essigsäure-Wasser 67:23:10),
$UV_{(Phosphatpuffer\ pH\ 7,4)}$: 250 nm (5200).

Beispiel 10.

Trockenampullen oder Vials enthaltend 0,5 g 7β-[D-α-(4-Aethyl-2,3-dioxo-1-piperazinocarbonylamino)-p-hydroxyphenylacetamido]-3-methoxy-3-cephem-4-carbonsäure-Natriumsalz werden wie folgt hergestellt:

Zusammensetzung (für 1 Ampulle oder Vial):

| | |
|---|---|
| 7β-[D-α-(4-Aethyl-2,3-dioxo-1-piperazinocarbonylamino)-p-hydroxyphenylacetamido]-3-methoxy-3-cephem-4-carbonsäure-Natriumsalz | 0,5 g |
| Mannit | 0,05 g |

Eine sterile wässrige Lösung des Natriumsalzes der 7β-[D-α-(4-Aethyl-2,3-dioxo-1-piperazinocarbonylamino)-p-hydroxyphenylacetamido]-3-methoxy-3-cephem-4-carbonsäure-Natriumsalz und des Mannits wird unter aseptischen Bedingungen in 5 ml-Ampullen oder 5 ml-Vials der Gefriertrocknung unterworfen und die Ampullen bzw. Vials verschlossen und geprüft.

In analoger Weise können Trockenampullen mit den anderen in vorstehenden Beispielen beschriebenen Wirkstoffen der Formel I, einem Hydrat oder pharmazeutisch annehmbaren Salz dieser Verbindungen erhalten werden.

Patentansprüche

1. Azacyclyl(thio)ureidoacetamido-cephem-Verbindungen der Formel

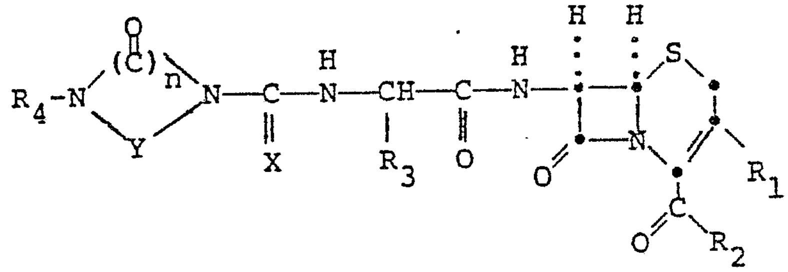

(I),

worin $R_1$ Wasserstoff, Halogen, veräthertes Hydroxy oder Mercapto, oder einen Rest $-CH_2-C(=O)-R_6$, worin $R_6$ Wasserstoff, Hydroxy, Niederalkoxy, Niederalkyl oder gegebenenfalls substituiertes Amino darstellt, $R_2$ Hydroxy oder eine verätherte Hydroxygruppe, die, zusammen mit der Gruppe -C=O, eine unter physiologischen Bedingungen spaltbare veresterte Carboxylgruppe $-C(=O)-OR_5$ bildet, $R_3$ Wasserstoff, einen gegebenenfalls substituierten, ungesättigten cyclischen Kohlenwasserstoffrest oder Heterocyclyl, X Sauerstoff oder Schwefel, der Index n den Wert 1 oder 2, $R_4$ Wasserstoff, einen gegebenenfalls substituierten niederaliphatischen oder cycloaliphatischen Rest oder Acyl und Y ein die Stickstoffatome durch zwei Kohlenstoffatome trennendes Niederalkylen bedeuten, Salze von Verbindungen der Formel I mit salzbildenden Gruppen, sowie Isomere davon.

2. Verbindungen der Formel I gemäss Anspruch 1, worin $R_1$ Wasserstoff, Halogen, Niederalkoxy, Niederalkylthio, gegebenenfalls durch Halogen, Niederalkoxy, Niederalkyl oder Nitro substituiertes Phenylthio, ein gegebenenfalls substituiertes und/oder gegebenenfalls benzokondensiertes monocyclisches, fünf- oder sechsgliedriges, aromatisches oder partiell gesättigtes Heterocylthio, worin

der Heteroring über ein Ring-Kohlenstoffatom an das Thioschwefelatom gebunden ist und mindestens ein Heteroatom aus der Gruppe N, O oder S sowie gegebenenfalls 1-3 weitere Ring N-Atome enthält, oder einen Carbonylmethylrest $-CH_2-C(=O)-R_6$, worin $R_6$ Wasserstoff, Hvdroxy, Niederalkyl, Niederalkoxy oder eine gegebenenfalls niederalkyliertes oder durch Niederalkylen oder Oxa-, Thia-, Aza- oder N-Niederalkylazaniederalkylen cyclisch disubstituiertes Amino ist, bedeutet, $R_2$ Hydroxy oder, zusammen mit der Carbonylgruppe, eine physiologisch spaltbare veresterte Carboxylgruppe der Teilformel (A)

$$-\underset{\underset{O}{\|}}{C}-O-\underset{\underset{R_8}{|}}{CH}-T-R_7 \qquad (A)$$

darstellt, worin $R_7$ gegebenenfalls durch Niederalkoxy oder Diniederalkylamino substituiertes Niederalkanoyl, Niederalkoxycarbonyl, Niederalkylthiocarbonyl, Carbamoyl oder gegebenenfalls durch Niederalkoxy substituiertes Niederalkyl, $R_8$ Wasserstoff oder Methyl, und T Sauerstoff oder Schwefel darstellen oder worin die Teilformel (A) eine 5-Oxodihydro-2-furfuryloxycarbonyl-, 5-Oxo-tetrahydro-2-furfuryloxycarbonyl-, Phthalidyloxycarbonyl- oder 5,6-Dimethoxyphthalidyloxycarbonylgruppe oder die Succinimidomethoxycarbonyl-, Saccharimidomethoxycarbonyl- oder Phthalimidomethoxycarbonylgruppe bildet, $R_3$ für Phenyl, durch Hydroxy, Niederalkanoyloxy, Niederalkylsulfonylamino, Carbamoyloxy, Halogen, Cyan oder Nitro ein- oder zweifach substituiertes Phenyl, Thienyl, Furyl, Cyclohexenyl, Cyclohexadienyl, Tetrazolyl, oder Aminothiazolyl steht, X Sauerstoff oder Schwefel und n 1 oder 2 ist, $R_4$ Wasserstoff, gegebenenfalls durch Hydroxy, Niederalkanoyloxy, Niederalkoxycarbonyloxy, Phenoxycarbonyloxy, Niederalkoxy, Niederalkylthio, Halogen, gegebenenfalls funktionell abgewandeltes Carboxy, oder gegebenenfalls mono- oder diniederalkyliertes Amino substituiertes Niederalkyl sowie Niederalkenyl oder Acyl bedeutet und Y ein Aethylenrest ist, Salze von solchen Verbindungen mit salzbildenden Gruppen, sowie Isomere von solchen Verbindungen der Formel I.

3. Verbindungen der Formel I gemäss Anspruch 1, worin $R_1$ Wasserstoff, Niederalkoxy mit bis zu 4 Kohlenstoffatomen, z.B. Methoxy, Niederalkylthio mit bis zu 4 Kohlenstoffatomen, z.B. Methylthio, Phenylthio, gegebenenfalls durch Niederalkyl, z.B. Methyl, Carboxyniederalkyl, z.B. Carboxymethyl, Sulfoniederalkyl, z.B. Sulfomethyl, Aminoniederalkyl, z.B. 2-Aminoäthyl, Diniederalkylaminoniederalkyl, z.B. 2-Diäthylaminoäthyl, Niederalkoxyniederalkyl, z.B. 2-Methoxyäthyl, oder Halogenniederalkyl, z.B. 2-Chloräthyl, substituiertes Oxadiazolylthio, Thiadiazolylthio, Triazolylthio oder Tetrazolylthio, z.B. entsprechendes 1.2.4- oder 1.3.4-Oxadiazolylthio, -Thiadiazolylthio oder -Triazolylthio oder entsprechendes 5-Tetrazolylthio, Carboxymethyl, Niederalkoxycarbonylmethyl, z.B. Methoxycarbonylmethyl, Niederalkylcarbonylmethyl, z.B. Methylcarbonylmethyl, oder Carbamoylmethyl bedeutet, $R_2$ Hydroxy, Niederalkoxymethoxy, z.B. Methoxymethoxy, Niederalkanoyloxymethoxy, z.B. Pivaloyloxymethoxy oder 2-Phthalidyloxymethoxy darstellt, $R_3$ für Phenyl, m-Methylsulfonylaminophenyl, p-Hydroxyphenyl, 1,4-Cyclohexadienyl, 2- oder 3-Thienyl, 2- oder 3-Furyl, oder 2-Amino-4-thiazolyl steht, X Sauerstoff oder Schwefel und n 1 oder 2 ist, und $R_4$ für Niederalkyl mit 1-4 Kohlenstoffatomen, z.B. Aethyl oder Isobutyl, gegebenenfalls, z.B. in 1-Stellung, durch Hydroxy, Halogen, z.B. Chlor, Carboxy, Niederalkoxy mit bis zu 4 Kohlenstoffatomen, z.B. Methoxy, oder Amino substituiertes Niederalkyl mit 1-4 Kohlenstoffatomen, z.B. entsprechendes Aethyl oder Isobutyl, Niederalkenyl mit 2-5 Kohlenstoffatomen, wie Isobutenyl, z.B. 1-Isobutenyl, oder Niederalkanoyl, z.B. Acetyl, Carbamoyl, Guanyl, Niederalkylsulfonyl, z.B. Mesyl, oder Sulfamoyl steht, pharmazeutisch verwendbare Salze von solchen Verbindungen mit salzbildenden Gruppen, sowie Isomere davon.

4. 7β-[D-α-(4-Aethyl-2.3-dioxo-1-piperazinocarbonylamino)-phenylacetamido]-3-methoxy-3-cephem-4-carbonsäure oder ein Salz davon.

5. 7β-[D-α-(4-Aethyl-2.3-dioxo-1-piperazinocarbonylamino)-phenylacetamido-3-cephem-4-carbonsäure oder ein Salz davon.

6. 7β-[D-α-(4-Aethyl-2.3-dioxo-1-piperazinocarbonylamino)-p-hydroxyphenylacetamido]-3-methoxy-3-cephem-4-carbonsäure oder ein Salz davon.

7. 7β-[D-α-(4-Aethyl-2,3-dioxo-1-piperazinocarbonylamino)-phenylacetamido]-3-chlor-3-cephem-4-carbonsäure oder ein Salz davon.

8. 7β-[D-α-(4-Aethyl-2,3-dioxo-1-piperazinocarbonylamino)-p-hydroxyphenylacetamido]-3-(1-methyl-1H-tetrazol-5-yl-mercapto)-3-cephem-4-carbonsäure oder ein Salz davon.

9. 7β-[D-α-(4-Aethyl-2,3-dioxo-1-piperazinocarbonylamino)-m-methylsulfonylaminophenylacetamido]-3-cephem-4-carbonsäure oder ein Salz davon.

10. 7β-[D-α-(2-Oxo-1-imidazolidinocarbonylamino)-m-methylsulfonylaminophenylacetamido]-3-cephem-4-carbonsäure oder ein Salz davon.

11. 7β-[D,L-α-[2-Oxo-1-imidazolidinocarbonylamino-(2-amino-4-thiazolyl)]-acetamido]-3-cephem-4-carbonsäure oder ein Salz davon.

12. Die in den Beispielen 1-9 beschriebenen neuen Verbindungen.

13. Die nach dem Verfahren der Beispiele 1-9 erhältlichen Verbindungen.

14. Verfahren zur Herstellung von Azacyclyl(thio)ureido-acetamido-cephem-Verbindungen der Formel

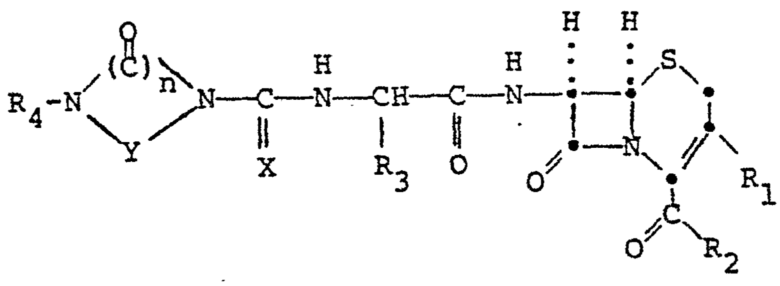

(I),

worin $R_1$ Wasserstoff, Halogen, veräthertes Hydroxy oder Mercapto, oder einen Rest $-CH_2-C(=O)-R_6$, worin $R_6$ Wasserstoff, Hydroxy, Niederalkoxy, Niederalkyl oder gegebenenfalls substituiertes Amino darstellt, $R_2$ Hydroxy oder eine verätherte Hydroxygruppe, die, zusammen mit der Gruppe -C=O, eine unter physiologischen Bedingungen spaltbare veresterte Carboxylgruppe $-C(=O)-OR_5$ bildet, $R_3$ Wasserstoff, einen gegebenenfalls substituierten, ungesättigten cyclischen Kohlenwasserstoffrest oder Heterocyclyl, X Sauerstoff oder Schwefel, der Index n den Wert 1 oder 2, $R_4$ Wasserstoff, einen gegebenenfalls substituierten niederaliphatischen oder cycloaliphatischen Rest oder Acyl und Y ein die Stickstoffatome durch zwei Kohlenstoffatome trennendes Niederalkylen bedeuten, Salze von Verbindungen der Formel I mit salzbildenden Gruppen, sowie Isomere davon, dadurch gekennzeichnet, dass man

a) in einer Verbindung der Formel

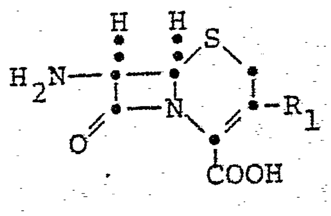

(II),

worin die Aminogruppe gegebenenfalls durch eine die Acylierung erlaubende Gruppe substituiert ist, und worin die 4-Carboxylgruppe und gegebenenfalls im Rest $R_1$ vorhandene weitere funktionelle Gruppen in geschützter Form vorliegen können, die Aminogruppe durch Behandeln mit einem, den Acylrest einer Carbonsäure der Formel

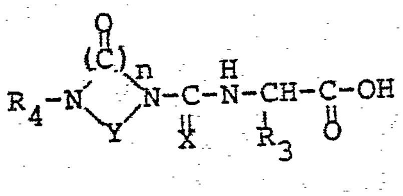

(III)

einführenden Acylierungsmittel, worin gegebenenfalls vorhandene weitere funktionelle Gruppen in geschützter Form vorliegen, acyliert, oder indem man

b) in einer Verbindung der Formel

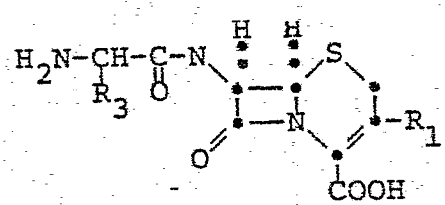

(IV),

worin die Aminogruppe gegebenenfalls durch eine, die Acylierung erlaubende Gruppe substituiert ist, und worin die 4-Carboxylgruppe und gegebenenfalls im Rest $R_1$ und im Rest $R_3$ vorhandene weitere funktionelle Gruppen in geschützter Form vorliegen können, die Aminogruppe durch Behandeln mit einem den entsprechenden Acylrest einer Kohlen- oder Thiokohlensäure der Formel

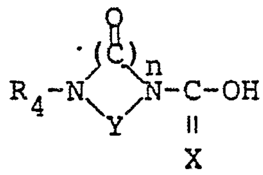

(V)

einführenden Acylierungsmittel, worin im Rest $R_4$ gegebenenfalls vorhandene funktionelle Gruppen in geschützter Form vorliegen können, acyliert oder indem man

c) eine Kohlensäure- oder Thiokohlensäureverbindung der Formel

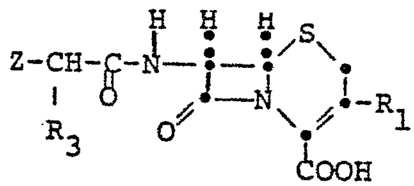

(VI),

worin die 4-Carboxylgruppe und gegebenenfalls in den Resten $R_1$ und $R_3$ vorhandene funktionelle Gruppen in geschützter Form vorliegen können, und worin Z eine von einem Amid der Kohlen- oder Thiokohlensäure abgeleitete, reaktive, mit Aminen unter Ausbildung einer Harnstoff- oder Thioharnstoffgruppierung reagierende Gruppe, z.B. eine Halogencarbonylamino-, Halogenthiocarbonylamino-, Isocyanato- oder Isothiocyanatogruppe ist, mit einem sekundären Amid der Formel

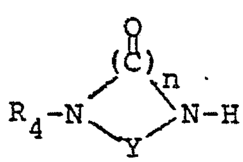

(VII),

worin im Rest $R_4$ gegebenenfalls vorhandene funktionelle Gruppen geschützt sein können und die sekundäre Amidogruppe in einer die Acylierung erlaubenden Form vorliegt, reagieren lässt, oder indem man

d) die unter b) bzw. c) genannte Acylierung in situ vornimmt, indem man eine Aminoverbindung der Formel IV und eine Amidoverbindung der Formel VII, beide mit der oben angegebenen Bedeutung, gleichzeitig oder nacheinander auf ein bivalentes reaktives Derivat der Kohlen- oder Thiokohlensäure einwirken lässt, oder indem man

e) eine Verbindung der Formel

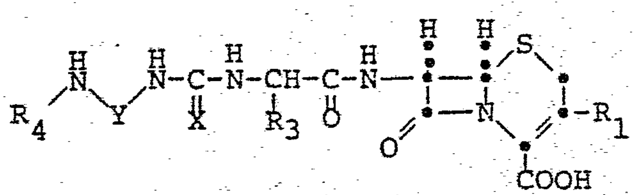
(VIII),

worin die 4-Carboxylgruppe und übrige gegebenenfalls vorhandene funtkionelle Gruppen geschützt sind, und die später den Piperazinring bildende sekundären Amino- bzw. Amidogruppen gegebenenfalls durch Gruppen substituiert sind, die die N-Acylierung erlauben, mit einem bivalenten acylierenden Derivat der Kohlensäure oder Oxalsäure unter Ringschluss diacyliert, oder indem man

f) zur Herstellung von Verbindungen der Formel I, worin $R_1$ eine verätherte Hydroxygruppe oder Halogen bedeutet, und worin funktionelle Gruppen geschützt sind, eine Verbindung der Formel

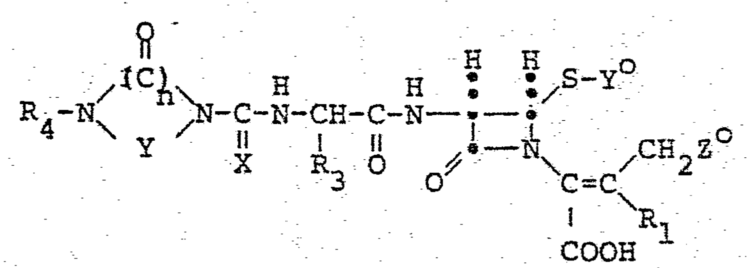
(IX),

worin $R_1$ die vorstehend genannte Bedeutung hat, $Y^o$ eine Abgangsgruppe darstellt und $Z^o$ Wasserstoff oder Halogen bedeutet, und worin funktionelle Gruppen geschützt sind, unter Abspaltung von $Y^o$ und $Z^o$ ringschliesst oder indem man

g) eine 2-Cephemverbindung der Formel

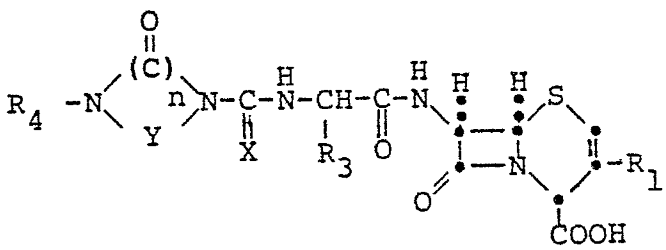

(X),

worin funktionelle Gruppen geschützt sind, zur entsprechenden 3-Cephemverbindung der Formel I isomerisiert, oder indem man

h) in einer Verbindung der Formel

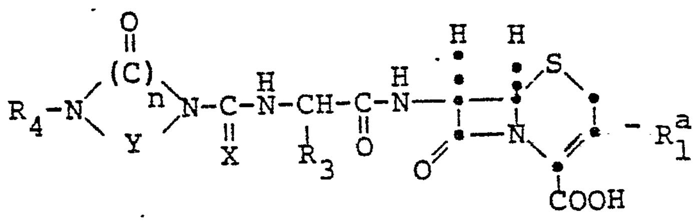

(XI),

worin $R_1^a$ eine freie oder sulfonylierte Hydroxygruppe ist und worin andere funktionelle Gruppen, insbesondere die 4-Carboxylgruppe, vorzugsweise geschützt sind, $R_1^a$ gegen Halogen ausgetauscht, oder indem man

i) in einer Verbindung der Formel XI, worin $R_1^a$ eine freie Hydroxygruppe ist und worin andere funktionelle Gruppen, insbesondere die 4-Carboxylgruppe, geschützt sind, die Hydroxygruppe veräthert, oder indem man

j) in einer Verbindung der Formel XI, worin funktionelle Gruppen vorzugsweise geschützt sind, eine durch ein Thiol substituierbare veresterte oder verätherte Hydroxygruppe $R_1^a$ durch Behandeln mit einem Thiol der Formel $R_1$-H in eine verätherte Mercaptogruppe $R_1$ überführt, oder indem man

k) in einer Verbindung der Formel XI, worin funktionelle Gruppen vorzugsweise geschützt sind, und worin $R_1^a$ eine Formylgruppe bedeutet, diese in Gegenwart von Decarbonylierungs-Katalysatoren zu einer Verbindung der Formel I, worin $R_1$ Wasserstoff bedeutet, decarbonyliert, oder indem man

l) in einer Verbindung der Formel XI, worin $R_1^a$ freies oder verestertes Hydroxy oder ein gegebenenfalls niederalkyliertes oder durch Niederalkylen oder Heteroniederalkylen cyclisch disubstituiertes Amino ist, und worin funktionelle Gruppen gegebenenfalls geschützt sind, die Gruppe $R_1^a$ durch Wasserstoff ersetzt, oder indem man

m) eine Verbindung der Formel XI, worin $R_1^a$ eine freie Hydroxygruppe ist und gegebenenfalls vorhandene weitere reaktive Gruppen und insbesondere die 4-Carboxylgruppe in geschützter Form vorliegen, oder ein 1-Oxid davon, mit einem Ylid der Formel

$$\overset{\oplus}{X} — \overset{\ominus}{CH} — C(=O) — R_6 \qquad (XII),$$

worin $R_6$ die unter Formel I genannte Bedeutung hat, wobei funktionelle Gruppen vorzugsweise geschützt vorliegen und $X^{\oplus}$ eine dreifach substituierte Phosphoniumgruppe oder eine zweifach veresterte Phosphonogruppe zusammen mit einem Kation bedeutet, umsetzt und

in einer nach einem der Verfahren a) - m) erhaltenen Verbindung der allgemeinen Formel I mit geschützten funktionellen Gruppen, die Schutzgruppen entfernt und/oder, wenn erwünscht, eine erhaltene Verbindung der Formel I in eine andere erfindungsgemässe Verbindung der Formel I umwandelt und/oder, wennn erwünscht, ein erhaltenenes Salz in ein anderes Salz oder die freie Verbindung oder eine erhaltene freie Verbindung in ein Salz umwandelt.

15. Verfahren nach Anspruch 14, dadurch gekennzeichnet, dass man Verbindungen der Formel I, worin $R_1$ Wasserstoff, Halogen, Niederalkoxy, Niederalkylthio, gegebenenfalls durch Halogen, Niederalkoxy, Niederalkyl oder Nitro substituiertes Phenylthio, ein gegebenenfalls substituiertes und/oder gegebenenfalls benzokondensiertes monocyclisches, fünf- oder sechsgliedriges, aromatisches oder partiell gesättigtes Heterocyclylthio, wobei der Heteroring über ein Ring-Kohlenstoffatom an das Thioschwefelatom gebunden ist und mindestens ein Heteroatom aus der Gruppe N, O oder S sowie gegebenenfalls 1-3 weitere Ring N-Atome enthält, oder einen Carbonylmethylrest $-CH_2-C(=O)-R_6$, worin $R_6$ Wasserstoff, Hydroxy, Niederalkyl, Niederalkoxy oder eine gegebenenfalls niederalkyliertes oder durch Niederalkylen oder Oxa-, Thia-, Aza- oder N-Niederalkylazaniederalkylen cyclisch disubstituiertes Amino ist, bedeutet, $R_2$ Hydroxy oder, zusammen mit der Carbonylgruppe, eine physiologisch spaltbare veresterte Carboxylgruppe der Teilformel (A)

$$-\underset{\substack{\| \\ O}}{C}-O-\underset{\substack{| \\ R_8}}{CH}-T-R_7 \qquad (A)$$

darstellt, worin $R_7$ gegebenenfalls durch

Niederalkoxy oder Diniederalkylamino substituiertes Niederalkanoyl, Niederalkoxycarbonyl, Niederalkylthiocarbonyl, Carbamoyl oder gegebenenfalls durch Niederalkoxy substituiertes Niederalkyl, $R_8$ Wasserstoff oder Methyl, und T Sauerstoff oder Schwefel darstellen oder worin die Teilformel (A) eine 5-Oxodihydro-2-furfuryloxycarbonyl-, 5-Oxo-tetrahydro-2-furfuryloxycarbonyl-, Phthalidyloxycarbonyl- oder 5,6-Dimethoxyphthalidyloxycarbonylgruppe oder die Succinimidomethoxycarbonyl-, Saccharimidomethoxycarbonyl- oder Phthalimidomethoxycarbonylgruppe bildet, $R_3$ für Rhenyl, durch Hydroxy, Niederalkanoyloxy, Niederalkylsulfonylamino, Carbamoyloxy, Halogen, Cyan oder Nitro ein- oder zweifach substituiertes Phenyl, Thienyl, Furyl, Cyclohexenyl, Cyclohexadienyl, Tetrazolyl, oder Aminothiazolyl steht, X Sauerstoff oder Schwefel und n 1 oder 2 ist, $R_4$ Wasserstoff, gegebenenfalls durch Hydroxy, Niederalkanoyloxy, Niederalkoxycarbonyloxy, Phenoxycarbonyloxy, Niederalkoxy, Niederalkylthio, Halogen, gegebenenfalls funktionell abgewandeltes Carboxy, oder gegebenenfalls mono- oder diniederalkyliertes Amino substituiertes Niederalkyl sowie Niederalkenyl oder Acyl bedeutet und Y eine Aethylenrest ist, Salze von solchen Verbindungen mit salzbildenden Gruppen, sowie Isomere von solchen Verbindungen der Formel I, herstellt.

0015240

Europäisches Patentamt

# EUROPÄISCHER RECHERCHENBERICHT

Nummer der Anmeldung
EP 80 81 0051

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| D | FR - A - 2 269 937 (TOYAMA)<br>* Seiten 210-233; Ansprüche 1,40,41 *<br>& DE - A - 2 519 400<br>-- | 1,14 |
| | FR - A - 2 261 010 (BAYER)<br>* Seiten 57-65; Ansprüche 1,8 *<br>---- | 1,14 |

KLASSIFIKATION DER ANMELDUNG (Int.Cl. 3)

C 07 D 501/59
501/00
501/20//
C 07 D 233/38
241/08

RECHERCHIERTE SACHGEBIETE (Int. Cl 3)

C 07 D 501/59
501/00
501/20

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Den Haag | 20-05-1980 | LUYTEN |